# EUROPEAN PATENT APPLICATION

(11) **EP 3 178 930 A1**
(43) Date of publication of application: **14.06.2017**
(21) Application number: 15829878.6
(22) Date of filing: 22.05.2015
(51) Int. Cl.: C12N 15/09, A01H 5/00, A61K 39/12, A61P 31/12, C07K 14/08, C07K 14/245, C07K 19/00, C12N 1/15, C12N 1/19, C12N 1/21, C12N 5/10, C12P 21/02

(54) **AGENT FOR CONTROLLING PORCINE REPRODUCTIVE AND RESPIRATORY SYNDROME**

(30) Priority: 08.08.2014 JP 2014163108
(71) Applicant: Idemitsu Kosan Co., Ltd., Chiyoda-ku Tokyo 100-8321 (JP)
(72) Inventor: SAWADA, Kazutoshi, Chiba 299-0293 (JP); MATSUI, Takeshi, Chiba 299-0293 (JP); KOIKE, Kazuyoshi, Chiba 299-0293 (JP)
(74) Representative: Vossius & Partner Patentanwälte Rechtsanwälte mbB
(86) International application number: PCT/JP2015/064750
(87) International publication number: WO 2016/021276

(57) **Abstract**

An object of the present invention is to optimize, and to increase the accumulation amount of, GP5 antigen, in order to enhance the performance of a PRRS vaccine. The present invention provides a fusion protein comprising an ectodomain (ectGP5) of Glycoprotein 5 (GP5) of porcine reproductive and respiratory syndrome (PRRS) virus, and an adjuvant protein.

## Description

### TECHNICAL FIELD

The present invention relates to a fusion protein having a controlling effect, namely, prophylactic and therapeutic effects, on porcine reproductive and respiratory syndrome (PRRS), a DNA construct containing a DNA coding for the same, a DNA construct containing DNAs coding for PRRS virus antigens, and a vector and a transformant containing these. Further, the present invention relates to a method for expressing PRRS virus antigens, and a method for producing PRRS virus antigens.

### BACKGROUND ART

Porcine reproductive and respiratory syndrome (PRRS) is the most problematic disease in the swine industry, and an estimated annual loss due to the disease in Japan has been calculated to be 28 billion yen. PRRS causes reproductive disorders in young pigs, and miscarriages in mother pigs. PRRS virus (PRRSV) which causes PRRS is characterized by infecting macrophages, which are immune cells, and thus induces a deficiency in the fundamental immune system of pigs. Therefore, PRRS virus induces the infections of other pathogenic bacteria or viruses, causing enormous damage. In recent years, an emergence of a highly pathogenic PRRSV has been reported overseas, including China. Even a single infection of this virus leads to a severe damage.

Today, vaccines are commercially available from manufacturers overseas, but they have currently proven to be poorly effective. One of the reasons attributable for this is the diversity of PRRSV. PRRSV is largely classified into Type 1 (European Type) and Type 2 (North American Type), and each type is further classified into subtypes. In Japan, attenuated vaccines produced based on North American Type viruses are commercially available. However, their efficacy against polymorphic viruses is regarded as questionable. Further, vaccines produced based on European Type viruses are commercially available overseas, but these have low effect on polymorphism. In addition, in the case of using the virus itself, although being attenuated, the recovery of pathogenicity cannot be totally denied.

Non-patent Document 1 describes the production of a full-length Glycoprotein 5 (GP5) of PRRSV as an antigen using a genetically engineered plant (banana), and it was confirmed that the oral administration of the thus produced GP5 to pigs had a prophylactic effect against PRRSV infection. However, the accumulated amount of GP5 was as low as up to 250 ng/g wet weight (leaves), and it was necessary to administer leaves amounting to a wet weight of 50 g three times, in order to obtain a vaccine effect.

Non-patent Document 2 discloses the production of a corn expressing M protein, and it has been shown that a neutralizing antibody was induced by the oral administration of the corn. However, in the study disclosed in Non-patent Document 2, the test for confirming the effect in pigs, which are actual subject animals, has not been performed. In addition, this document is silent about separately expressing GP5 and M proteins.

Patent Document 1 discloses non-infectious virus-like particles containing GP5 and M proteins. The virus-like particles disclosed in Patent Document 1 also contain envelope proteins other than GP5 and M proteins. Further, in the study disclosed in Patent Document 1, proteins are expressed in such a manner that a plurality of types of PRRSV proteins are translated from one transcription product, but not in a manner that GP5 and M are transcribed as separate transcription units. Still further, since the virus-like particles disclosed in Patent Document 1 are produced using mammalian cultured cells as host cells, there are problems in terms of cost and productivity in order to commercially produce the virus-like particles as a vaccine.

Non-patent Document 3 discloses the fact that the use of an epitope region of GP5 alone as an antigen has been confirmed to exhibit an effect as a PRRS vaccine. However, there is a problem that the amount of accumulated GP5 is low, and thus requires mass production in order to allow GP5 to demonstrate a vaccine effect.

Non-patent Document 4 discloses the expression of a vaccine antigen in which a B subunit of *Escherichia coli* heat-labile toxin (LTB) and a full-length GP5 are fused, in tobacco. However, the vaccine antigen has no significant improvement in performance as compared to a tobacco which expresses the full-length GP5 alone, and the improvement such as the optimization of the GP5 to be fused with LTB has been required in order to enhance the performance of the vaccine.

Accordingly, there has been a demand to optimize, and to increase the accumulation amount of, GP5 antigen, for the purpose of enhancing the performance of a PRRS vaccine.

### PRIOR ART DOCUMENT

### PATENT DOCUMENT

Patent Document 1: EP 1156111 A

### NON-PATENT DOCUMENTS

Non-patent Document 1: Chan et al., Plant Biotechnology J. 11 (2013) 315-324
Non-patent Document 2: Hu et al., Vaccine (2012) 68-74
Non-patent Document 3: Ostrowski et al., J. Virology 2002, 4241-4250
Non-patent Document 4: Min-Yuan Chia et al., Veterinary Immunology and Immunopathology 2011, 140, 215-225

### SUMMARY OF THE INVENTION

### PROBLEMS TO BE SOLVED BY THE INVENTION

An object of the present invention is to optimize, and to increase the accumulated amount of, GP5 antigen, in order to enhance the performance of a PRRS vaccine.

### MEANS FOR SOLVING THE PROBLEMS

The present inventors have found, as a result of intensive studies to solve the above mentioned problems, that it is possible to effectively accumulate GP5 antigen in a plant body, by fusing LTB with GP5 ectodomain (ectGP5).

Further, the present inventors has found that, by co-expressing a full-length GP5 protein including the GP5 ectodomain, and M protein, as separate transcription units, the amount of accumulated GP5 antigen can be significantly increased, as compared to the case of the single expression of GP5.

The present inventors have thereby completed the present invention.

Specifically, the present invention is as follows.
(1) A fusion protein comprising an ectodomain (ectGP5) of Glycoprotein 5 (GP5) of porcine reproductive and respiratory syndrome (PRRS) virus, and an adjuvant protein.
(2) The fusion protein according to (1), wherein the ectodomain has the amino acid sequence from asparagine at position 30 to threonine at position 53 in the amino acid sequence represented by SEQ ID NO: 1, or an amino acid sequence having at least 80% sequence identity to the amino acid sequence, and is capable of inducing a prophylactic effect against PRRS virus infection.
(3) The fusion protein according to (1) or (2), wherein the GP5 is of European Type or North American Type.
(4) The fusion protein according to (3), wherein the GP5 is of any one of Types I and III of the European Type, and Types II, III and IV of the North American Type.
(5) The fusion protein according to (1), wherein the ectodomain has any one of the amino acid sequences represented by SEQ ID NOs: 3, 6, and 21 to 34, or an amino acid sequence having at least 80% sequence identity to the amino acid sequence represented by SEQ ID NO: 6, and is capable of inducing a prophylactic effect against PRRS virus infection.
(6) The fusion protein according to any one of (1) to (5), wherein the adjuvant protein is a B subunit (LTB) of *Escherichia coli* heat-labile toxin and/or a B subunit (Stx2eB) of Type 2 Shiga toxin subclass e (Stx2e).
(7) The fusion protein according to any one of (1) to (6), wherein the ectodomain and the adjuvant protein are linked via a peptide linker.
(8) The fusion protein according to (7), wherein the number of amino acids constituting the peptide linker is from 5 to 25.
(9) The fusion protein according to (7) or (8), wherein, the linker is PG12 (SEQ ID NO: 55), PG17 (SEQ ID NO: 57), or PG22 (SEQ ID NO: 59); or has an amino acid sequence having at least 80% sequence identity to the amino acid sequence represented by SEQ ID NO: 55, 57 or 59.
(10) The fusion protein according to (9), wherein said fusion protein comprises the amino acid sequence represented by SEQ ID NO: 61, or an amino acid sequence having at least 80% sequence identity to the amino acid sequence represented by SEQ ID NO: 61.
(11) A DNA coding for the fusion protein according to any one of (1) to (10).
(12) A DNA construct comprising the DNA according to (11).
(13) A DNA construct comprising a DNA coding for Glycoprotein 5 (GP5) and a DNA coding for Matrix protein (M) of porcine reproductive and respiratory syndrome (PRRS) virus, wherein each of the DNAs is linked to a promoter and a terminator.
(14) The DNA construct according to (13), wherein the DNA coding for GP5 is a DNA coding for any one of the amino acid sequences represented by SEQ ID NOs: 1 and 7 to 20, or an amino acid sequence having at least 80% sequence identity to the amino acid sequence represented by SEQ ID NO: 1; and wherein the GP5 is capable of inducing a prophylactic effect against PRRS virus infection.
(15) The DNA construct according to (13) or (14), wherein the DNA coding for GP5 comprises a DNA coding for the same amino acid sequence as the amino acid sequence represented by SEQ ID NO: 1 except that serine at position 32 is replaced by alanine, and aspartic acid at position 33 is replaced by serine.
(16) The DNA construct according to any one of (13) to (15), wherein the M is of European Type or North American Type.
(17) The DNA construct according to (16), wherein the M is of any one of Types I and II of the European Type, and Types II, III and IV of the North American Type.
(18) The DNA construct according to any one of (13) to (15), wherein the M has any one of the amino acid sequences represented by SEQ ID NOs: 35 and 37 to 50, or an amino acid sequence having at least 80% sequence identity to the amino acid sequence represented by SEQ ID NO: 35, and is capable of inducing a prophylactic effect against PRRS virus infection.
(19) A recombinant vector comprising the DNA construct according to any one of (12) to (18).
(20) A transformant transformed with the recombinant vector according to (19).
(21) The transformant according to (20), wherein the transformant is a plant.
(22) An agent for controlling PRRS comprising the transformant according to (21), or a protein obtained therefrom.
(23) A method for treating or preventing PRRS, comprising administering the agent for controlling PRRS according to (22) to a pig.
(24) A method for expressing GP5 and M in the same cell, comprising transforming a eukaryote having a vesicular transport pathway, with a recombinant vector containing the DNA construct according to any one of (13) to (18).
(25) A method for producing an agent for controlling PRRS, comprising transforming a eukaryote having a vesicular transport pathway, with a recombinant vector containing the DNA construct according to any one of (13) to (18), to express GP5 and M in the same cell.

### EFFECT OF THE INVENTION

By transforming plant cells using a vector containing a DNA coding for the fusion protein according to the present invention, or a vector containing the DNA construct according to the present invention, it is possible to significantly increase the amount of antigen accumulation in the plant cells and improve the performance of controlling PRRS (vaccine effect, immunostimulating effect, or therapeutic effect), as compared to the vaccines disclosed in prior art documents.

The fusion protein according to the present invention or a DNA construct coding for the same allows for preventing or treating PRRS. When a transformed plant transformed with a vector containing a DNA coding for the fusion protein according to the present invention, or with a vector containing the DNA construct according to the present invention, is administered to a healthy pig, an effect as a vaccine or an immunostimulant can be expected.

In the present invention, since an edible plant in which a higher amount of protein antigen is accumulated is orally administered to a pig, an increased productivity can be expected, as compared to conventional vaccines, due to the reduction in the cost, manpower, and stress in pigs.

### BRIEF DESCRIPTION OF THE DRAWINGS

FIG. 1 is a schematic diagram illustrating vectors containing a DNA coding for LTB-fused ectGP5. ER, Apo and Vac indicate that signal sequences are added such that the expressed protein is transported to the endoplasmic reticulum, apoplast, and vacuole, respectively.
FIG. 2 is a schematic diagram showing vectors containing DNAs coding for full-length envelope proteins (GP5 and M) of PRRS virus.
FIG. 3 illustrates the transient expression of the LTB-fused ectGP5s according to the present invention in tobacco cultured cells or lettuce cells (electrophoresis images).
FIG. 4 illustrates the expression of the LTB-fused ectGP5s according to the present invention in stable transformants of tobacco cultured cells (electrophoresis images).
FIG. 5 illustrates the expression of the full-length envelope proteins (GP5, GP4, and M) which are encoded by DNA constructs according to the present invention, in stable transformants of tobacco cultured cells (electrophoresis images).
FIG. 6 illustrates the accumulation of the LTB-fused ectGP5 and the full-length envelope proteins (GP5 and M) in lettuce (electrophoresis images).
FIG. 7 is a graph illustrating a prophylactic effect against PRRV infection by the oral administration of transformed lettuces to pigs. To the negative control group, lettuce prepared with an empty vector was administered. The graph shows the percentage of body weight increase (%) from the first day of challenge until the day of autopsy (three weeks after the challenge).
FIG. 8 shows a list of: subtypes of PRRS virus, names of the strains, locations of discovery, years of discovery, and EMBL protein IDs and SEQ ID NOs of full-length GP5s, ectGP5s and Ms.
FIG. 9 shows the alignments of the amino acid sequences of various types of full-length GP5s.
FIG. 10 shows the alignments of the amino acid sequences of various types of ectGP5s.
FIG. 11 shows the alignments of the amino acid sequences of various types of full-length Ms.
FIG. 12 is a schematic diagram illustrating vectors each containing one of the DNAs coding for the LTB-fused ectGP5s (NA stands for North American Type, EU stands for European Type). A sugar chain is added to each of the fragments of the DNAs, excluding No. 2 and No. 4. To No. 8 and No. 9, a Stx2eB is also added.
FIG. 13 illustrates the accumulation of the LTB-fused ectGP5s in yeast (electrophoresis images). Circles indicate the bands of recombinant proteins to which no sugar chain is added, and stars indicate the bands of recombinant proteins to which a sugar chain is added.

### EMBODIMENTS FOR CARRYING OUT THE INVENTION

### < Fusion protein containing GP5 ectodomain and adjuvant protein, and DNA construct coding for the same (first DNA construct) >

The fusion protein according to the present invention contains an ectodomain (ectGP5) of Glycoprotein 5 (GP5) of PRRS virus, which is fused to an adjuvant protein.

The fusion protein of ectGP5 and an adjuvant protein according to the present invention is preferably a protein produced by a genetic engineering technique, such that two or more protein genes (protein coding region) are linked *in-frame*, to be transcribed and translated continuously, thereby producing one protein. In the fusion protein to be used in the present invention, proteins may be directly fused to each other, or alternatively, proteins may be bound via a peptide linker.

The PRRSV to be used in the present invention may be either a Type 1 (European Type) or Type 2 (North American Type) virus. North American Type is further classified into Types I to VI. In Japan, it is preferred that a Type 2 (North American Type) virus be used. In particular, Types I or III of Type 1 (European Type) virus is preferably used. Further, Types II, III or IV of Type 2 (North American Type) virus is preferably used.

GP5 is one of the envelope proteins of PRRS virus, and GP5 includes the ectodomain (ectGP5). ectGP5 is described, for example, in Ostrowski et al., Journal of virology (2002) 76:4241-4250. In the present invention, ectGP5 is preferably an epitope region capable of inducing a neutralizing antibody against PRRS virus. In the present invention, ectGP5 is more preferably a region from asparagine at position 30 to threonine at position 53 in the amino acid sequence represented by SEQ ID NO: 1.

The amino acid sequence of ectGP5 is represented, for example, by SEQ ID NO: 3 derived from EDRD-I strain, and the nucleotide sequence thereof is represented by SEQ ID NO: 4. An N-linked sugar chain is added to each of the asparagines at positions 15 and 22 in the amino acid sequence represented by SEQ ID NO: 3. However, in the present invention, an amino acid at a glycosylation site may be substituted such that a sugar chain is not added thereto.

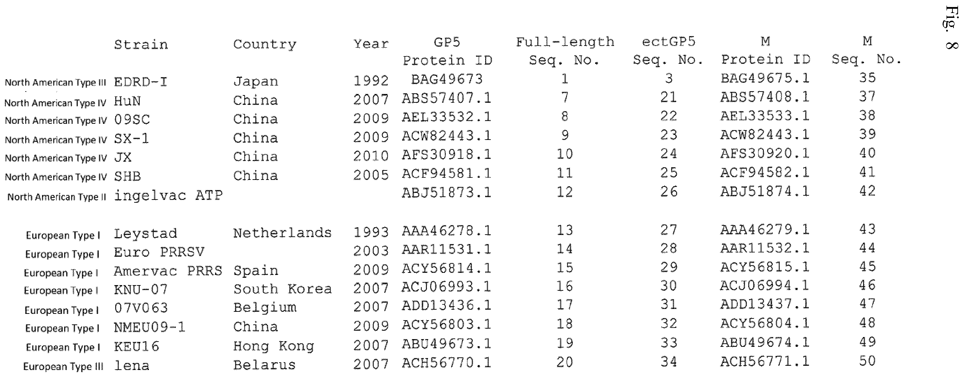

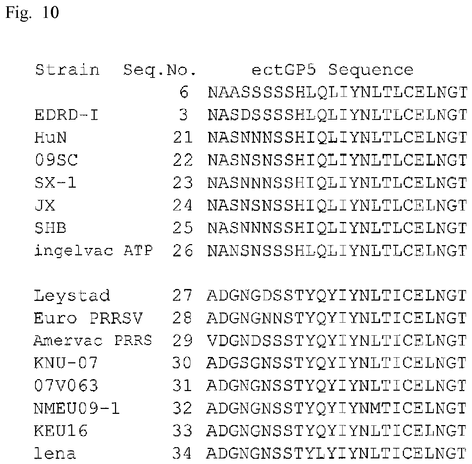

ectGP5 may have any one of the amino acid sequences represented by SEQ ID NOs: 21 to 34 derived from strains other than EDRD-I strain. The names and the like of the strains from which these sequences are derived are listed in FIG. 8. Further, the alignments of the amino acid sequences of various types of ectGP5s are shown in FIG. 10. The sequence identities between the amino acid sequence of SEQ ID NO: 6 and the amino acid sequences of SEQ ID NOs: 21 to 34 are 79, 83, 79, 83, 79, 92, 86, 86, 75, 71, 86, 79, 86 and 92%, respectively.

The amino acid sequence of ectGP5 may be a region from asparagine at position 30 to threonine at position 53 in the amino acid sequence represented by SEQ ID NO: 1, or may be the same as the amino acid sequence represented by SEQ ID NO: 3, except that one or several amino acids are substituted, deleted, inserted and/or added, as long as ectGP5 is capable of inducing a prophylactic effect against PRRS virus infection. In the description of ectGP5, the term "several" refers preferably to a number of from 2 to 10, more preferably from 2 to 5, and still more preferably from 2 to 3. Further, the definition of ectPG5 includes those having a sequence identity of at least 70%, more preferably at least 80%, and still more preferably at least 90% to the above described amino acid sequences, and having a function of inducing a prophylactic effect against PRRS virus infection.

In the present invention, ectGP5 preferably has the amino acid sequence of SEQ ID NO: 6, which is the same as the amino acid sequence of SEQ ID NO: 3, except that the serine at position 3 is replaced by alanine, and the aspartic acid at position 4 is replaced by serine. Further, ectGP5 may be a protein having a sequence identity of at least 70%, more preferably at least 80%, and still more preferably at least 90% to the amino acid sequence represented by SEQ ID NO: 6, and having the above mentioned function.

The adjuvant protein to be used in the present invention is not particularly limited, as long as it facilitates or enhances the function of inducing a prophylactic effect against PRRS virus infection. Examples thereof include *Escherichia coli* heat-labile toxin (LT), which is a proteinaceous toxin produced by enterotoxigenic *Escherichia coli* (ETEC) which causes *Escherichia coli* diarrhea. LT is a holotoxin composed of one A subunit molecule which is the main body of the toxin and five B subunit molecules. The A subunit of LT (LTA) enters into a cytoplasm, and increases the concentration of intracellular cAMP to activate plasma membrane chloride channels, thereby causing a leakage of water into the intestinal tract, in other words, inducing a state of diarrhea. The B subunit of LT (LTB) is nontoxic, and is known to be involved in the adhesion of the LT toxin to intestinal tract cells, and to have a mucosal adjuvant effect.

LTB may have the same amino acid sequence as the amino acid sequence represented by SEQ ID NO: 53, except that one or several amino acids are substituted, deleted, inserted and/or added, as long as it has a mucosal adjuvant effect. In the description of LTB, for example, the term "several" refers preferably to a number of from 2 to 10, more preferably from 2 to 5, and still more preferably from 2 to 3. The amino acid sequence represented by SEQ ID NO: 53 has been registered in Gen Bank under the Accession No. AAL55672.

Further, LTB may be a protein having a sequence identity of preferably at least 85%, more preferably at least 90%, and still more preferably at least 95% to the amino acid sequence represented by SEQ ID NO: 53, and having an adjuvant effect.

The residue at position 90 of the B subunit of *Escherichia coli* heat-labile toxin (namely, the position 90 in the sequence of SEQ ID NO: 53) is asparagine, and a sugar chain is added thereto when the LTB is expressed using a vesicular transport pathway in a eukaryote. In the present invention, LTB in which the asparagine at position 90 is replaced by serine, and to which no sugar chain is added, was used. In the description regarding the LTB subunit protein in which position 90 in its amino acid sequence is a serine residue, the term "several" means that, preferably from 2 to 10, more preferably from 2 to 5, and still more preferably from 2 to 3 amino acids may be substituted, deleted, inserted and/or added in the amino acid sequence represented by SEQ ID NO: 53, at positions other than the position 90 at which the serine residue resides.

Further, the LTB subunit protein in which position 90 in its amino acid sequence is a serine residue, may be a protein having a sequence identity of preferably at least 85%, more preferably at least 90%, and still more preferably at least 95% to the amino acid sequence represented by SEQ ID NO: 53, other than having a serine residue at position 90 in the amino acid sequence represented by SEQ ID NO: 53.

As described above, since the fusion protein according to the present invention is a fusion protein of ectGP5 and an adjuvant protein, and a DNA construct according to the present invention contains a DNA coding for the fusion protein of ectGP5 and an adjuvant protein, it is possible to increase the immunogenicity of the GP5 ectodomain.

The expression "inducing a prophylactic effect against PRRS virus infection" regarding the vaccine to be used in the present invention means that a clinical score indicative of the effect of preventing PRRS in the animals administered with the vaccine is improved, for example, the body weight of the animals is increased to 1.2 times or more, as compared to the animals administered with no vaccine. In the present invention, the accumulated amount of ectGP5 antigen fused to LTB is increased to, for example, 10 times, 100 times, or 400 times or more, as compared to that of full-length GP5 fused to LTB.

The adjuvant protein may be a subunit protein of Shiga toxin, as described in WO 2009/004842. Shiga toxin (Stx) is a proteinaceous toxin produced by enterohemorrhagic *Escherichia coli* (EHEC, STEC), and classified into type 1 (Stx1) and type 2 (Stx2). Stx1 is further classified into subclasses a to d, and Stx2 is further classified into subclasses a to g. Shiga toxin is a holotoxin composed of one A subunit molecule which is the main body of the toxin, and five B subunit molecules responsible for binding to the intestinal mucosa. The toxin has a function of inhibiting protein synthesis by acting on ribosomes in eukaryotic cells. Among these, the adjuvant protein is particularly preferably a B subunit (Stx2eB) of Stx2e, and examples of the B subunit (Stx2eB) include a protein comprising the amino acid sequence of SEQ ID NO: 187. Further, Stx2eB may be a mutant in which Asn73 (namely, the Asn residue at position 55 in the amino acid sequence of SEQ ID NO: 187) is replaced by Ser, or a mutant such as one disclosed in JP 2012-019719 A. Further, Stx2eB may be a protein comprising at least 85%, more preferably at least 90%, and still more preferably at least 95% sequence identity to the amino acid sequence represented by SEQ ID NO: 187, and having an adjuvant effect.

The adjuvant may be a combination of LTB and Stx2eB.

In a preferred embodiment of the fusion protein according to the present invention, the GP5 ectodomain and the adjuvant protein are linked via a linker.

In the fusion protein according to the present invention, the arrangement order in which the GP5 ectodomain and the adjuvant protein are fused may be arbitrary, and either one may be arranged on the N-terminus side.

The peptide linker to be used in the present invention preferably has from 5 to 25, more preferably from 10 to 22, and still more preferably from 12 to 22 amino acids. Further, the peptide linker to be used in the present invention preferably has a proline content of from 20 to 27%, and more preferably from 20 to 25%.

Prolines are preferably arranged with an interval of two or three amino acids in the peptide linker. However, even in the above mentioned arrangement, five or less, preferably four or less amino acids other than proline may be arranged consecutively, at the terminus of the peptide. Such a preferred peptide linker is disclosed, for example, in WO 2009/133882 A.

In the present invention, preferred peptide linkers are: a peptide (PG12) composed of the amino acid sequence represented by SEQ ID NO: 55, or a peptide having at least 80%, and more preferably at least 90% sequence identity to the amino acid sequence of SEQ ID NO: 55 (PG12); a peptide (PG17) composed of the amino acid sequence represented by SEQ ID NO: 57, or a peptide having at least 80%, and more preferably at least 90% sequence identity to the amino acid sequence of SEQ ID NO: 57 (PG17); and a peptide (PG22) composed of the amino acid sequence represented by SEQ ID NO: 59, or a peptide having at least 80%, and more preferably at least 90% sequence identity to the amino acid sequence of SEQ ID NO: 59 (PG22).

In the fusion protein according to the present invention, the GP5 ectodomain and the adjuvant protein are more preferably linked via PG12 (SEQ ID NO: 55). The amino acid sequence of the fusion protein according to the present invention is represented, for example, by SEQ ID NO: 61.

The fusion protein according to the present invention may contain an A subunit of *Escherichia coli* heat-labile toxin (LT), and when it does, it is preferred that the A subunit be detoxified.

By using peptides such as the PG12, PG17 and PG22 as linkers for linking proteins in the fusion protein, the level of the fusion protein accumulated in plant cells can be increased.

In the fusion protein according to the present invention, a secretory signal peptide derived from a plant is preferably added to its amino terminus.
The term "added" as used herein is a concept including both the case where the secretory signal peptide is directly bound to the amino terminus of the fusion protein in which the subunit proteins are linked via the above mentioned peptide, and the case where the secretory signal peptide is bound thereto via another peptide.

The secretory signal peptide is preferably derived from a plant belonging to the family *Solanaceae, Rosaceae*, *Brassicaceae,* or *Asteraceae,* more preferably, derived from a plant belonging to the genus *Nicotiana*, *Arabidopsis*, *Fragaria*, *Lactuca* or the like, and still more preferably derived from tobacco *(Nicotiana tabacum*), *Arabidopsis thaliana*, strawberry (*Fragaria* × *ananassa*), lettuce (Lactuca sativa) or the like.

Further, the secretory signal peptide is preferably derived from β-D-glucan exohydrolase of *Nicotiana tabacum* or 38k-Da peroxidase of *Nicotiana tabacum* (GenBank Accession D 42064). The secretory signal peptide may be, for example, a peptide derived from the β-D-glucan exohydrolase of *Nicotiana tabacum* and comprising the amino acid sequence represented by SEQ ID NO: 63. The nucleotide sequence of a DNA which codes for the β-D-glucan exohydrolase of *Nicotiana tabacum* is represented, for example, by the sequence of SEQ ID NO: 64.

When the fusion protein is expressed in yeast, a secretory signal which functions in yeast, such as an invertase secretory signal, may be used.

In the fusion protein according to the present invention, a signal peptide such as an endoplasmic reticulum retention signal peptide or a vacuolar transport signal peptide may be added to its carboxyl terminus. The term "added" as used herein is a concept including both the case where the signal peptide is directly bound to the carboxyl terminus of the fusion protein, and the case where the signal peptide is bound thereto via another peptide.

In the present specification, a fusion protein in which the secretory signal peptide is added to its amino terminus and the endoplasmic reticulum retention signal peptide is added to the carboxyl terminus is also referred to as an endoplasmic reticulum-type (ER) fusion protein, and a DNA construct coding for the endoplasmic reticulum-type fusion protein is also referred to as an endoplasmic reticulum-type DNA construct. Many studies have reported that the endoplasmic reticulum-type fusion protein is efficiently accumulated in eukaryotes.

In the present specification, a fusion protein in which the secretory signal peptide is added to its amino terminus and neither the endoplasmic reticulum retention signal peptide nor the vacuolar transport signal peptide is added to the carboxyl terminus is also referred to as an apoplast-type (Apo) fusion protein, and a DNA construct coding for the apoplast-type fusion protein is also referred to as an apoplast-type DNA construct.

In the present specification, a fusion protein in which the secretory signal peptide is added to its amino terminus and the vacuolar transport signal peptide is added to the carboxyl terminus fusion protein is also referred to as a vacuolar-type (Vac) fusion protein, and a DNA construct coding for the vacuolar-type fusion protein is also referred to as a vacuolar-type DNA construct.

In the fusion protein according to the present invention, the endoplasmic reticulum retention signal peptide or the vacuolar transport signal peptide is preferably added to its carboxyl terminus. Preferred endoplasmic reticulum retention signal peptides are disclosed, for example, in WO 2009/004842 A and WO 2009/133882 A. Among these, HDEL sequence (SEQ ID NO: 65) can be used. Preferred vacuolar transport signal peptides are disclosed, for example, in WO 2009/004842 A and WO 2009/133882 A. Among these, VSD sequence (SEQ ID NO: 66) can be used.

The fusion protein according to the present invention can be synthesized chemically, or may be produced by genetic engineering. A method for producing the fusion protein by genetic engineering will be described later.

The first DNA construct according to the present invention is characterized by containing a DNA coding for the fusion protein according to the present invention.

In other words, the first DNA construct to be used in the present invention contains a DNA coding for the GP5 ectodomain of PRRS virus, and a DNA coding for an adjuvant protein, preferably a DNA coding for the B subunit of *Escherichia coli* heat-labile toxin. A DNA coding for the peptide linker is represented, for example, by SEQ ID NO: 56 (PG12), SEQ ID NO: 58 (PG17), or SEQ ID NO: 60 (PG22). Examples of the DNA coding for the *Escherichia coli* heat-labile toxin include a DNA (SEQ ID NO: 54) coding for the B subunit (Asn90). The DNA coding for the GP5 ectodomain, and the DNA coding for the adjuvant protein, such as the *Escherichia coli* heat-labile toxin, are linked *in-frame,* excluding stop codons.

The DNA coding for the GP5 ectodomain and the DNA coding for the *Escherichia coli* heat-labile toxin can be obtained by a common genetic engineering technique based, for example, on the nucleotide sequences of SEQ ID NO: 34 and 54, respectively. Specifically, a cDNA library is prepared from a virus or bacterium which produces each protein according to a conventional method, and a desired clone is selected from the library using a probe prepared based on the above mentioned nucleotide sequence. Alternatively, each of the DNAs can also be synthesized chemically, based on the above mentioned nucleotide sequence, or synthesized by PCR using genomic DNA as a template, and 5'- and 3'- terminal nucleotide sequences of the above mentioned nucleotide sequence as primers.

The DNA coding for the fusion protein according to the present invention is represented, for example, by SEQ ID NO: 62.

In the DNA coding for the fusion protein according to the present invention, it is also preferred that a codon(s) corresponding to an amino acid(s) constituting the fusion protein be modified as appropriate such that the amount of the translated hybrid protein is increased depending on the host cell in which the fusion protein is produced.

The modification of the codon(s) can be carried out, for example, by referring to a method disclosed by Kang et al., (2004). Further, examples of the modification method include a method for selecting a codon(s) frequently used in the host cell, a method for selecting a codon(s) having a high GC content, and a method for selecting a codon(s) frequently used in housekeeping genes in the host cell.

The DNA coding for the fusion protein according to the present invention may also be a DNA which hybridizes with the DNA having the nucleotide sequence of SEQ ID NO: 62 under stringent conditions. The term "stringent conditions" refers to the conditions in which a so-called specific hybrid is formed, but not a nonspecific hybrid. Examples of the stringent conditions include those in which two DNAs having a high sequence identity to one another, preferably two DNAs having a sequence identity of at least 80%, more preferably at least 90%, and particularly preferably at least 95% to one another are hybridized with each other, but two DNAs having a sequence identity lower than that described above are not hybridized. The conditions maybe, for example: 2 × SSC (300 mM NaCl, 30 mM citric acid) at 42°C; and preferably: 0.1 × SSC (15 mM NaCl, 1.5 mM citric acid) at 60°C.

### < DNA construct containing DNAs coding for GP5 and M (second DNA construct) >

The DNA construct according to the second embodiment of the present invention contains DNAs coding for Glycoprotein 5 (GP5) and Matrix protein (M) of PRRV virus, respectively. The DNAs coding for GP5 and M are each linked to a set of a promoter and a terminator, and GP5 and M are expressed as separate transcription units.

In the DNA construct coding for GP5 and M according to the present invention, since each of the DNAs is linked to a set of a promoter and a terminator, the protein genes (protein coding regions) of GP5 and M are not linked, and are translated separately to produce two separate proteins.

The DNA construct coding for GP5 and M according to the present invention may be a construct in which a set of the DNA coding for GP5 and a promoter and a terminator linked thereto, and a set of the DNA coding for M and a promoter and a terminator linked thereto, are linked together to form a single DNA construct. Alternatively, in the DNA construct coding for GP5 and M according to the present invention, a set of the DNA coding for GP5 and a promoter and a terminator linked thereto, and a set of the DNA coding for M and a promoter and a terminator linked thereto, may not be liked together. In other words, the DNA construct may be two separate DNA constructs, each containing the DNA coding for GP5 or the DNA coding for M.

GP5 is one of the envelope proteins of PRRS virus, and GP5 includes the ectodomain (ectGP5). In the present invention, GP5 is preferably a full-length protein. However, GP5 need not be a full-length protein, and a partial sequence thereof may be used.

The amino acid sequence of GP5 is represented, for example, by SEQ ID NO: 1 derived from EDRD-I strain, and the nucleotide sequence thereof is represented by SEQ ID NO: 2. There is a possibility that an N-linked sugar chain is added to each of the asparagines at positions 30, 44, and 51 in the amino acid sequence of SEQ ID NO: 1. However, in the present invention, an amino acid at a glycosylation site may be substituted such that a sugar chain is not added thereto.

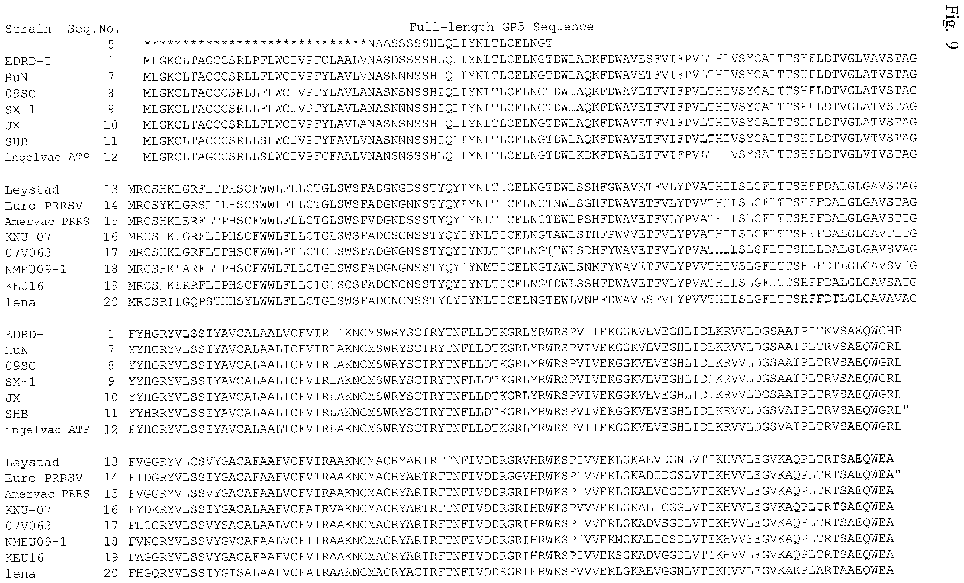

GP5 may have any one of the amino acid sequences represented by SEQ ID NOs: 7 to 20 derived from strains other than EDRD-I strain. The names and the like of the strains from which these sequences are derived are listed in FIG. 8. Further, the alignments of the amino acid sequences of various types of GP5s are shown in FIG. 9. The sequence identities between the amino acid sequence of SEQ ID NO: 5 and the amino acid sequences of SEQ ID NOs: 7 to 20 are 89, 89, 89, 89, 88, 91, 57, 56, 63, 55, 57, 60, 60 and 57%, respectively.

GP5 may have the same amino acid sequence as the amino sequence represented by SEQ ID NO: 1, except that one or several amino acids are substituted, deleted, inserted and/or added, as long as it contains the ectodomain (ectGP5). In the description of GP5, the term "several" refers preferably to a number of from 2 to 10, more preferably from 2 to 5, and still more preferably from 2 to 3.

Further, GP5 may be a protein having a sequence identity of preferably at least 50%, more preferably at least 70%, still more preferably at least 80%, and still more preferably at least 90% to the amino acid sequence represented by SEQ ID NO: 1, and having the above mentioned function.

Still further, GP5 may be a protein having a sequence identity of preferably at least 85%, more preferably at least 90%, and still more preferably at least 95% to the amino acid sequence represented by SEQ ID NO: 1 in which aspartic acid at position 33 is replaced by serine, or to the amino acid sequence represented by SEQ ID NO: 1 in which serine at position 32 is replaced by alanine and aspartic acid at position 33 is replaced by serine (SEQ ID NO: 5), and having the above mentioned function.

Matrix protein (M) is one of the envelope proteins of PRRS virus, and M also includes a domain capable of inducing a prophylactic effect against PRRS virus infection. M and GP5 form a disulfide bond. In the present invention, M is preferably a full-length protein. However, M need not be a full-length protein, and a partial sequence thereof may be used.

The amino acid sequence of M is represented, for example, by SEQ ID NO: 35, and the nucleotide sequence thereof is represented by SEQ ID NO: 36.

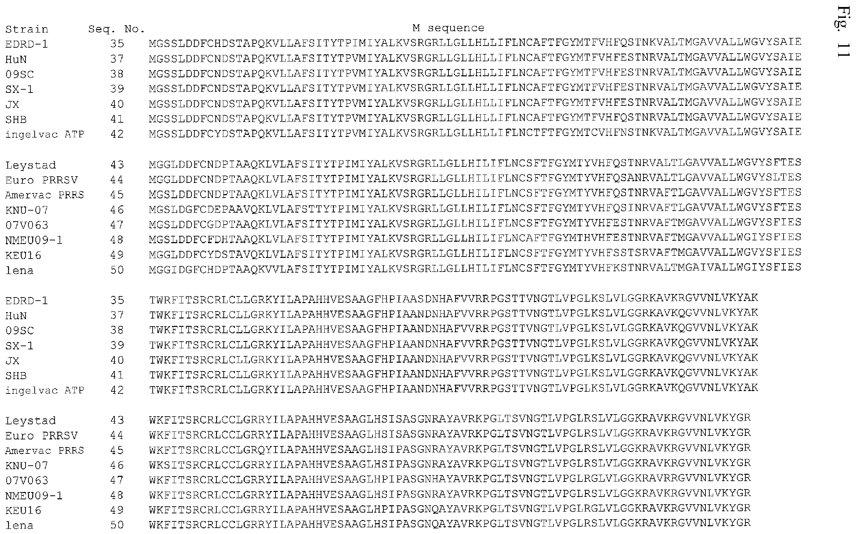

M may have any one of the amino acid sequences represented by SEQ ID NOs: 37 to 50 derived from strains other than EDRD-I strain. The names and the like of the strains from which these sequences are derived are listed in FIG. 8. Further, the alignments of the amino acid sequences of various types of Ms are shown in FIG. 11. The sequence identities between the amino acid sequence of SEQ ID NO: 35 and the amino acid sequences of SEQ ID NOs: 37 to 50 are 96, 96, 96, 96, 97, 95, 81, 81, 81, 78, 82, 82, 82 and 81%, respectively.

M may have the same amino acid sequence as the amino sequence represented by SEQ ID NO: 35, except that one or several amino acids are substituted, deleted, inserted and/or added, as long as it is capable of inducing a prophylactic effect against PRRS virus infection, preferably, as long as it is capable of inducing a prophylactic effect against PRRS virus infection and capable of forming a disulfide bond with GP5. In the description of M, for example, the term "several" refers preferably to a number of from 2 to 10, more preferably from 2 to 5, and still more preferably from 2 to 3.

Further, M may be a protein having a sequence identity of preferably at least 85% or more, more preferably at least 90%, and still more preferably at least 95% to the amino acid sequence represented by SEQ ID NO: 35, and having the above mentioned function.

As described above, since the second DNA construct according to the present invention contains DNAs which respectively code for GP5 and M, as separate transcription units, it is possible to efficiently produce GP5 containing the GP5 ectodomain, and M. Further, since no virus particles are used in the second DNA construct according to the present invention, there is no risk of recovery of the pathogenicity, as there is in the virus particles disclosed in Patent Document 1. In addition, the second DNA construct is capable of selectively expressing M and GP5, unlike the virus particles which are highly likely to induce antibodies other than M and GP5. Accordingly, the second DNA construct serves to produce antigens which are highly effective as a vaccine, unlike the virus particles, which are highly likely to replicate themselves after being taken into macrophages, and to cause damage.

In the first and the second DNA construct to be used in the present invention, it is preferred that the DNA coding for the fusion protein, or each of the DNAs coding for GP5 and the M, be operably-linked to an enhancer. The term "operably" as used herein means that, when the DNA construct to be used in the present invention is inserted into a vector including a suitable promoter, and the vector is introduced into a suitable host cell, the fusion protein or the GP5 and the M is/are produced in the host cell. Further, the term "linked" refers to both the case in which two DNAs are directly linked and the case in which two DNAs are linked via another nucleotide sequence.

Examples of the enhancer include Kozak sequence and a 5'-untranslated region of an alcohol dehydrogenase gene derived from a plant. Particularly preferably, the DNA coding for the hybrid protein is operably-linked to the 5'-untranslated region of an alcohol dehydrogenase gene derived from a plant.

The 5'-untranslated region of an alcohol dehydrogenase gene refers to a region including a nucleotide sequence from the transcription start site before the translation start site (ATG, methionine), of a gene coding for the alcohol dehydrogenase. This region has a function to increase the translation level. The expression "function to increase the translation level" refers to a function to increase the amount of a protein produced by translation when the information encoded in a structural gene is transcribed and then translated to produce the protein. The above mentioned region may be any region as long as it is derived from a plant. However, it is preferably derived from a plant belonging to the family *Solanaceae, Brassicaceae*, *Rosaceae*, *or Asteraceae,* more preferably, derived from a plant belonging to the genus *Nicotiana, Arabidopsis, Fragaria*, *Lactuca*, or the like, and still more preferably derived from tobacco (*Nicotiana tabacum*), *Arabidopsis thaliana,* strawberry (*Fragaria* × *ananassa*), lettuce (*Lactuca sativa*) or the like.

The 5'-untranslated region of an alcohol dehydrogenase gene may be, for example, the 5'-untranslated region of an alcohol dehydrogenase gene (NtADH 5'UTR) (SEQ ID NO: 67) derived from tobacco (*Nicotiana tabacum*). By using the NtADH 5'UTR region in which three nucleotides upstream of the translation start site are modified (NtADHmod 5'UTR) (SEQ ID NO: 68), in particular, a higher translation level can be expected.

Methods for obtaining the 5'-untranslated region of an alcohol dehydrogenase gene derived from a plant are described, for example, in JP 2012-19719 A and WO 2009/133882 A.

In the nucleotide sequence of the NtADHmod 5'UTR such as one represented by SEQ ID NO: 68, one or several nucleotides may be substituted, deleted, inserted and/or added, as long as its function to increase the translation level is maintained. The term "several" as used above refers preferably to a number of from 2 to 10, more preferably from 2 to 5, and particularly preferably from 2 to 3.

In addition, a DNA having a sequence identity of preferably at least 85%, and particularly preferably at least 90% to the NtADHmod 5'UTR and having a function to increase the translation level may also be used.

It is possible to determine whether the above mentioned region has an intended function to increase the translation level or not, for example, by a transient assay using a GUS (β-glucuronidase) gene or luciferase gene as a reporter gene in tobacco cultured cells, or by an assay in transformed cells engineered to carry those genes in a chromosome.

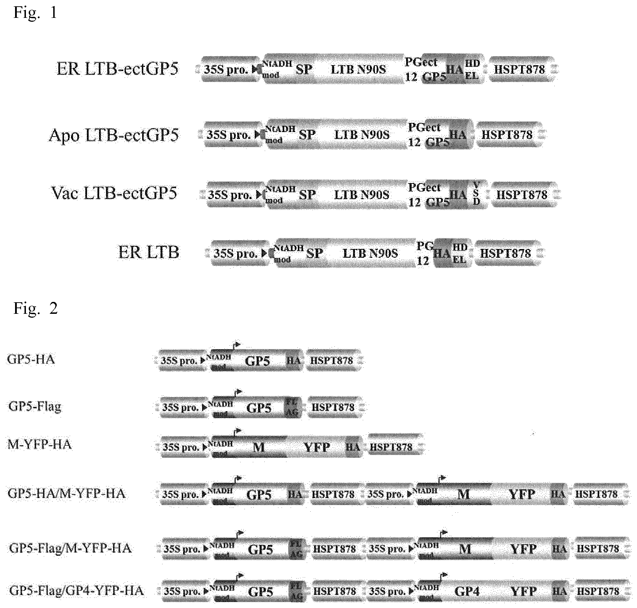

The first DNA construct coding for the fusion protein to be used in the present invention comprises, for example, the nucleotide sequence represented by any one of SEQ ID NOs: 69 to 71 (FIG. 1).

The DNA construct comprising the nucleotide sequence represented by SEQ ID NO: 69 (ER LTB-ectGP5) is a DNA construct in which the DNA coding for the fusion protein in which a LTB protein (mutant type Asn90Ser) and an ectGP5 protein are linked via PG12, the secretory signal peptide is added to the amino terminus, and an HA tag and the endoplasmic reticulum retention signal peptide are added to the carboxyl terminus, is linked to the NtADHmod 5'UTR (SEQ ID NO: 68).

The DNA construct comprising the nucleotide sequence represented by SEQ ID NO: 70 (Apo LTB-ectGP5) is a DNA construct in which the DNA coding for the fusion protein in which a LTB protein (mutant type Asn90Ser) and an ectGP5 protein are linked via PG12, the secretory signal peptide is added to the amino terminus, and an HA tag is added to the carboxyl terminus, is linked to the NtADHmod 5'UTR (SEQ ID NO: 68).

The DNA construct comprising the nucleotide sequence represented by SEQ ID NO: 71 (Vac LTB-ectGP5) is a DNA construct in which the DNA coding for the fusion protein in which a LTB protein (mutant type Asn90Ser) and an ectGP5 protein are linked via PG12, the secretory signal peptide is added to the amino terminus, and an HA tag and the vacuolar transport signal peptide are added to the carboxyl terminus, is linked to the NtADHmod 5'UTR (SEQ ID NO: 68).

The DNA construct comprising the nucleotide sequence represented by SEQ ID NO: 72 (ER LTB) is a DNA construct in which the DNA coding for the fusion protein in which a LTB protein (mutant type Asn90Ser) and PG12 are linked, the secretory signal peptide is added to the amino terminus, and an HA tag and the endoplasmic reticulum retention signal peptide are added to the carboxyl terminus, is linked to the NtADHmod 5'UTR (SEQ ID NO: 68).

The second DNA construct containing DNAs coding for the full-length envelope proteins to be used in the present invention comprises, for example, the nucleotide sequence represented by any one of SEQ ID NOs: 73 to 78 (FIG. 2).

The DNA construct comprising the nucleotide sequence represented by SEQ ID NO: 73 (GP5-HA) is a DNA construct in which the DNA coding for the GP5 protein in which an HA tag is added to the carboxyl terminus, is linked to the NtADHmod 5'UTR (SEQ ID NO: 68).

The DNA construct comprising the nucleotide sequence represented by SEQ ID NO: 74 (GP5-Flag) is a DNA construct in which the DNA coding for the GP5 protein in which a Flag tag is added to the carboxyl terminus, is linked to the NtADHmod 5'UTR (SEQ ID NO: 68).

The DNA construct comprising the nucleotide sequence represented by SEQ ID NO: 75 (M-YFP-HA) is a DNA construct in which the DNA coding for the M protein in which a YFP tag and an HA tag are added to the carboxyl terminus, is linked to the NtADHmod 5'UTR (SEQ ID NO: 68).

The DNA construct comprising the nucleotide sequence represented by SEQ ID NO: 76 (GP5-HA/M-YFP-HA) is a recombinant vector in which the above described GP5-HA and M-YFP-HA are linked to each other, wherein a cauliflower mosaic virus 35S promoter and a HSPT878 terminator described below are added to each of the GP5-HA and M-YFP-HA so that GP5 and M are transcribed as separate transcription units.

The DNA construct comprising the nucleotide sequence represented by SEQ ID NO: 77 (GP5-Flag/M-YFP-HA) is a recombinant vector in which the above described GP5-Flag and M-YFP-HA are linked to each other, wherein a cauliflower mosaic virus 35S promoter and a HSPT878 terminator described below are added to each of the GP5-Flag and M-YFP-HA so that GP5 and M are transcribed as separate transcription units.

The DNA construct comprising the nucleotide sequence represented by SEQ ID NO: 78 (GP5-Flag/GP4-YFP-HA) is a recombinant vector in which the above described GP5-Flag and GP4-YFP-HA are linked to each other, wherein a cauliflower mosaic virus 35S promoter and a HSPT878 terminator described below are added to each of the GP5-Flag and GP4-YFP-HA so that GP5 and GP4 are transcribed as separate transcription units.

The first and the second DNA constructs to be used in the present invention can be prepared by a common genetic engineering technique, which includes the following procedures: digesting each of the DNAs, such as the 5'-untranslated region of an alcohol dehydrogenase gene derived from a plant, a DNA coding for the secretory signal peptide derived from a plant, the DNA coding for the fusion protein, and a DNA coding for the endoplasmic reticulum retention signal peptide or the vacuolar transport signal peptide, with a suitable restriction enzyme; and ligating the resulting fragments with a suitable ligase.

The recombinant vector to be used in the present invention is characterized by including the first or the second DNA construct. The recombinant vector to be used in the present invention may be any vector in which the DNA coding for the fusion protein or the DNAs coding for GP5 and M is/are inserted into the vector such that the DNA can be expressed in a host cell into which the vector is introduced. The vector is not particularly limited as long as it can be replicated in a host cell, and examples thereof include a plasmid DNA, a viral DNA and the like. Further, it is preferred that the vector include a selective marker such as a drug resistance gene. The plasmid DNA can be prepared from *Escherichia coli* or *Agrobacterium tumefaciens* by the alkaline extraction method (Birnboim, H. C. & Doly, J. (1979) Nucleic acid Res 7: 1513) or a variation thereof. Commercially available plasmids such as pRI909, pRI910, pBI221, pBI121, pBI101, pIG121Hm and the like can also be used. As the viral DNA, pTB2 and the like can be used, for example (see, Donson J., Kerney C M., Hilf M E., Dawson W O. Systemic expression of a bacterial gene by a tobacco mosaic virus-based vector. Proc. Natl. Acad. Sci. (1991) 88: 7204-7208).

A promoter to be used in the vector can be selected as appropriate depending on the type of host cell into which the vector is introduced. Preferred examples of the promoter include a cauliflower mosaic virus 35S promoter (Odell et al. 1985 Nature 313:810), a rice actin promoter (Zhang et al. 1991 Plant Cell 3:1155), a corn ubiquitin promoter (Cornejo et al. 1993 Plant Mol. Biol. 23:567), a lettuce ubiquitin promoter (Hirai et al. 2011 Plant Cell Rep. 30:2255-65), and the like. Further, a terminator to be used in the vector may also be selected as appropriate depending on the type of host cell into which the vector is introduced. Preferred examples of the terminator include a nopaline synthase gene transcription terminator, a cauliflower mosaic virus 35S terminator, *Arabidopsis thaliana* heat shock protein 18.2 gene terminator (HSP-T), and the like. A preferred terminator to be used in the present invention is, for example, HSPT878 represented by SEQ ID NO: 79, as described in Matsui et al., 2014.

The recombinant vector according to the present invention can be prepared, for example, as follows.

First, the above mentioned first or second DNA construct is digested with a suitable restriction enzyme, or a restriction enzyme site is added to the DNA construct by PCR. Subsequently, the resulting DNA construct is inserted into the restriction enzyme site or multicloning site of a vector.

The transformant to be used in the present invention is characterized by being transformed with the above mentioned recombinant vector. The host cells to be used for the transformation may be eukaryotic cells or prokaryotic cells. However, eukaryotic cells having a vesicular transport pathway are preferred.

The eukaryotic cells are preferably plant cells, and among these, particularly preferred are cells of plants belonging to the family *Asteraceae* (including those belonging to the genus *Lactuca*, for example), *Solanaceae, Brassicaceae, Rosaceae*, and *Chenopodiaceae.* Further, preferred eukaryotic cells are cells of plants belonging to the genus *Lactuca*, particularly lettuce (*Lactuca sativa*) cells, and cells of plants belonging to the genus *Fragaria*, particularly strawberry (*Fragaria* × *ananassa*) cells. When the lettuce cells are used as the host cells, a cauliflower mosaic virus 35S RNA promoter, a lettuce ubiquitin promoter (Hirai et al. 2011 Plant Cell Rep. 30:2255-65), or the like can be used in the vector. In addition, cells of microorganisms such as yeast (*Saccharomyces cerevisiae*), rice malt (*Aspergillus*), or the like can also be used.

The prokaryotic cells may be cells of *Escherichia coli, Lactobacillus, Bacillus, Brevibacillus*, *Agrobacterium tumefaciens,* and the like.

The transformant to be used in the present invention can be prepared by introducing the vector to be used in the present invention into host cells, using a common genetic engineering technique. Examples of the method which can be used to introduce the vector include: a method using *Agrobacterium* (Hood, et al., 1993, Transgenic, Res. 2: 218, Hiei, et al., 1994 Plant J. 6: 271), an electroporation method (Tada, et al., 1990, Theor. Appl. Genet, 80: 475), a polyethylene glycol method (Lazzeri, et al., 1991, Theor. Appl. Genet. 81: 437), a particle gun method (Sanford, et al., 1987, J. Part. Sci. tech. 5: 27), a polycation method (Ohtsuki), and the like.

After introducing the vector to be used in the present invention into the host cells, the above mentioned transformant can be selected based on the phenotype of the selective marker. Further, the fusion protein, or the GP5 and the M can be produced by culturing the selected transformant. The culture medium and conditions to be used in the culture can be selected as appropriate, depending on the type of the transformant.

In cases where plant cells are used as the host cells, culture of selected plant cells in accordance with a conventional method allows for regeneration of a plant body, and for accumulation of the GP5 protein inside the plant cells or outside the cell membrane of the plant cells. The method varies depending on the type of plant cells to be used, and examples thereof include the method of Visser et al. (Theor. Appl. Genet 78: 594 (1989)) for potato cells, and the method of Nagata and Takebe (Planta 99: 12 (1971)) for tobacco cells.

In the case of lettuce (*Lactuca sativa*) for example, the regeneration of a shoot is possible in MS culture medium containing 0.1 mg/l of NAA (naphthaleneacetic acid), 0.05 mg/l of BA (benzyladenine) and 0.5 g/l of polyvinylpyrrolidone, and the rooting of the regenerated shoot can be achieved by culturing it in 1/2 MS culture medium containing 0.5 g/l of polyvinylpyrrolidone.

Further, the seed to be used in the present invention can be obtained by collecting a seed from the thus regenerated plant body. When the seed to be used in the present invention is seeded and grown by an appropriate method, a plant body capable of producing the GP5 protein can be obtained, and the thus obtained plant body is also included in the above mentioned transformant.

*Agrobacterium tumefaciens* infects a plant through a wound in the plant, and carries a large extrachromosomal element referred to as a Ti (tumor-inducing) plasmid. Many laboratories have devoted considerable effort over several years to develop an *Agrobacterium* system, and as a result, it has become possible to transform various types of plant tissues as desired. Representative plants transformed by the above mentioned technique include tobacco, tomato, sunflower, cotton, rapeseed, potato, poplar, soybean, strawberry, rice, and the like.

It has been demonstrated that various species of plants can be regenerated from tissues transformed with *Agrobacterium tumefaciens.* Examples of such plants include sunflower, tomato, white clover, rapeseed, cotton, tobacco, potato, corn, strawberry, rice, and many other kinds of vegetable crops.

In the present invention, it is preferred that an edible plant such as lettuce, as described above, be transformed with an *Agrobacterium* Ti vector.

In the present specification, the term "agent for controlling PRRS" may be used to generally refer to: the fusion protein of the GP5 ectodomain and the adjuvant protein; the fusion protein in which the GP5 ectodomain and the adjuvant protein are linked via a peptide linker; the first DNA construct containing a DNA coding for the fusion protein; the second DNA construct containing DNAs coding for GP5 and M of PRRS virus; and a plant transformed with a vector containing the first or the second DNA construct; and the like. The agent for controlling PRRS according to the present invention may be in the form of a pharmaceutical such as a vaccine or an immunostimulant, or in the form of a feed, as long as it includes the above mentioned fusion protein, or as long as it includes two separate DNAs coding for GP5 and M of PRRS virus, respectively. In the present invention, the term "controlling" includes both prophylaxis and treatment.

The agent for controlling PRRS according to the present invention has an effect of controlling PRRS (vaccine effect, immunostimulating effect, or therapeutic effect). The symptoms of PRRS as referred to in the present invention include immunodeficiency, weight loss, and respiratory diseases in young pigs, and reproductive disorders (miscarriages) in mother pigs. PRRS infects macrophages and compromises the immune system, and thereby increases the risk of secondary infection of other diseases (such as those caused by mycoplasma and circovirus). In a broad sense, however, the symptoms due to other diseases can also be considered as part of the symptoms of PRRS virus. Further, highly pathogenic PRRS, which is epidemic mainly in China in recent years, could cause death even by a single infection.

The agent for controlling PRRS according to the present invention may contain the above described transformant. The agent for controlling PRRS according to the present invention may include the entire or a part of the transformant containing the fusion protein, or GP5 and M. Further, the transformant can be used as it is, or it can be dried, crushed, and/or the like before being used. It is also possible to add any of other adjuvants which enhance the immunogenicity of the fusion protein, or of GP5 and M, to the agent for controlling PRRS according to the present invention. In general, aluminum hydroxide, cholera toxin (CTB), or a bacterial flagellum such as salmonella flagellin is used as an adjuvant.

The method for controlling PRRS according to the present invention is characterized by administering a plant body transformed with the above mentioned DNA construct, or a dried product or a ground product thereof, to an animal. Examples of subjects to be administered with the agent for controlling PRRS according to the present invention include pigs.

Examples of the method for administering the agent for controlling PRRS according to the present invention to a pig include a method in which a plant body transformed with the DNA construct, or a dried product or a ground product thereof, is mixed with a feed to be fed to a pig; a method in which the plant body, or the dried or ground product thereof, is administered to a pig by nasal drops; and the like. The dose of the agent in this case is preferably 0.2 mg or more, and more preferably 0.5 mg or more per day, in terms of the mass of the fusion protein or the mass of GP5 and M. It is preferred that the agent for controlling PRRS according to the present invention be administered for a plurality of times at certain intervals. For example, the agent may be administered every four to seven days for a total of two to three times.

Examples of the present invention will now be described below. However, the present invention is not limited by the following Examples.

### EXAMPLES

### Example 1. Construction of vaccine genes for expression in plants

The GP5 ectodomain (ectGP5) (Ostrowski et al., 2002), which is known to be a neutralization epitope of PRRS virus, as a vaccine antigen, was fused to a LTB, which is known to have a mucosal adjuvant activity. The vaccine gene constructs were each designed such that the expressed antigen is transported to the endoplasmic reticulum, apoplast, or vacuole, as the accumulation site in a plant cell. Further, protein genes coding for GP5, M or GP4 was constructed for the expression of each of the full-length envelope proteins of PRRS virus. YFP, HA and/or Flag tags as detection tags was/were fused to each of the protein genes. The details are as follows.

### Preparation of vectors containing DNA construct coding for the fusion protein according to the present invention

A LTB (Asn90Ser mutant) fragment (the region from Ala22 to Asn124, excluding a secretory signal peptide to periplasm) was prepared as follows. Two oligonucleotides as shown below were annealed at their 3' complementary ends, and then an elongation reaction was carried out using T4 DNA polymerase (Toyobo, Fukui, Japan). In the first stage reaction, LTB-A and LTB-B, LTB-C and LTB-D, LTB-E and LTB-F, LTB-G and LTB-H, LTB-IN90S and LTB-J were used to prepare A + B, C + D, E + F, G + H, and IN90S + J fragments, respectively. In the second stage reaction, the fragments A + B and C + D, E + F and G + H were used to prepare A + B + C + D and E + F + G + H fragments, respectively. Finally, the fragments A + B + C + D and E + F + G + Hand IN90S + J were used to prepare a full-length A + B + C + D + E + F + G + H + IN90S + J fragment.

LTB-A: 5'-ttggatccgccccccagaccatcaccgagttgtgcagcgagtac-3' (SEQ ID NO: 80)
LTB-B: 5'-cgttgatggtgtagatttgggtgttgcggtactcgctgc-3' (SEQ ID NO: 81)
LTB-C: 5'-ccatcaacgacaagatcctcagctacaccgagagcatgg-3' (SEQ ID NO: 82)
LTB-D: 5'-cttgaaggtgatgatcaccatctccctcttgccggccatgctc-3' (SEQ ID NO: 83)
LTB-E: 5'-atcaccttcaagagcggcgagaccttccaggtcgaggtc-3' (SEQ ID NO: 84)
LTB-F: 5'-cttctggctgtcgatgtgctggctgccggggacctcgacctggaa-3' (SEQ ID NO: 85)
LTB-G: 5'-gacagccagaagaaggccatcgagaggatgaaggacaccctcaggatc-3' (SEQ ID NO: 86)
LTB-H: 5'-gcagagcttgtcgatcttggtctcggtgaggtaggtgatcctga-3' (SEQ ID NO: 87) LTB-IN90S: 5'-aagctctgcgtctggaacagcaagaccccc-3' (SEQ ID NO: 88)
LTB-J: 5'-aaagatctgttctccatgctgatggcggcgatgctgttgggggtcttgttgttccagacgcagagctt-3' (SEQ ID NO: 89)

The resulting LTB (Asn90Ser) fragment was digested with BamHI and BglII, and inserted into the dephosphorylated BamHI-BglII gap of Plasmid 14 (Matsui et al., 2009, Biosci.Biotechnol. Biochem., 73, 1628-34). The resulting plasmid was treated with BglII, followed by dephosphorylation, and a phosphorylated PG12 fragment was inserted thereto. The PG12 fragment was prepared by annealing a PG12-F primer (5'-gatcccctggttctggtcctggttctccta-3') (SEQ ID NO: 90) and a PG12-R primer (5'-gatctaggagaaccaggaccagaaccaggg-3') (SEQ ID NO: 91), followed by phosphorylation with T4 polynucleotide kinase (PNK) (New England Biolabs, Hitchin, UK).

The resulting plasmid was treated with BglII, followed by dephosphorylation, and a phosphorylated HA fragment was inserted thereto (LTBN90S-PG12-HA). The HA fragment was prepared by annealing a HA-F primer (5'-gatcttatccttatgattatcctgattatgctg-3') (SEQ ID NO: 92) and a HA-R primer (5'-gatccagcataatcaggataatcataaggataa-3') (SEQ ID NO: 93), followed by phosphorylation with T4 polynucleotide kinase (PNK).

As an ectGP5 fragment of PRRSV, artificial genes were synthesized based on the amino acid sequence of the EDRD-1 strain (Yoshii et al., Arch Virol, 2005, 150:2313-24), by replacing Ser32 with Ala, and Asp33 with Ser in the sequence. ectGP5-2 (5'-tcg tcc tcc tcg cac ctc cag ctc atc tac aac ttg acc ctc-3') (SEQ ID NO: 95) and ectGP5-3 (5'-tt agatct ggt gcc gtt cag ctc gca gag ggt caa gtt gta-3') (SEQ ID NO: 96) were annealed at their 3' complementary ends, and then an elongation reaction was carried out using T4 DNA polymerase (Toyobo, Fukui, Japan). The resulting ectGP5-2 + 3 fragment was thermally denatured, and then annealed with ectGP5-1 (5'-aa gcatgc ggatcc aac gcc gcc tcc tcg tcc tcc tcg cac-3') (SEQ ID NO: 94), and then an elongation reaction was carried out using T4 DNA polymerase. The resulting ectGP5-1 + 2 + 3 fragment was treated with BamHI and BglII, and then inserted into a vector obtained by treating the above described LTBN90S-PG12-HA plasmid with BglII, followed by dephosphorylation.

Then, a LTBN90S-PG12-ectGP5-HA-HDEL fragment was cleaved out from the resulting plasmid using BamHI and SacI, and the fragment was inserted into the BamHI-SacI gap of a 2BH plasmid (Matsui et al., Transgenic Res., 2011,20; 735-48). The *Arabidopsis thaliana* heat shock protein gene transcription terminator (HSPT) was removed from the resulting plasmid, using SacI and EcoRI, and a SacI-EcoRI fragment of a long chain version of HSPT (HSPT878, Matsui et al., 2014) was inserted into the plasmid In this manner, pRI909 ER LTBN90S-PG12-ectGP5 (SEQ ID NO: 69) plasmid was constructed (FIG. 1).

Further, pRI909 Apo LTBN90S-PG12-ectGP5 (SEQ ID NO: 70) and pRI909 Vac LTBN90S-PG12-ectGP5 (SEQ ID NO: 71) were prepared in the same manner as described above, except for using Plasmids 13 and 15 (Matsui et al., 2009, Biosci. Biotechnol. Biochem., 73, 1628-34), respectively, instead of the Plasmid 14 used in the construction of ER LTB-ectGP5 (FIG. 1).

### Preparation of vectors containing DNA construct coding for full-length GP5, M and/or GP4

The full-length GP5, M, GP4 proteins were prepared based on the amino acid sequence of the EDRD-1 strain (Yoshii et al., Arch Virol, 2005, 150:2313-24), in the following manner. GP5 was prepared in the same manner as the preparation of ectGP5, by replacing Ser32 with Ala, and Asp33 with Ser. Glycoprotein 4 (GP4) is one of the envelope proteins of PRRS virus. The amino acid sequence of GP4 is represented, for example, by SEQ ID NO: 51, and the nucleotide sequence thereof is represented by SEQ ID NO: 52.

Two oligonucleotides as shown below were annealed at their 3' complementary ends, and then an elongation reaction was carried out using T4 DNA polymerase (Toyobo, Fukui, Japan). Thus, each of the full-length genes was synthesized in the same manner as in the construction of LTB.

GP5-1: 5'-aatgcatgttgggca agtgcttgac cgccggctgc-3' (SEQ ID NO: 97)
GP5-2: 5'-gcaccacaagaaggggagcctggagcagcagccgg-3' (SEQ ID NO: 98)
GP5-3: 5'-tctt gtggtgcatc gtgcccttct gcttggccgc c-3' (SEQ ID NO: 99)
GP5-4: 5'-ggaggacgaggaggcggcgttcaccaaggcggcca-3' (SEQ ID NO: 100)
GP5-5: 5'-tcg tcc tcc tcg cac ctc cag ctc atc tac aac ttg acc ctc-3' (SEQ ID NO: 101)
GP5-6: 5'-accagtcggtgccgttcagctcgcagagggtcaag-3' (SEQ ID NO: 102)
GP5-7: 5'-ccg actggttggc cgacaagttc gactgggccg tg-3' (SEQ ID NO: 103)
GP5-8: 5'-tcaacacggggaagatcacgaagctctccacggcc-3' (SEQ ID NO: 104)
GP5-9: 5'-ccgtgttga cccacatcgt gagctactgc gccttg-3' (SEQ ID NO: 105)
GP5-10: 5'-cacggtgtccaagaagtggctggtggtcaaggcgc-3' (SEQ ID NO: 106)
GP5-11: 5'-gacacc gtgggcttgg tggccgtgag caccgccgg-3' (SEQ ID NO: 107)
GP5-12: 5'-gctcaacacgtatctgccgtggtagaagccggcgg-3' (SEQ ID NO: 108)
GP5-13: 5'-cgt gttgagcagc atctacgccg tgtgcgcctt gg-3' (SEQ ID NO: 109)
GP5-14: 5'-gcctgatcacgaagcacaccaaggcggccaaggcg-3' (SEQ ID NO: 110)
GP5-15: 5'-tgatcaggct caccaagaac tgcatgagct ggaga-3' (SEQ ID NO: 111)
GP5-16: 5'-agttggtgtatctggtgcagctgtatctccagctc-3' (SEQ ID NO: 112)
GP5-17: 5'-ga tacaccaact tcttgttgga caccaagggc agg-3' (SEQ ID NO: 113)
GP5-18: 5'-tgatcacggggctcctccatctgtagagcctgccc-3' (SEQ ID NO: 114)
GP5-19: 5'-cccgtgatc atcgagaagg gcggcaaagt ggaagt-3' (SEQ ID NO: 115)
GP5-20: 5'-tcaagtcgatcaagtggccctccacttccactttg-3' (SEQ ID NO: 116)
GP5-21: 5'-tcgacttgaa gagggtggtg ttggacggca gcgcc-3' (SEQ ID NO: 117)
GP5-22: 5'-ggtgatgggggtggcggcgctg-3' (SEQ ID NO: 118)
GP5-23: 5'-ccccatcacc aaagtgagcg ccga-3' (SEQ ID NO: 119)
GP5-24: 5'-ttagatctggggtggccccactgctcggcgctc-3' (SEQ ID NO: 120)
M-1: 5'-aatgcatg ggcagca gcttggacga cttctgccac-3' (SEQ ID NO: 121)
M-2: 5'-acactttctggggggcggtgctgtcgtggcagaag-3' (SEQ ID NO: 122)
M-3: 5'-ccagaa agtgttgttg gccttcagca tcacctaca-3' (SEQ ID NO: 123)
M-4: 5'-caaggcgtagatcatgatgggggtgtaggtgatgc-3' (SEQ ID NO: 124)
M-5: 5'-ctacgcct tgaaagtgag caggggcagg ctcttgg-3' (SEQ ID NO: 125)
M-6: 5'-caagaagatcaacaagtgcaacaagcccaagagcc-3' (SEQ ID NO: 126)
M-7: 5'-gttgatcttc ttgaactgcg ccttcacctt cggct-3' (SEQ ID NO: 127)
M-8: 5'-gctctggaagtgcacgaaggtcatgtagccgaagg-3' (SEQ ID NO: 128)
M-9: 5'-cttccagag caccaacaaa gtggccttga ccatgg-3' (SEQ ID NO: 129)
M-10: 5'-cccacaacaaggccaccacggcgcccatggtcaag-3' (SEQ ID NO: 130)
M-11: 5'-gttgtggg gcgtgtacag cgccatcgag acctgga-3' (SEQ ID NO: 131)
M-12: 5'-agcctgcatctgctggtgatgaatctccaggtctc-3' (SEQ ID NO: 132)
M-13: 5'-gatgcagg ctctgcttgt tgggcaggaa gtacatc-3' (SEQ ID NO: 133)
M-14: 5'-ctccacgtggtgggcgggggccaagatgtacttcc-3' (SEQ ID NO: 134)
M-15: 5'-cacgtgga gagcgccgcc ggcttccacc ccatcgc-3' (SEQ ID NO: 135)
M-16: 5'-accacgaaggcgtggttgtcgctggcggcgatggg-3' (SEQ ID NO: 136)
M-17: 5'-ccttcg tggtgaggag gcccggcagc accaccgtg-3' (SEQ ID NO: 137)
M-18: 5'-gccgggcaccaaggtgccgttcacggtggt-3' (SEQ ID NO: 138)
M-19: 5'-tgcccggc ttgaagagct tggtg-3' (SEQ ID NO: 139)
M-20: 5'-tcttcacggccttcctgccgcccaacaccaagctc-3' (SEQ ID NO: 140)
M-21: 5'-gccgtgaaga ggggcgtggt gaacttg-3' (SEQ ID NO: 141)
M-22: 5'-ttagatctcttggcgtacttcaccaagttcaccac-3' (SEQ ID NO: 142)
GP4-1: 5'-aatgcatg gccgcca gcttgttgtt cttgttggtg-3' (SEQ ID NO: 143)
GP4-2: 5'-ggctcaccaagaagcactcgaagcccaccaacaag-3' (SEQ ID NO: 144)
GP4-3: 5'-ggt gagccaggcc ttcgcctgca agccctgctt c-3' (SEQ ID NO: 145)
GP4-4: 5'-gtcttgatgtcgctcaagctgctgctgaagcaggg-3' (SEQ ID NO: 146)
GP4-5: 5'-gaca tcaagaccaa caccaccgcc gccgccagct t-3' (SEQ ID NO: 147)
GP4-6: 5'-agcagctgatgtcctgcaacacggcgaagctggcg-3' (SEQ ID NO: 148)
GP4-7: 5'-catc agctgcttga ggcacggcga cagcagcccc c-3' (SEQ ID NO: 149)
GP4-8: 5'-ctgcactgcctgctcttcctgatggtctgggggct-3' (SEQ ID NO: 150)
GP4-9: 5'-gcagtgcagg accgccatcg gcacccccgt gtaca-3' (SEQ ID NO: 151)
GP4-10: 5'-tcggtcacgttggcggtgatggtgatgtacacggg-3' (SEQ ID NO: 152)
GP4-11: 5'-cgtgaccga cgagaactac ttgcacagca gcgact-3' (SEQ ID NO: 153)
GP4-12: 5'-gaacaagcagctgctcaacatcaacaagtcgctgc-3' (SEQ ID NO: 154)
GP4-13: 5'-gctgcttgt tctacgccag cgagatgagc gagaag-3' (SEQ ID NO: 155)
GP4-14: 5'-cgttgccgaacaccactttgaagcccttctcgctc-3' (SEQ ID NO: 156)
GP4-15: 5'-cggcaac gtgagcggca tcgtggccgt gtgcgtga-3' (SEQ ID NO: 157)
GP4-16: 5'-acgtgctgcacgtagctggtgaagttcacgcacac-3' (SEQ ID NO: 158)
GP4-17: 5'-gcagca cgtgagggag ttcacccaga ggagcttgg-3' (SEQ ID NO: 159)
GP4-18: 5'-gtgcaagagcctcacgtggtccaccaccaagctcc-3' (SEQ ID NO: 160)
GP4-19: 5'-gctct tgcacttcat gacccccgag accatgag-3' (SEQ ID NO: 161)
GP4-20: 5'-acggtggcccatctcatggtctc-3' (SEQ ID NO: 162)
GP4-21: 5'-gccaccgt gttggcctgc ttgttcgcca-3' (SEQ ID NO: 163)
GP4-22: 5'-ttagatctgatggccaacaagatggcgaacaagc-3' (SEQ ID NO: 164)

The 5'untranslated region (NtADHmod 5'-UTR) of an alcohol dehydrogenase gene derived from tobacco (*Nicotiana tabacum*), in which the sequences neighboring the initiation codon are modified, was amplified by PCR, using ADH-221 (Sato et. al., 2004) as a template, and using an ADH XbaI-F primer (5'-aatctagagtctatttaactcagtattcagaaacaacaaaa-3') (SEQ ID NO: 165) and an ADHmod NsiI-R primer (5'-aaatgcatcttttttcttgatttccttcac-3') (SEQ ID NO: 166). The resulting DNA fragment was treated with XbaI and NsiI, and inserted into the XbaI-BglII gap of Plasmid 14 (Matsui et al., 2009, Biosci. Biotechnol. Biochem., 73, 1628-34), along with a GP5 fragment which had been treated with NsiI and BglII. The resulting plasmid was digested with NsiI, and then self-ligated to be fused such that the initiation codon (atg) of the NtADHmod 5'-UTR coincided with the initiation codon of GP5. The resulting plasmid was digested with BglII, and the HA fragment was inserted thereto. PCR was performed using the resulting plasmid as a template, and using an ADH KpnI-F primer (5'-aaggtacctatttaactcagtattcagaaacaacaaaa-3') (SEQ ID NO: 167) and a NOST-R primer (5'- tgccaaatgtttgaacgatc-3') (SEQ ID NO: 168), to amplify the NtADHmod 5'-UTR-GP5-HA fragment. The resulting fragment was inserted into the KpnI-Sac gap of pRI909 ER LTBN90S-PG12-ectGP5 (GP5-HA) (SEQ ID NO: 73) (FIG. 2). GP5-Flag was prepared in the same manner as described above except that a Flag fragment was inserted instead of the HA fragment. The Flag fragment was prepared by annealing Flag-F (5'-gatctgattataaggatgacgatgacaagg-3') (SEQ ID NO: 171) and Flag-R (5'-gatcccttgtcatcgtcatccttataatca-3') (SEQ ID NO: 172).

The above described NtADHmod 5'-UTR was treated with XbaI and NsiI, and inserted into the XbaI-BglII gap of Plasmid 14 (Matsui et al., 2009, Biosci. Biotechnol. Biochem., 73, 1628-34), along with a M or GP4 fragment which had been treated with NsiI and BglII. Each of the resulting plasmid was digested with NsiI, and then self-ligated to be fused such that the initiation codon (atg) of the NtADHmod 5'-UTR coincided with the initiation codon of M or GP4. A YFP gene fragment (pEYFP, Clontech) was amplified using YFP-F (5'-tttggatcc agcaagggcgaggagctgttca-3') (SEQ ID NO: 169) and YFP-R (5'-tttagatct cttgtacagctcgtccatgccgag-3') (SEQ ID NO: 170). Thereafter, the resultant was digested with BamHI and BglII, and then inserted into the above described GP4 or M plasmid which had been digested with BglII followed by dephosphorylation. Each of the resulting plasmids was digested with BglII, and the HA fragment was inserted thereto. PCR was performed using each of the resulting fragments as a template, and using an ADH KpnI-F primer (SEQ ID NO: 167) and a NOST-R primer (SEQ ID NO: 168), to amplify the NtADHmod 5'-UTR-M/GP4-YFP-HA fragment. Each of the resulting fragments was inserted into the KpnI-Sac gap of pRI909 ER LTBN90S-PG12-ectGP5 (M-YFP-HA (SEQ ID NO: 75), GP4-YFP-HA) (FIG. 2).

Cassettes for co-expressing GP5 and M, or GP5 and GP4, were constructed in the following manner. GP5-HA was treated with SpeI and EcoRI to digest the downstream portion of HSPT878, and the XbaI-EoRI fragment of 35Spro-NtADHmod 5'-UTR-M/GP4-HA-HSPT878 was inserted thereto (GP5-HA/M-YFP-HA) (SEQ ID NO: 76). In the same manner as described above, GP5-Flag/M-YFP-HA (SEQ ID NO: 77) and GP5-Flag/GP4-YFP-HA (SEQ ID NO: 78) were prepared, using GP5-Flag.

### Example 2. Transient expression experiment using protoplasts

The transient expression using protoplasts were carried out in accordance with Matsui et al., 2011, Transgenic Res. 20 (4): 735-48.

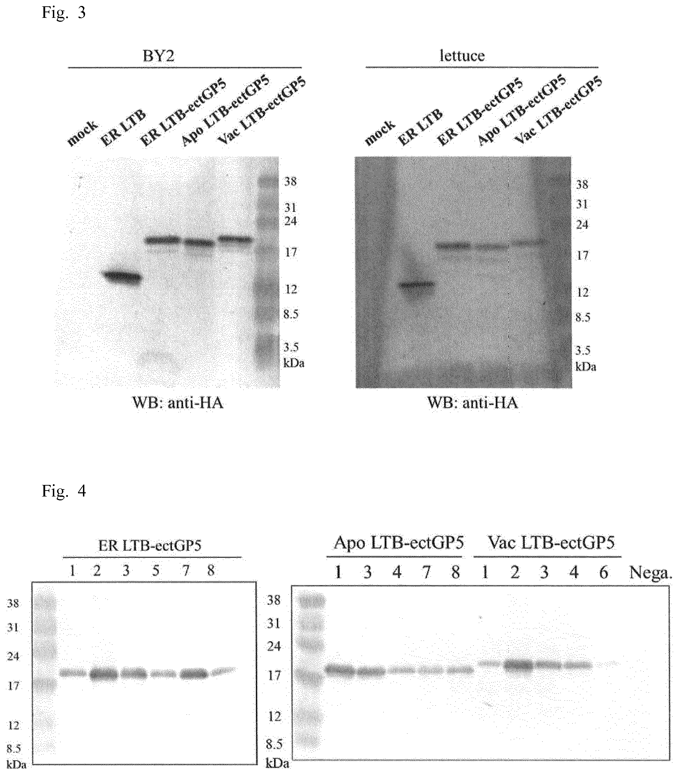

The transient expression of three types of LTB-fused ectGP5s was carried out, using tobacco cultured cells (*Nicotiana tabacum* L. cv. BY2) (RIKEN BioResource Center) and lettuce protoplasts (*Lactucasativa* L. cv. greenwave) (Takii Co., Ltd.). As a result, the accumulation of the antigen was confirmed in both the BY2 and lettuce host cells transformed with any one of the endoplasmic reticulum-, apoplast-, and vacuolar- type DNA constructs (FIG. 3).

### Example 3. Gene transfer into tobacco cultured cells

The transformation of tobacco cultured cells was carried out in accordance with Nakayama et al., 2000, Plant Physiol. 122: 1239-47.

Each vaccine was expressed in stable transformants of tobacco cultured cells. The accumulation of LTB-fused ectGP5 was confirmed in all of the tobacco cells transformed with any one of the endoplasmic reticulum-, apoplast-, and vacuolar-type DNA constructs (FIG. 4).

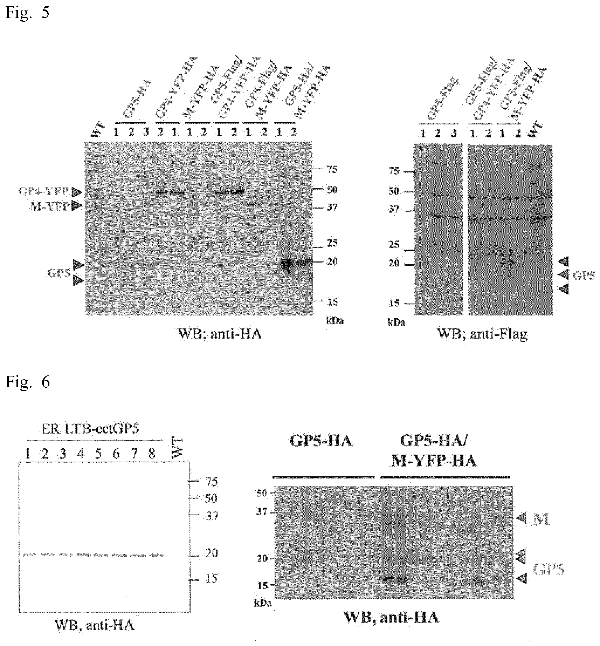

In the expression of the full-length envelope proteins, the accumulation of each of the GP5-HA, M-YFP-HA, and GP4-HA was confirmed. Further, it has been found that the accumulated amount of GP5 is increased when it is co-expressed with M, as compared to the case of the single expression of GP5. The effect of increasing the accumulated amount of GP5 was not observed in the case of co-expression with GP4 (FIG. 5).

### Example 4. Gene transfer into lettuce using Agrobacterium tumefaciens

Genetically engineered lettuces were prepared using three types of DNA constructs: ER LTB-ectGP5, GP5-HA, and GP5-HA/M-YFP-HA, as follows.

Lettuce (*Lactuca sativa L*.), cultivar: Green wave (Takii Co., Ltd.) was seeded aseptically in MS culture medium [1/2 × mixed salts for Murashige and Skoog medium (MS salts, Wako Pure Chemical Industries, Ltd.), 1 × Murashige and Skoog vitamin solution (MS vitamins, Sigma-Aldrich), 3% sucrose, 0.8% agar, pH 5.8]. Ten to 16 days after the seeding, a true leaf was collected, and a section of approximately 5 mm square was cut out. After immersing the section in a suspension of *Agrobacterium tumefaciens* (EHA105) carrying a binary plasmid (pRI909) containing each of the vector constructs for 10 minutes, the section was placed in a co-culture medium [1 × MS salts, 1 × MS vitamins, 0.05 mg/l 6-benzylaminopurine (BA), 0.1 mg/l 1-naphthylacetic acid (NAA), 0.1 M acetosyringone, 3% sucrose, 0.8% agar, pH 5.8], and cultured for two days at 25°C in the dark. After washing with sterilized water, the section was placed on a selection medium [1 × MS salts, 1 × MS vitamins, 0.05 mg/l BA, 0.1 mg/l NAA, 0.5 g/l polyvinylpyrrolidone (PVP), 50 mg/l kanamycin (Km), 250 mg cefotaxime (Cef), 3% sucrose, 0.8% agar, pH 5.8], and cultured at 25°C under fluorescence light (2,000 to 3,000 lux). Thereafter, the section was transferred to a new selection medium every three to four days (twice per week) until adventitious shoots were obtained. Redifferentiated individuals formed from the adventitious shoots were transplanted to a rooting medium [1/2 × MS salts, 1 × MS vitamins, 0.5 g/l PVP, 250 mg Cef, 3% sucrose, 0.8% agar, pH 5.8], and cultured under the same conditions. Thereafter, the redifferentiated individuals were transplanted to a new rooting medium every three to four days (twice per week). The rooted redifferentiated individuals were transplanted to a pot, and cultured under the same conditions.

### Example 5. Extraction of proteins from plant

The extraction of proteins was carried out in accordance with the TCA-acetone method (Shultz et al. Plant Mol Biol Rep, 2005, 23:405), using true leaves of the transgenic lettuces which had been frozen with liquid nitrogen and stored at-80°C. A quantity of 100 to 200 mg of each lettuce sample was crushed using Tissue Lyzer II (QIAGEN), and to the resultant, TCA-acetone (10% trichloroacetic acid, 90% acetone, and 0.07% 2-mercaptoethanol) in an amount five times the amount of the sample was added. The resultant was mixed and left to stand for one hour at -20°C, and then centrifuged at 16,000 × g and at 4°C for 30 minutes, followed by removing the supernatant, thereby obtaining precipitates containing proteins. Further, in order to remove impurities, acetone/BME (100% acetone, 0.07% 2-mercaptoethanol) in an amount five times the amount of the sample was added, and the resultant was mixed and centrifuged at 16,000 × g and at 4 °C for 10 minutes, followed by removing the supernatant. The above described operation to remove impurities was carried out for two more times. The resulting precipitates were dried under reduced pressure, and suspended in extraction I buffer [0.5 M sodium chloride, 5 mM imidazole, 6M urea, 20 mM tris(hydroxymethyl)aminomethane (Tris)-HCl, pH 7.9] in an amount two times the amount of the sample. The resulting suspension was centrifuged at 16,000 × g and at 4°C for 10 minutes, and the supernatant was collected, thereby obtaining a protein solution. The concentration of the proteins was measured using Protein Assay Kit II (Bio-Rad).

### Example 6. Western analysis

The thus obtained protein solution was placed in a microtube in an appropriate amount, and the same amount of sample buffer (EZ Apply, manufactured by ATTO) was added thereto. The resultant was then mixed, and heated for five minutes in boiling water to carry out SDS treatment of the sample. The purified LTB (LTB (Asn90)) was used as a standard reference material when carrying out the quantification of proteins. The purified LTB was repeatedly diluted two-fold using the extraction I buffer to prepare a dilution series, and the dilution series was used as a standard.

The electrophoresis (SDS-PAGE) of proteins was carried out using an electrophoresis tank (Mini Protean Tetracell) and Mini Protean TGX- gel (BIO RAD). An electrophoresis buffer (EZ Run, manufactured by ATTO) was added, 5 µl of the SDS-treated sample was applied to a well, and the electrophoresis was carried out at a constant voltage of 200 V for 40 minutes.

After the electrophoresis, the blotting of the gel was carried out using a Trans-Blot Transfer Pack (BIO RAD) and Trans-Blot Turbo (BIO RAD).

The blotted membrane was immersed in a blocking solution (TBS-based, pH 7.2, Nakalai Tesque, Inc.), followed by shaking at room temperature for one hour, or left to stand at 4°C for 16 hours. The membrane was then shaken in TBS-T (137 mM sodium chloride, 2.68 mM potassium chloride, 1% polyoxyethylene sorbitan monolaurate, 25 mM Tris-HCl, pH 7.4) at room temperature for five minutes, and the shaking was repeated for a total of three times to carry out washing.

A primary antibody diluted 10,000-fold with TBS-T was used for the detection of the recombinant protein. The membrane was immersed in the diluted liquid, followed by shaking at room temperature for two hours to allow an antigen-antibody reaction to proceed. The membrane was then shaken in TBS-T at room temperature for five minutes, and the shaking was repeated for a total of three times to carry out washing. A secondary antibody was diluted 10,000 fold with TBS-T, and the membrane was immersed in the diluted liquid, followed by shaking at room temperature for one hour to allow an antigen-antibody reaction to proceed. The membrane was then shaken in TBS-T at room temperature for five minutes, and the shaking was repeated for a total of three times to carry out washing.

### Anti-HA tags:

Primary antibody Anti-HA antibody (Roche, Cat No. 11-867-423-001)
Secondary antibody Anti-Rat IgG, AP-conjugate (Promega, Cat No. S3831) Anti-FLAG tags:
Primary antibody Anti-FLAG antibody (SIGMA, Cat No. F1804)
Secondary antibody Anti-Rabbit IgG, AP-linked Antibody (Cell Signaling TECHNOLOGY, Cat No. 7054S

To carry out a chromogenic reaction with alkaline phosphatase, the washed membrane was immersed in a chromogenic solution (0.1 M sodium chloride, 5 mM chlorinated magnesium, 0.33 mg/ml nitro blue tetrazolium, 0.33 mg/ml 5-bromo-4-chloro-3-indolyl-phosphoric acid, 0.1 M Tris-HCl, pH 9.5), followed by shaking at room temperature for seven minutes. The membrane was then washed with distilled water and dried at normal temperature. The stained membrane was imaged at a resolution of 600 dpi using a scanner (PM-A900, Epson), and the quantification of the antigen proteins was carried out using an image analysis software (CS Analyzer ver. 3.0, ATTO). Table 1 shows the comparison of the accumulated amount of antigen, in the cases of using, as the antigen: the full-length GP5-LTB (corresponding to Non-patent Document 4); the full-length GP5 (corresponding to Comparative Example); the full-length GP5/M (corresponding to the second invention); and the LTB-ectGP5 (corresponding to the first invention). It can be seen from the results shown in Table 1 that, when the full-length GP5 and M are expressed simultaneously, the accumulated amount of antigen is increased to 2.5 times or more as compared to the case in which the full-length GP5 alone is expressed. Further, it can also be seen that the accumulated amount of antigen (ectGP5) in LTB-ectGP5 is 400 times or more higher than the accumulated amount of antigen (full-length GP5-LTB) of Non-patent Document 4.

**[Table 1]**

| | Host | Antigen | Accumulated amount of antigen (ng/l g (wet weight) of leaves) | Accumulated amount of antigen, in terms of a mount of ectGP5 |
|---|---|---|---|---|
| Non-patent Document 4 | Tobacco | Full-length GP5 -LTB | 155 | 18.6 |
| Comparative Example | Lettuce | Full-length GP5 | ~2000 | ~240 |
| Second Invention | Lettuce | Full-length GP5 /M | 5000 ~ 15000 | 600 ~ 1800 |
| First Invention | Lettuce | LTB-ectGP5 | 40000 ~ 85000 | 7500 ~ 16000 |

These results confirmed that the accumulation of recombinant antigen can be achieved by using any one of the constructs. Further, as with the case of tobacco cultured cells, the accumulated amount of GP5 was higher when GP5 and M were co-expressed, as compared to the case of the single expression of GP5 (FIG. 6).

### Example 7. Phylaxis test of PRRS virus

The test for confirming the prophylactic effect of the vaccines against PRRS virus infection was carried out by oral administration of vaccine lettuces, prepared using two types of vaccines: ER LTB-ectGP5 (vaccine 1) and GP5-HA/M-YFP-HA (vaccine 2). Recombinant lettuce prepared with an empty vector (pRI909) was used as a negative control. On days 9, 16, 29, 30, 31, 36, 37, 38, 43, 44, 45, 51, and 52 after the delivery, a solution obtained by suspending the freeze-dried powder (0.5 g/administration) of each of the vaccine lettuces or of the lettuce prepared with an empty vector in water was administered by forced feeding, using a syringe connected to a tube.

The cells of MA-104 strain (African green monkey kidney cells) (ATCC Accession No. CRL-2378) which had been grown to a confluent state in DMEM (supplemented with 10% inactivated fetal bovine serum, 100 U/ml penicillin, 100 U/ml streptomycin) were infected with the infecting virus, and cultured in a CO₂ incubator at 37°C for six days. After culturing, a centrifugal separation (3,000 rpm, 15 minutes) was carried out, and the resulting supernatant was used as a virus suspension. A diluted liquid of the virus suspension was administered to 65 day-old pigs by spraying the liquid into both nasal cavities of the pigs, using a canyon spray. The dosage of the virus was 8.89 × 10⁸ copies per pig.

After the challenge, the body weight, feed intake, and body temperature of the pigs were measured. Further, the collection of blood was carried out periodically, and the PRRSV-specific antibody titer was measured by ELISA. The pigs were euthanized on day 21 after the challenge with STEC, subjected to an autopsy, and the abnormalities in each of their organs and tissue were macroscopically observed. Pathology specimens of primary organs were prepared, and histopathological findings were examined.

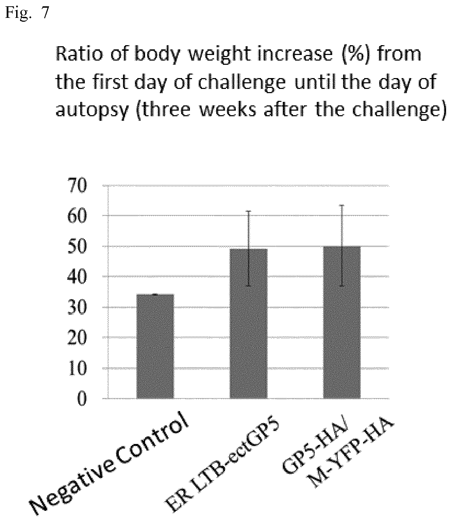

As a result, no difference in the respiratory symptoms and the body temperature was observed between the groups. Further, although there was no difference in the body weight increase between the groups during the vaccination period, within three weeks after the challenge, a higher tendency of the body weight increase was observed in pigs in the vaccine administered groups (FIG. 7).

### Example 8. Expression of recombinant proteins in yeast

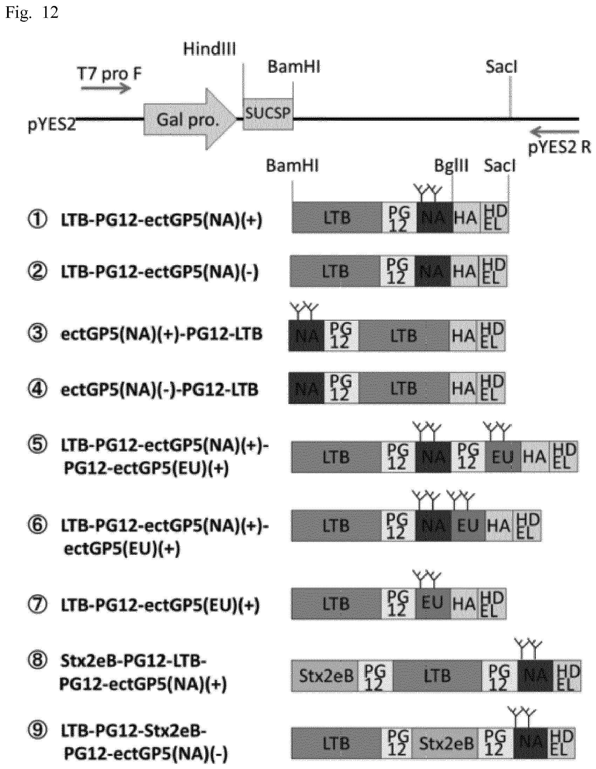

Gene constructs for the expression of recombinant proteins in yeast as shown in FIG. 12 were prepared. A secretory signal peptide of yeast invertase (SUCSP, Hashimoto et al., Protein Engineering, 1998 2;75-77) was used in order to transport the recombinant protein to the vesicular transport pathway of yeast. A DNA fragment of SUCSP was prepared as follows. A HindIII-SUCSP-F primer (5'-aaagcttaagatgcttttgcaagccttccttttcctcttggctggtttcgccgccaag-3' (SEQ ID NO: 173), the underline indicates the HindIII site) and a BamHI-SUC2SP-R primer (5'-aaggatccggcagaaatcttggcggcgaaaccagccaagag-3' (SEQ ID NO: 174), the underline indicates the BamHI site) were annealed, and then the resultant was treated with T4 DNA polymerase. The resulting fragment was then digested with HindIII and BamHI. A DNA fragment corresponding to LTB-PG12-ectGP5(NA)(+) (NA stands for North American, + indicates the presence of N-linked glycosylation site) was cleaved from ER LTB-ectGP5 (SEQ ID NO: 69) by digestion with BamHI and SacI. The thus prepared SUCSP and LTB-PG12-ectGP5(NA)(+) were ligated via the BamHI site, and the resulting fragment was inserted into the HindIII-SacI gap of pYES2 (Invitrogen), to prepare pYES2 SUCSP-LTB-PG12-ectGP5(NA)(+). The nucleotide sequence of the resulting plasmid was confirmed using a T7 promoter primer (T7 pro F) (5'-taatacgactcactataggg-3' (SEQ ID NO: 175)) and a pYES2-R primer (5'-gtaagcgtgacataactaattacatga-3' (SEQ ID NO: 176)).

A fragment of the ectGP5 of a European Type virus strain (ectGP5(EU)(+); DGSGSSSTYQYIYNLTICELNGT (SEQ ID NO: 177), the underline indicates the N-linked glycosylation sites) was prepared by performing a PCR, using an EURO type-F primer (5'-aaggatccgacggctccgggtcctcctcgacctaccagtacatctacaacttgaccatctgc-3' (SEQ ID NO: 178), the underline indicates the BamHI site) and an HDEL-SacI-R primer (5'-aagagctcacaattcatcatgttcaga-3' (SEQ ID NO: 179), the underline indicates the SacI site), and using LTB-PG12-ectGP5(NA)(+) as a template. The resulting ectGP5(EU)(+) fragment was digested with BamHI and SacI, and fused to the C terminus of LTB-PG12 or LTB-PG12-ectGP5(NA)(+), to prepare LTB-PG12-ectGP5(EU)(+) or LTB-PG12-ectGP5(NA)(+)-ectGP5(EU)(+).

The construction of LTB-PG12-ectGP5(NA)(+)-PG12-ectGP5(EU)(+) was carried out by performing an inverse PCR using LTB-PG12-ectGP5(NA)(+)-ectGP5(EU)(+) as a template, to insert PG12 between ectGP5(NA)(+) and ectGP5(EU)(+).

A fragment of LTB-PG12-ectGP5(NA)(-) in which the asparagine, to which an N-linked sugar chain is added, is replaced with serine, was prepared in the following manner. A PCR was performed using LTB-PG12-ectGP5(NA)(+) as a template, and using a LTB IF primer (5'-ttggatccgccccccagaccatcaccgagttgtgcagcgagtac-3' (SEQ ID NO: 180), the underline indicates the BamHI site) and an ectGP5(NA) deglycosylation-R primer (5'-tttagatctggtgccggacagctcgcagagggtcaaggagtagat-3' (SEQ ID NO: 181), the underline indicates the BglII site). The amplified DNA fragment was digested with BamHI and BglI, and inserted into the dephosphorylated BamHI-BglII gap of pYES2 SUCSP-LTB-PG12-ectGP5(NA)(+), to prepare LTB-PG12-ectGP5(NA)(-).

The construction of the fusion antigen in which ectGP5(NA) is added to the N terminus of LTB was performed as follows. A fragment of ectGP5(NA) to which the N-terminus side sequence of PG12 had been added, was amplified by PCR, using a HindIII-SUCSP-F primer and a PGGP5-R primer (5'-gagaaccaggaccagaaccaggggatctggtgccgttcagctcgcagagggtcaagtt-3' (SEQ ID NO: 182), the underline indicates the N-terminus side sequence of PG12, and the subsequent sequence indicates the sequence of ectGP5), and using pYES2 SUCSP-LTB-PG12-ectGP5(NA)(+) as a template. Further, a fragment of LTB to which the C-terminus side sequence of PG12 had been added, was amplified by PCR, using a PGLTB-F primer (5'-cctggttctggtcctggttctcctagatccgccccccagaccatcaccga-3' (SEQ ID NO: 183), the underline indicates the C-terminus side sequence of PG12, and the subsequent sequence indicates the sequence of the LTB region) and a LTB-BglII-R primer (5'-aaagatctgttctccatgctgatggcggcgatgctgttgggggtcttgttgttccagacgcagagctt-3' (SEQ ID NO: 184), the underline indicates the BglII site), and using pYES2 SUCSP-LTB-PG12-ectGP5(NA)(+) as a template. Both of the resulting PCR products were mixed, and annealed at the PG12 region, followed by the second PCR using a NA ectGP5-F primer (5'-aagcatgcggatccaacgccgcctcctcgtcctcctcgcac-3' (SEQ ID NO: 185), the underline indicates the BamHI site) and a LTB-BglII-R primer. The resulting DNA fragment was digested with BamHI and BglII, and inserted into the dephosphorylatted BamHI-BglII gap of pYES2 SUCSP-LTB-PG12-ectGP5(NA)(+), to prepare ectGP5(NA)(+)-PG12-LTB.

A fragment of ectGP5(NA)(-)-PG12-LTB in which a mutation had been introduced into the N-linked glycosylation site was constructed basically in the same manner as described above except that a PGGP5minusGly-R primer (5'-gagaaccaggaccagaaccaggggatctggtgccggacagctcgcagagggtcaaggagtagat-3' (SEQ ID NO: 186), the underline indicates the N-terminus side sequence of PG12, and the subsequent sequence indicates the sequence of the ectGP5 region) was used instead of the PGGP5-R primer.

Fragments of Stx2eB-PG12-LTB-PG12-ectGP5(NA)(+) and LTB-PG12-Stx2eB-PG12-ectGP5(NA)(+), each obtained by further fusing ectGP5 to the fusion protein of LTB and Stx2eB, were constructed as follows. DNA fragments of Stx2eB-PG12-LTB-PG12 and LTB-PG12-Stx2eB-PG12 were prepared according to the method disclosed in Japanese Patent Application No.2013-243932. Each of the fragment was fused with ectGP5(NA)(+), and inserted into pYES2.

The thus prepared gene constructs were each introduced into yeast cells for the expression of the respective recombinant proteins.

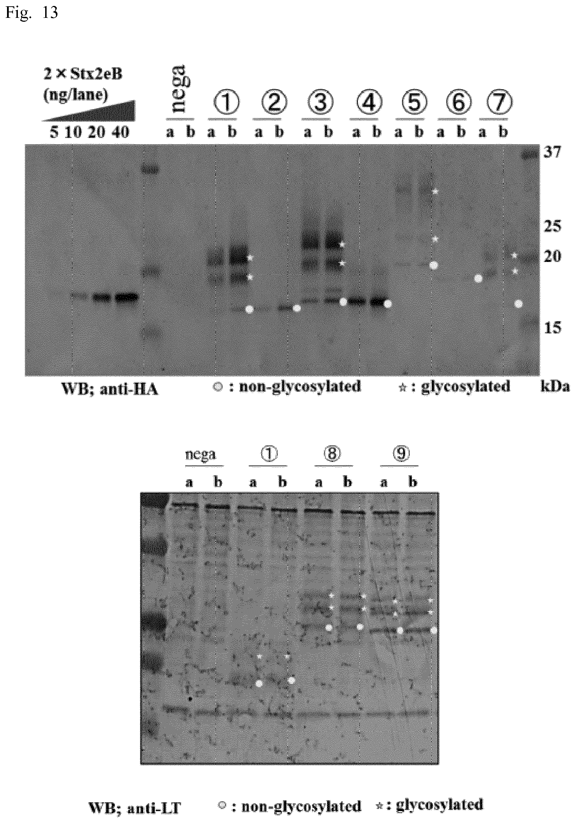

The transformation of yeast (Saccharomyces cerevisiae INVSc1, Invitrogen) was carried out according to the method described in the manual attached to S. c. EasyComp (TM) Transformation Kit (Invitrogen). The LTB fusion protein was subjected to galactose treatment for 12 hours to induce expression. After the induction by galactose, 200 µl of yeast culture liquid and 200 µl of 2 × SDS-PAGE sample buffer (EZ Apply, ATTO) were mixed, and the resultant was heat treated at 95°C for five minutes. A quantity of 4 µl of the resulting sample was subjected to SDS-PAGE. A Western analysis was carried out, using an anti-HA antibody (No. 11 867 423 001, Roche) or an anti-LT toxin antibody. Two independent clones of each of the constructs were analyzed. As a result, the expression of each of the recombinant proteins was confirmed, as shown in FIG. 13. In addition, the bands corresponding to the glycosylation products were also confirmed. However, the glycosylation products of the proteins expressed from the constructs in which a mutation had been introduced into the glycosylation site were not observed. The addition of PG12 between ectGP5(NA)(+) and ectGP5(EU)(+) resulted in an increase in the accumulated amount of the recombinant protein.

### INDUSTRIAL APPLICABILITY

The fusion protein according to the present invention is useful in the field of livestock farming.

## Claims

1. A fusion protein comprising an ectodomain (ectGP5) of Glycoprotein 5 (GP5) of porcine reproductive and respiratory syndrome (PRRS) virus, and an adjuvant protein.

2. The fusion protein according to claim 1, wherein the ectodomain has the amino acid sequence from asparagine at position 30 to threonine at position 53 in the amino acid sequence represented by SEQ ID NO: 1, or an amino acid sequence having at least 80% sequence identity to the amino acid sequence, and is capable of inducing a prophylactic effect against PRRS virus infection.

3. The fusion protein according to claim 1 or 2, wherein the GP5 is of European Type or North American Type.

4. The fusion protein according to claim 3, wherein the GP5 is of any one of Types I and II of the European Type, and Types II, III and IV of the North American Type.

5. The fusion protein according to claim 1, wherein the ectodomain has any one of the amino acid sequences represented by SEQ ID NOs: 3, 6, and 21 to 34, or an amino acid sequence having at least 80% sequence identity to the amino acid sequence represented by SEQ ID NO: 6, and is capable of inducing a prophylactic effect against PRRS virus infection.

6. The fusion protein according to any one of claims 1 to 5, wherein the adjuvant protein is a B subunit (LTB) of *Escherichia coli* heat-labile toxin and/or a B subunit (Stx2eB) of Type 2 Shiga toxin subclass e (Stx2e).

7. The fusion protein according to any one of claims 1 to 6, wherein the ectodomain and the adjuvant protein are linked via a peptide linker.

8. The fusion protein according to claim 7, wherein the number of amino acids constituting the peptide linker is from 5 to 25.

9. The fusion protein according to claim 7 or 8, wherein, the linker is PG12 (SEQ ID NO: 55), PG17 (SEQ ID NO: 57), or PG22 (SEQ ID NO: 59); or has an amino acid sequence having at least 80% sequence identity to the amino acid sequence represented by SEQ ID NO: 55, 57 or 59.

10. The fusion protein according to claim 9, wherein said fusion protein comprises the amino acid sequence represented by SEQ ID NO: 61, or an amino acid sequence having at least 80% sequence identity to the amino acid sequence represented by SEQ ID NO: 61.

11. A DNA coding for the fusion protein according to any one of claims 1 to 10.

12. A DNA construct comprising the DNA according to claim 11.

13. A DNA construct comprising a DNA coding for Glycoprotein 5 (GP5) and a DNA coding for Matrix protein (M) of porcine reproductive and respiratory syndrome (PRRS) virus, wherein each of the DNAs is linked to a promoter and a terminator.

14. The DNA construct according to claim 13, wherein the DNA coding for GP5 is a DNA coding for any one of the amino acid sequences represented by SEQ ID NOs: 1 and 7 to 20, or an amino acid sequence having at least 80% sequence identity to the amino acid sequence represented by SEQ ID NO: 1; and wherein the GP5 is capable of inducing a prophylactic effect against PRRS virus infection.

15. The DNA construct according to claim 13 or 14, wherein the DNA coding for GP5 comprises a DNA coding for the same amino acid sequence as the amino acid sequence represented by SEQ ID NO: 1 except that serine at position 32 is replaced by alanine, and aspartic acid at position 33 is replaced by serine.

16. The DNA construct according to any one of claims 13 to 15, wherein the M is of European Type or North American Type.

17. The DNA construct according to claim 16, wherein the M is of any one of Types I and II of the European Type, and Types II, III and IV of the North American Type.

18. The DNA construct according to any one of claims 13 to 15, wherein the M has any one of the amino acid sequences represented by SEQ ID NOs: 35 and 37 to 50, or an amino acid sequence having at least 80% sequence identity to the amino acid sequence represented by SEQ ID NO: 35, and is capable of inducing a prophylactic effect against PRRS virus infection.

19. A recombinant vector comprising the DNA construct according to any one of claims 12 to 18.

20. A transformant transformed with the recombinant vector according to claim 19.

21. The transformant according to claim 20, wherein the transformant is a plant.

22. An agent for controlling PRRS comprising the transformant according to claim 21, or a protein obtained therefrom.

23. A method for treating or preventing PRRS, comprising administering the agent for controlling PRRS according to claim 22 to a pig.

24. A method for expressing GP5 and M in the same cell, comprising transforming a eukaryote having a vesicular transport pathway, with a recombinant vector containing the DNA construct according to any one of claims 13 to 18.

25. A method for producing an agent for controlling PRRS, comprising transforming a eukaryote having a vesicular transport pathway, with a recombinant vector containing the DNA construct according to any one of claims 13 to 18, to express GP5 and M in the same cell.


SEQUENCE LISTING

<110>  Idemitsu Kosan Co., Ltd.

<120>  An agent for preventing and treating porcine reproductive
and respiratory syndrome

<130>  140253-15220

<150>  JP2014-163108
<151>  2014-08-08

<160>  187

<170>  PatentIn version 3.5

<210>  1
<211>  200
<212>  PRT
<213>  Porcine reproductive and respiratory syndrome virus

<400>  1
Met Leu Gly Lys Cys Leu Thr Ala Gly Cys Cys Ser Arg Leu Pro Phe
1               5                   10                  15
Leu Trp Cys Ile Val Pro Phe Cys Leu Ala Ala Leu Val Asn Ala Ser
            20                  25                  30
Asp Ser Ser Ser Ser His Leu Gln Leu Ile Tyr Asn Leu Thr Leu Cys
        35                  40                  45
Glu Leu Asn Gly Thr Asp Trp Leu Ala Asp Lys Phe Asp Trp Ala Val
    50                  55                  60
Glu Ser Phe Val Ile Phe Pro Val Leu Thr His Ile Val Ser Tyr Cys
65                  70                  75                  80
Ala Leu Thr Thr Ser His Phe Leu Asp Thr Val Gly Leu Val Ala Val
                85                  90                  95
Ser Thr Ala Gly Phe Tyr His Gly Arg Tyr Val Leu Ser Ser Ile Tyr
            100                 105                 110
Ala Val Cys Ala Leu Ala Ala Leu Val Cys Phe Val Ile Arg Leu Thr
        115                 120                 125
Lys Asn Cys Met Ser Trp Arg Tyr Ser Cys Thr Arg Tyr Thr Asn Phe
    130                 135                 140
Leu Leu Asp Thr Lys Gly Arg Leu Tyr Arg Trp Arg Ser Pro Val Ile
145                 150                 155                 160
Ile Glu Lys Gly Gly Lys Val Glu Val Glu Gly His Leu Ile Asp Leu
                165                 170                 175
Lys Arg Val Val Leu Asp Gly Ser Ala Ala Thr Pro Ile Thr Lys Val
            180                 185                 190
Ser Ala Glu Gln Trp Gly His Pro
        195                 200

<210>  2
<211>  606
<212>  DNA
<213>  Porcine reproductive and respiratory syndrome virus

<400>  2
atgttgggca agtgcttgac cgccggctgc tgctccaggc tccccttctt gtggtgcatc      60
gtgcccttct gcttggccgc cttggtgaac gcctccgact cgtcctcctc gcacctccag     120
ctcatctaca acttgaccct ctgcgagctg aacggcaccg actggttggc cgacaagttc     180
gactgggccg tggagagctt cgtgatcttc cccgtgttga cccacatcgt gagctactgc     240
gccttgacca ccagccactt cttggacacc gtgggcttgg tggccgtgag caccgccggc     300
ttctaccacg gcagatacgt gttgagcagc atctacgccg tgtgcgcctt ggccgccttg     360
gtgtgcttcg tgatcaggct caccaagaac tgcatgagct ggagatacag ctgcaccaga     420

tacaccaact tcttgttgga caccaagggc aggctctaca gatggaggag ccccgtgatc 480
atcgagaagg gcggcaaagt ggaagtggag ggccacttga tcgacttgaa gagggtggtg 540
ttggacggca gcgccgccac ccccatcacc aaagtgagcg ccgagcagtg gggccacccc 600
agatct 606

<210> 3
<211> 24
<212> PRT
<213> Porcine reproductive and respiratory syndrome virus

<400> 3
Asn Ala Ser Asp Ser Ser Ser Ser His Leu Gln Leu Ile Tyr Asn Leu
1               5                   10                  15
Thr Leu Cys Glu Leu Asn Gly Thr
            20

<210> 4
<211> 72
<212> DNA
<213> Porcine reproductive and respiratory syndrome virus

<400> 4
aacgcctccg actcgtcctc ctcgcacctc cagctcatct acaacttgac cctctgcgag 60
ctgaacggca cc 72

<210> 5
<211> 200
<212> PRT
<213> Artificial Sequence

<220>
<223> an artificially synthesized polypeptide

<400> 5
Met Leu Gly Lys Cys Leu Thr Ala Gly Cys Cys Ser Arg Leu Pro Phe
1               5                   10                  15
Leu Trp Cys Ile Val Pro Phe Cys Leu Ala Ala Leu Val Asn Ala Ala
            20                  25                  30
Ser Ser Ser Ser Ser His Leu Gln Leu Ile Tyr Asn Leu Thr Leu Cys
        35                  40                  45
Glu Leu Asn Gly Thr Asp Trp Leu Ala Asp Lys Phe Asp Trp Ala Val
    50                  55                  60
Glu Ser Phe Val Ile Phe Pro Val Leu Thr His Ile Val Ser Tyr Cys
65                  70                  75                  80
Ala Leu Thr Thr Ser His Phe Leu Asp Thr Val Gly Leu Val Ala Val
                85                  90                  95
Ser Thr Ala Gly Phe Tyr His Gly Arg Tyr Val Leu Ser Ser Ile Tyr
            100                 105                 110
Ala Val Cys Ala Leu Ala Ala Leu Val Cys Phe Val Ile Arg Leu Thr
        115                 120                 125
Lys Asn Cys Met Ser Trp Arg Tyr Ser Cys Thr Arg Tyr Thr Asn Phe
    130                 135                 140
Leu Leu Asp Thr Lys Gly Arg Leu Tyr Arg Trp Arg Ser Pro Val Ile
145                 150                 155                 160
Ile Glu Lys Gly Gly Lys Val Glu Val Glu Gly His Leu Ile Asp Leu
                165                 170                 175
Lys Arg Val Val Leu Asp Gly Ser Ala Ala Thr Pro Ile Thr Lys Val
            180                 185                 190
Ser Ala Glu Gln Trp Gly His Pro
        195                 200

<210>  6
<211>  24
<212>  PRT
<213>  Artificial Sequence

<220>
<223>  an artificially synthesized polypeptide

<400>  6
Asn Ala Ala Ser Ser Ser Ser Ser His Leu Gln Leu Ile Tyr Asn Leu
1               5                   10                  15
Thr Leu Cys Glu Leu Asn Gly Thr
            20


<210>  7
<211>  200
<212>  PRT
<213>  Porcine reproductive and respiratory syndrome virus

<400>  7
Met Leu Gly Lys Cys Leu Thr Ala Cys Cys Cys Ser Arg Leu Leu Phe
1               5                   10                  15
Leu Trp Cys Ile Val Pro Phe Tyr Leu Ala Val Leu Val Asn Ala Ser
            20                  25                  30
Asn Asn Asn Ser Ser His Ile Gln Leu Ile Tyr Asn Leu Thr Leu Cys
        35                  40                  45
Glu Leu Asn Gly Thr Asp Trp Leu Ala Gln Lys Phe Asp Trp Ala Val
    50                  55                  60
Glu Thr Phe Val Ile Phe Pro Val Leu Thr His Ile Val Ser Tyr Gly
65                  70                  75                  80
Ala Leu Thr Thr Ser His Phe Leu Asp Thr Val Gly Leu Ala Thr Val
                85                  90                  95
Ser Thr Ala Gly Tyr Tyr His Gly Arg Tyr Val Leu Ser Ser Ile Tyr
            100                 105                 110
Ala Val Cys Ala Leu Ala Ala Leu Ile Cys Phe Val Ile Arg Leu Ala
        115                 120                 125
Lys Asn Cys Met Ser Trp Arg Tyr Ser Cys Thr Arg Tyr Thr Asn Phe
    130                 135                 140
Leu Leu Asp Thr Lys Gly Arg Leu Tyr Arg Trp Arg Ser Pro Val Ile
145                 150                 155                 160
Val Glu Lys Gly Gly Lys Val Glu Val Glu Gly His Leu Ile Asp Leu
                165                 170                 175
Lys Arg Val Val Leu Asp Gly Ser Ala Ala Thr Pro Leu Thr Arg Val
            180                 185                 190
Ser Ala Glu Gln Trp Gly Arg Leu
        195                 200


<210>  8
<211>  200
<212>  PRT
<213>  Porcine reproductive and respiratory syndrome virus

<400>  8
Met Leu Gly Lys Cys Leu Thr Ala Cys Cys Cys Ser Arg Leu Leu Phe
1               5                   10                  15
Leu Trp Cys Ile Val Pro Phe Tyr Leu Ala Val Leu Ala Asn Ala Ser
            20                  25                  30
Asn Ser Asn Ser Ser His Ile Gln Leu Ile Tyr Asn Leu Thr Leu Cys
        35                  40                  45

Glu Leu Asn Gly Thr Asp Trp Leu Ala Gln Lys Phe Asp Trp Ala Val
    50                  55                  60
Glu Thr Phe Val Ile Phe Pro Val Leu Thr His Ile Val Ser Tyr Gly
65                  70                  75                  80
Ala Leu Thr Thr Ser His Phe Leu Asp Thr Val Gly Leu Ala Thr Val
                85                  90                  95
Ser Thr Ala Gly Tyr Tyr His Gly Arg Tyr Val Leu Ser Ser Ile Tyr
            100                 105                 110
Ala Val Cys Ala Leu Ala Ala Leu Ile Cys Phe Val Ile Arg Leu Ala
        115                 120                 125
Lys Asn Cys Met Ser Trp Arg Tyr Ser Cys Thr Arg Tyr Thr Asn Phe
    130                 135                 140
Leu Leu Asp Thr Lys Gly Arg Leu Tyr Arg Trp Arg Ser Pro Val Ile
145                 150                 155                 160
Val Glu Lys Gly Gly Lys Val Glu Val Glu Gly His Leu Ile Asp Leu
                165                 170                 175
Lys Arg Val Val Leu Asp Gly Ser Ala Ala Thr Pro Leu Thr Arg Val
            180                 185                 190
Ser Ala Glu Gln Trp Gly Arg Leu
        195                 200

<210>  9
<211>  200
<212>  PRT
<213>  Porcine reproductive and respiratory syndrome virus

<400>  9
Met Leu Gly Lys Cys Leu Thr Ala Cys Cys Cys Ser Arg Leu Leu Phe
1               5                   10                  15
Leu Trp Cys Ile Val Pro Phe Tyr Leu Ala Val Leu Ala Asn Ala Ser
            20                  25                  30
Asn Asn Asn Ser Ser His Ile Gln Leu Ile Tyr Asn Leu Thr Leu Cys
        35                  40                  45
Glu Leu Asn Gly Thr Asp Trp Leu Ala Gln Lys Phe Asp Trp Ala Val
    50                  55                  60
Glu Thr Phe Val Ile Phe Pro Val Leu Thr His Ile Val Ser Tyr Gly
65                  70                  75                  80
Ala Leu Thr Thr Ser His Phe Leu Asp Thr Val Gly Leu Ala Thr Val
                85                  90                  95
Ser Thr Ala Gly Tyr Tyr His Gly Arg Tyr Val Leu Ser Ser Ile Tyr
            100                 105                 110
Ala Val Cys Ala Leu Ala Ala Leu Ile Cys Phe Val Ile Arg Leu Ala
        115                 120                 125
Lys Asn Cys Met Ser Trp Arg Tyr Ser Cys Thr Arg Tyr Thr Asn Phe
    130                 135                 140
Leu Leu Asp Thr Lys Gly Arg Leu Tyr Arg Trp Arg Ser Pro Val Ile
145                 150                 155                 160
Val Glu Lys Gly Gly Lys Val Glu Val Glu Gly His Leu Ile Asp Leu
                165                 170                 175
Lys Arg Val Val Leu Asp Gly Ser Ala Ala Thr Pro Leu Thr Arg Val
            180                 185                 190
Ser Ala Glu Gln Trp Gly Arg Leu
        195                 200

<210>  10
<211>  200
<212>  PRT
<213>  Porcine reproductive and respiratory syndrome virus

<400>  10
Met Leu Gly Lys Cys Leu Thr Ala Cys Cys Cys Ser Arg Leu Leu Phe

1                   5                   10                  15
Leu Trp Cys Ile Val Pro Phe Tyr Leu Ala Val Leu Ala Asn Ala Ser
                20                  25                  30
Asn Ser Asn Ser Ser His Ile Gln Leu Ile Tyr Asn Leu Thr Leu Cys
            35                  40                  45
Glu Leu Asn Gly Thr Asp Trp Leu Ala Gln Lys Phe Asp Trp Ala Val
    50                  55                  60
Glu Thr Phe Val Ile Phe Pro Val Leu Thr His Ile Val Ser Tyr Gly
65                  70                  75                  80
Ala Leu Thr Thr Ser His Phe Leu Asp Thr Val Gly Leu Ala Thr Val
                85                  90                  95
Ser Thr Ala Gly Tyr Tyr His Gly Arg Tyr Val Leu Ser Ser Ile Tyr
            100                 105                 110
Ala Val Cys Ala Leu Ala Ala Leu Ile Cys Phe Val Ile Arg Leu Ala
        115                 120                 125
Lys Asn Cys Met Ser Trp Arg Tyr Ser Cys Thr Arg Tyr Thr Asn Phe
    130                 135                 140
Leu Leu Asp Thr Lys Gly Arg Leu Tyr Arg Trp Arg Ser Pro Val Ile
145                 150                 155                 160
Val Glu Lys Gly Gly Lys Val Glu Val Glu Gly His Leu Ile Asp Leu
                165                 170                 175
Lys Arg Val Val Leu Asp Gly Ser Ala Ala Thr Pro Leu Thr Arg Val
            180                 185                 190
Ser Ala Glu Gln Trp Gly Arg Leu
        195                 200


<210>  11
<211>  200
<212>  PRT
<213>  Porcine reproductive and respiratory syndrome virus

<400>  11
Met Leu Gly Lys Cys Leu Thr Ala Gly Cys Cys Ser Arg Leu Leu Ser
1               5                   10                  15
Leu Trp Cys Ile Val Pro Phe Tyr Phe Ala Val Leu Val Asn Ala Ser
                20                  25                  30
Asn Asn Asn Ser Ser His Ile Gln Leu Ile Tyr Asn Leu Thr Leu Cys
            35                  40                  45
Glu Leu Asn Gly Thr Asp Trp Leu Ala Gln Lys Phe Asp Trp Ala Val
    50                  55                  60
Glu Thr Phe Val Ile Phe Pro Val Leu Thr His Ile Val Ser Tyr Gly
65                  70                  75                  80
Ala Leu Thr Thr Ser His Phe Leu Asp Thr Val Gly Leu Val Thr Val
                85                  90                  95
Ser Thr Ala Gly Tyr Tyr His Arg Arg Tyr Val Leu Ser Ser Ile Tyr
            100                 105                 110
Ala Val Cys Ala Leu Ala Ala Leu Ile Cys Phe Val Ile Arg Leu Ala
        115                 120                 125
Lys Asn Cys Met Ser Trp Arg Tyr Ser Cys Thr Arg Tyr Thr Asn Phe
    130                 135                 140
Leu Leu Asp Thr Lys Gly Arg Leu Tyr Arg Trp Arg Ser Pro Val Ile
145                 150                 155                 160
Val Glu Lys Gly Gly Lys Val Glu Val Glu Gly His Leu Ile Asp Leu
                165                 170                 175
Lys Arg Val Val Leu Asp Gly Ser Val Ala Thr Pro Leu Thr Arg Val
            180                 185                 190
Ser Ala Glu Gln Trp Gly Arg Leu
        195                 200


<210>  12
<211>  200

<212>  PRT
<213>  Porcine reproductive and respiratory syndrome virus

<400>  12
Met Leu Gly Arg Cys Leu Thr Ala Gly Cys Cys Ser Arg Leu Leu Ser
1               5                   10                  15
Leu Trp Cys Ile Val Pro Phe Cys Phe Ala Ala Leu Val Asn Ala Asn
            20                  25                  30
Ser Asn Ser Ser Ser His Leu Gln Leu Ile Tyr Asn Leu Thr Leu Cys
        35                  40                  45
Glu Leu Asn Gly Thr Asp Trp Leu Lys Asp Lys Phe Asp Trp Ala Leu
    50                  55                  60
Glu Thr Phe Val Ile Phe Pro Val Leu Thr His Ile Val Ser Tyr Ser
65                  70                  75                  80
Ala Leu Thr Thr Ser His Phe Leu Asp Thr Val Gly Leu Val Thr Val
                85                  90                  95
Ser Thr Ala Gly Phe Tyr His Gly Arg Tyr Val Leu Ser Ser Ile Tyr
            100                 105                 110
Ala Val Cys Ala Leu Ala Ala Leu Thr Cys Phe Val Ile Arg Leu Ala
        115                 120                 125
Lys Asn Cys Met Ser Trp Arg Tyr Ser Cys Thr Arg Tyr Thr Asn Phe
    130                 135                 140
Leu Leu Asp Thr Lys Gly Arg Leu Tyr Arg Trp Arg Ser Pro Val Ile
145                 150                 155                 160
Ile Glu Lys Gly Gly Lys Val Glu Val Glu Gly His Leu Ile Asp Leu
                165                 170                 175
Lys Arg Val Val Leu Asp Gly Ser Val Ala Thr Pro Leu Thr Arg Val
            180                 185                 190
Ser Ala Glu Gln Trp Gly Arg Leu
        195                 200


<210>  13
<211>  201
<212>  PRT
<213>  Porcine reproductive and respiratory syndrome virus

<400>  13
Met Arg Cys Ser His Lys Leu Gly Arg Phe Leu Thr Pro His Ser Cys
1               5                   10                  15
Phe Trp Trp Leu Phe Leu Leu Cys Thr Gly Leu Ser Trp Ser Phe Ala
            20                  25                  30
Asp Gly Asn Gly Asp Ser Ser Thr Tyr Gln Tyr Ile Tyr Asn Leu Thr
        35                  40                  45
Ile Cys Glu Leu Asn Gly Thr Asp Trp Leu Ser Ser His Phe Gly Trp
    50                  55                  60
Ala Val Glu Thr Phe Val Leu Tyr Pro Val Ala Thr His Ile Leu Ser
65                  70                  75                  80
Leu Gly Phe Leu Thr Thr Ser His Phe Phe Asp Ala Leu Gly Leu Gly
                85                  90                  95
Ala Val Ser Thr Ala Gly Phe Val Gly Gly Arg Tyr Val Leu Cys Ser
            100                 105                 110
Val Tyr Gly Ala Cys Ala Phe Ala Ala Phe Val Cys Phe Val Ile Arg
        115                 120                 125
Ala Ala Lys Asn Cys Met Ala Cys Arg Tyr Ala Arg Thr Arg Phe Thr
    130                 135                 140
Asn Phe Ile Val Asp Asp Arg Gly Arg Val His Arg Trp Lys Ser Pro
145                 150                 155                 160
Ile Val Val Glu Lys Leu Gly Lys Ala Glu Val Asp Gly Asn Leu Val
                165                 170                 175
Thr Ile Lys His Val Val Leu Glu Gly Val Lys Ala Gln Pro Leu Thr
            180                 185                 190
Arg Thr Ser Ala Glu Gln Trp Glu Ala

195                 200


<210>  14
<211>  201
<212>  PRT
<213>  Porcine reproductive and respiratory syndrome virus

<400>  14
Met Arg Cys Ser Tyr Lys Leu Gly Arg Ser Leu Ile Leu His Ser Cys
1               5                   10                  15
Ser Trp Trp Phe Phe Leu Leu Cys Thr Gly Leu Ser Trp Ser Phe Ala
            20                  25                  30
Asp Gly Asn Gly Asn Asn Ser Thr Tyr Gln Tyr Ile Tyr Asn Leu Thr
        35                  40                  45
Ile Cys Glu Leu Asn Gly Thr Asn Trp Leu Ser Gly His Phe Asp Trp
    50                  55                  60
Ala Val Glu Thr Phe Val Leu Tyr Pro Val Val Thr His Ile Leu Ser
65                  70                  75                  80
Leu Gly Phe Leu Thr Thr Ser His Phe Phe Asp Ala Leu Gly Leu Gly
                85                  90                  95
Ala Val Ser Thr Ala Gly Phe Ile Asp Gly Arg Tyr Val Leu Ser Ser
            100                 105                 110
Ile Tyr Gly Ala Cys Ala Phe Ala Ala Phe Val Cys Phe Val Ile Arg
        115                 120                 125
Ala Ala Lys Asn Cys Met Ala Cys Arg Tyr Ala Arg Thr Arg Phe Thr
    130                 135                 140
Asn Phe Ile Val Asp Asp Arg Gly Gly Val His Arg Trp Lys Ser Pro
145                 150                 155                 160
Ile Val Val Glu Lys Leu Gly Lys Ala Asp Ile Asp Gly Ser Leu Val
                165                 170                 175
Thr Ile Lys His Val Val Leu Glu Gly Val Lys Ala Gln Pro Leu Thr
            180                 185                 190
Arg Thr Ser Ala Glu Gln Trp Glu Ala
        195                 200


<210>  15
<211>  201
<212>  PRT
<213>  Porcine reproductive and respiratory syndrome virus

<400>  15
Met Arg Cys Ser His Lys Leu Glu Arg Phe Leu Thr Pro His Ser Cys
1               5                   10                  15
Phe Trp Trp Leu Phe Leu Leu Cys Thr Gly Leu Ser Trp Ser Phe Val
            20                  25                  30
Asp Gly Asn Asp Ser Ser Ser Thr Tyr Gln Tyr Ile Tyr Asn Leu Thr
        35                  40                  45
Ile Cys Glu Leu Asn Gly Thr Glu Trp Leu Pro Ser His Phe Asp Trp
    50                  55                  60
Ala Val Glu Thr Phe Val Leu Tyr Pro Val Ala Thr His Ile Leu Ser
65                  70                  75                  80
Leu Gly Phe Leu Thr Thr Ser His Phe Phe Asp Ala Leu Gly Leu Gly
                85                  90                  95
Ala Val Ser Thr Thr Gly Phe Val Gly Gly Arg Tyr Val Leu Ser Ser
            100                 105                 110
Val Tyr Gly Ala Cys Ala Phe Ala Ala Leu Val Cys Phe Val Ile Arg
        115                 120                 125
Ala Ala Lys Asn Cys Met Ala Cys Arg Tyr Ala Arg Thr Arg Phe Thr
    130                 135                 140
Asn Phe Ile Val Asp Asp Arg Gly Arg Ile His Arg Trp Lys Ser Pro
145                 150                 155                 160

Ile Val Val Glu Lys Leu Gly Lys Ala Glu Val Gly Gly Asp Leu Val
                165                 170                 175
Thr Ile Lys His Val Val Leu Glu Gly Val Lys Ala Gln Pro Leu Thr
            180                 185                 190
Arg Thr Ser Ala Glu Gln Trp Glu Ala
        195                 200

<210>  16
<211>  201
<212>  PRT
<213>  Porcine reproductive and respiratory syndrome virus

<400>  16
Met Arg Cys Ser His Lys Leu Gly Arg Phe Leu Ile Pro His Ser Cys
1               5                   10                  15
Phe Trp Trp Leu Phe Leu Leu Cys Thr Gly Leu Ser Trp Ser Phe Ala
            20                  25                  30
Asp Gly Ser Gly Asn Ser Ser Thr Tyr Gln Tyr Ile Tyr Asn Leu Thr
        35                  40                  45
Ile Cys Glu Leu Asn Gly Thr Ala Trp Leu Ser Thr His Phe Pro Trp
    50                  55                  60
Val Val Glu Thr Phe Val Leu Tyr Pro Val Ala Thr His Ile Leu Ser
65                  70                  75                  80
Leu Gly Phe Leu Thr Thr Ser His Phe Phe Asp Ala Leu Gly Leu Gly
                85                  90                  95
Ala Val Phe Ile Thr Gly Phe Tyr Asp Lys Arg Tyr Val Leu Ser Ser
            100                 105                 110
Ile Tyr Gly Ala Cys Ala Leu Ala Ala Phe Val Cys Phe Ala Ile Arg
        115                 120                 125
Val Ala Lys Asn Cys Met Ala Cys Arg Tyr Ala Arg Thr Arg Phe Thr
    130                 135                 140
Asn Phe Ile Val Asp Asp Arg Gly Arg Ile His Arg Trp Lys Ser Pro
145                 150                 155                 160
Val Val Val Glu Lys Leu Gly Lys Ala Glu Ile Gly Gly Gly Leu Val
                165                 170                 175
Thr Ile Lys His Val Val Leu Glu Gly Val Lys Ala Gln Pro Leu Thr
            180                 185                 190
Arg Thr Ser Ala Glu Gln Trp Glu Ala
        195                 200

<210>  17
<211>  201
<212>  PRT
<213>  Porcine reproductive and respiratory syndrome virus

<400>  17
Met Arg Cys Ser His Lys Leu Gly Arg Phe Leu Thr Pro His Ser Cys
1               5                   10                  15
Phe Trp Trp Leu Phe Leu Leu Cys Thr Gly Leu Ser Trp Ser Phe Ala
            20                  25                  30
Asp Gly Asn Gly Asn Ser Ser Thr Tyr Gln Tyr Ile Tyr Asn Leu Thr
        35                  40                  45
Ile Cys Glu Leu Asn Gly Thr Thr Trp Leu Ser Asp His Phe Tyr Trp
    50                  55                  60
Ala Val Glu Thr Phe Val Leu Tyr Pro Val Ala Thr His Ile Leu Ser
65                  70                  75                  80
Leu Gly Phe Leu Thr Thr Ser His Leu Leu Asp Ala Leu Gly Leu Gly
                85                  90                  95
Ala Val Ser Val Ala Gly Phe His Gly Gly Arg Tyr Val Leu Ser Ser
            100                 105                 110
Val Tyr Ser Ala Cys Ala Leu Ala Ala Leu Val Cys Phe Val Ile Arg

115                 120                 125
Ala Ala Lys Asn Cys Met Ala Cys Arg Tyr Ala Arg Thr Arg Phe Thr
    130                 135                 140
Asn Phe Ile Val Asp Asp Arg Gly Arg Ile His Arg Trp Arg Ser Pro
145                 150                 155                 160
Ile Val Val Glu Arg Leu Gly Lys Ala Asp Val Ser Gly Asp Leu Val
                165                 170                 175
Thr Ile Lys His Val Val Leu Glu Gly Val Lys Ala Gln Pro Leu Thr
            180                 185                 190
Arg Thr Ser Ala Glu Gln Trp Glu Ala
        195                 200

<210>  18
<211>  201
<212>  PRT
<213>  Porcine reproductive and respiratory syndrome virus

<400>  18
Met Arg Cys Ser His Lys Leu Ala Arg Phe Leu Thr Pro His Ser Cys
1               5                   10                  15
Phe Trp Trp Leu Phe Leu Leu Cys Thr Gly Leu Ser Trp Ser Phe Ala
            20                  25                  30
Asp Gly Asn Gly Asn Ser Ser Thr Tyr Gln Tyr Ile Tyr Asn Met Thr
        35                  40                  45
Ile Cys Glu Leu Asn Gly Thr Ala Trp Leu Ser Asn Lys Phe Tyr Trp
    50                  55                  60
Ala Val Glu Thr Phe Val Leu Tyr Pro Val Val Thr His Ile Val Ser
65                  70                  75                  80
Leu Gly Phe Leu Thr Thr Ser His Leu Phe Asp Thr Leu Gly Leu Gly
                85                  90                  95
Ala Val Ser Val Thr Gly Phe Val Asn Gly Arg Tyr Val Leu Ser Ser
            100                 105                 110
Val Tyr Gly Val Cys Ala Phe Ala Ala Leu Val Cys Phe Ile Ile Arg
        115                 120                 125
Ala Ala Lys Asn Cys Met Ala Cys Arg Tyr Ala Arg Thr Arg Phe Thr
    130                 135                 140
Asn Phe Ile Val Asp Asp Arg Gly Arg Ile His Arg Trp Lys Ser Pro
145                 150                 155                 160
Ile Val Val Glu Lys Met Gly Lys Ala Glu Ile Gly Ser Asp Leu Val
                165                 170                 175
Thr Ile Lys His Val Val Phe Glu Gly Val Lys Ala Gln Pro Leu Thr
            180                 185                 190
Arg Thr Ser Ala Glu Gln Trp Glu Ala
        195                 200

<210>  19
<211>  201
<212>  PRT
<213>  Porcine reproductive and respiratory syndrome virus

<400>  19
Met Arg Cys Ser His Lys Leu Arg Arg Phe Leu Ile Pro His Ser Cys
1               5                   10                  15
Phe Trp Trp Leu Phe Leu Leu Cys Ile Gly Leu Ser Cys Ser Phe Ala
            20                  25                  30
Asp Gly Asn Gly Asn Ser Ser Thr Tyr Gln Tyr Ile Tyr Asn Leu Thr
        35                  40                  45
Ile Cys Glu Leu Asn Gly Thr Asp Trp Leu Ser Ser His Phe Asp Trp
    50                  55                  60
Ala Val Glu Thr Phe Val Leu Tyr Pro Val Ala Thr His Ile Leu Ser
65                  70                  75                  80

Leu Gly Phe Leu Thr Thr Ser His Phe Phe Asp Ala Leu Gly Leu Gly
                85                  90                  95
Ala Val Ser Ala Thr Gly Phe Ala Gly Gly Arg Tyr Val Leu Ser Ser
            100                 105                 110
Val Tyr Gly Ala Cys Ala Phe Ala Ala Phe Val Cys Phe Val Ile Arg
        115                 120                 125
Ala Ala Lys Asn Cys Met Ala Cys Arg Tyr Ala Arg Thr Arg Phe Thr
    130                 135                 140
Asn Phe Ile Val Asp Asp Arg Gly Arg Ile His Arg Trp Arg Ser Pro
145                 150                 155                 160
Ile Val Val Glu Lys Ser Gly Lys Ala Asp Val Gly Gly Asp Leu Val
                165                 170                 175
Thr Ile Lys His Val Val Leu Glu Gly Val Lys Ala Gln Pro Leu Thr
            180                 185                 190
Arg Thr Ser Ala Glu Gln Trp Glu Ala
        195                 200

<210>  20
<211>  201
<212>  PRT
<213>  Porcine reproductive and respiratory syndrome virus

<400>  20
Met Arg Cys Ser Arg Thr Leu Gly Gln Pro Ser Thr His His Ser Tyr
1               5                   10                  15
Leu Trp Trp Leu Phe Leu Leu Cys Thr Gly Leu Ser Trp Ser Phe Ala
            20                  25                  30
Asp Gly Asn Gly Asn Ser Ser Thr Tyr Leu Tyr Ile Tyr Asn Leu Thr
        35                  40                  45
Ile Cys Glu Leu Asn Gly Thr Glu Trp Leu Val Asn His Phe Asp Trp
    50                  55                  60
Ala Val Glu Ser Phe Val Phe Tyr Pro Val Val Thr His Ile Leu Ser
65                  70                  75                  80
Leu Gly Phe Leu Thr Thr Ser His Phe Phe Asp Thr Leu Gly Leu Gly
                85                  90                  95
Ala Val Ala Val Ala Gly Phe His Gly Gln Arg Tyr Val Leu Ser Ser
            100                 105                 110
Ile Tyr Gly Ile Ser Ala Leu Ala Ala Phe Val Cys Phe Ala Ile Arg
        115                 120                 125
Ala Ala Lys Asn Cys Met Ala Cys Arg Tyr Ala Cys Thr Arg Phe Thr
    130                 135                 140
Asn Phe Ile Val Asp Asp Arg Gly Arg Ile His Arg Trp Lys Ser Pro
145                 150                 155                 160
Val Val Val Glu Lys Leu Gly Lys Ala Glu Val Gly Gly Asn Leu Val
                165                 170                 175
Thr Ile Lys His Val Val Leu Glu Gly Val Lys Ala Lys Pro Leu Ala
            180                 185                 190
Arg Thr Ala Ala Glu Gln Trp Glu Ala
        195                 200

<210>  21
<211>  24
<212>  PRT
<213>  Porcine reproductive and respiratory syndrome virus

<400>  21
Asn Ala Ser Asn Asn Asn Ser Ser His Ile Gln Leu Ile Tyr Asn Leu
1               5                   10                  15
Thr Leu Cys Glu Leu Asn Gly Thr
            20

<210>  22
<211>  24
<212>  PRT
<213>  Porcine reproductive and respiratory syndrome virus

<400>  22
Asn Ala Ser Asn Ser Asn Ser Ser His Ile Gln Leu Ile Tyr Asn Leu
1               5                   10                  15
Thr Leu Cys Glu Leu Asn Gly Thr
            20

<210>  23
<211>  24
<212>  PRT
<213>  Porcine reproductive and respiratory syndrome virus

<400>  23
Asn Ala Ser Asn Asn Asn Ser Ser His Ile Gln Leu Ile Tyr Asn Leu
1               5                   10                  15
Thr Leu Cys Glu Leu Asn Gly Thr
            20

<210>  24
<211>  24
<212>  PRT
<213>  Porcine reproductive and respiratory syndrome virus

<400>  24
Asn Ala Ser Asn Ser Asn Ser Ser His Ile Gln Leu Ile Tyr Asn Leu
1               5                   10                  15
Thr Leu Cys Glu Leu Asn Gly Thr
            20

<210>  25
<211>  24
<212>  PRT
<213>  Porcine reproductive and respiratory syndrome virus

<400>  25
Asn Ala Ser Asn Asn Asn Ser Ser His Ile Gln Leu Ile Tyr Asn Leu
1               5                   10                  15
Thr Leu Cys Glu Leu Asn Gly Thr
            20

<210>  26
<211>  24
<212>  PRT
<213>  Porcine reproductive and respiratory syndrome virus

<400>  26
Asn Ala Asn Ser Asn Ser Ser Ser His Leu Gln Leu Ile Tyr Asn Leu
1               5                   10                  15
Thr Leu Cys Glu Leu Asn Gly Thr
            20

<210>  27
<211>  24
<212>  PRT

<213>  Porcine reproductive and respiratory syndrome virus

<400>  27
Ala Asp Gly Asn Gly Asp Ser Ser Thr Tyr Gln Tyr Ile Tyr Asn Leu
1               5                   10                  15
Thr Ile Cys Glu Leu Asn Gly Thr
            20

<210>  28
<211>  24
<212>  PRT
<213>  Porcine reproductive and respiratory syndrome virus

<400>  28
Ala Asp Gly Asn Gly Asn Asn Ser Thr Tyr Gln Tyr Ile Tyr Asn Leu
1               5                   10                  15
Thr Ile Cys Glu Leu Asn Gly Thr
            20

<210>  29
<211>  24
<212>  PRT
<213>  Porcine reproductive and respiratory syndrome virus

<400>  29
Val Asp Gly Asn Asp Ser Ser Ser Thr Tyr Gln Tyr Ile Tyr Asn Leu
1               5                   10                  15
Thr Ile Cys Glu Leu Asn Gly Thr
            20

<210>  30
<211>  24
<212>  PRT
<213>  Porcine reproductive and respiratory syndrome virus

<400>  30
Ala Asp Gly Ser Gly Asn Ser Ser Thr Tyr Gln Tyr Ile Tyr Asn Leu
1               5                   10                  15
Thr Ile Cys Glu Leu Asn Gly Thr
            20

<210>  31
<211>  24
<212>  PRT
<213>  Porcine reproductive and respiratory syndrome virus

<400>  31
Ala Asp Gly Asn Gly Asn Ser Ser Thr Tyr Gln Tyr Ile Tyr Asn Leu
1               5                   10                  15
Thr Ile Cys Glu Leu Asn Gly Thr
            20

<210>  32
<211>  24
<212>  PRT
<213>  Porcine reproductive and respiratory syndrome virus

<400>  32

Ala Asp Gly Asn Gly Asn Ser Ser Thr Tyr Gln Tyr Ile Tyr Asn Met
1               5                   10                  15
Thr Ile Cys Glu Leu Asn Gly Thr
            20

<210>  33
<211>  24
<212>  PRT
<213>  Porcine reproductive and respiratory syndrome virus

<400>  33
Ala Asp Gly Asn Gly Asn Ser Ser Thr Tyr Gln Tyr Ile Tyr Asn Leu
1               5                   10                  15
Thr Ile Cys Glu Leu Asn Gly Thr
            20

<210>  34
<211>  24
<212>  PRT
<213>  Porcine reproductive and respiratory syndrome virus

<400>  34
Ala Asp Gly Asn Gly Asn Ser Ser Thr Tyr Leu Tyr Ile Tyr Asn Leu
1               5                   10                  15
Thr Ile Cys Glu Leu Asn Gly Thr
            20

<210>  35
<211>  174
<212>  PRT
<213>  Porcine reproductive and respiratory syndrome virus

<400>  35
Met Gly Ser Ser Leu Asp Asp Phe Cys His Asp Ser Thr Ala Pro Gln
1               5                   10                  15
Lys Val Leu Leu Ala Phe Ser Ile Thr Tyr Thr Pro Ile Met Ile Tyr
            20                  25                  30
Ala Leu Lys Val Ser Arg Gly Arg Leu Leu Gly Leu Leu His Leu Leu
        35                  40                  45
Ile Phe Leu Asn Cys Ala Phe Thr Phe Gly Tyr Met Thr Phe Val His
    50                  55                  60
Phe Gln Ser Thr Asn Lys Val Ala Leu Thr Met Gly Ala Val Val Ala
65                  70                  75                  80
Leu Leu Trp Gly Val Tyr Ser Ala Ile Glu Thr Trp Arg Phe Ile Thr
                85                  90                  95
Ser Arg Cys Arg Leu Cys Leu Leu Gly Arg Lys Tyr Ile Leu Ala Pro
            100                 105                 110
Ala His His Val Glu Ser Ala Ala Gly Phe His Pro Ile Ala Ala Ser
        115                 120                 125
Asp Asn His Ala Phe Val Val Arg Arg Pro Gly Ser Thr Thr Val Asn
    130                 135                 140
Gly Thr Leu Val Pro Gly Leu Lys Ser Leu Val Leu Gly Gly Arg Lys
145                 150                 155                 160
Ala Val Lys Arg Gly Val Val Asn Leu Val Lys Tyr Ala Lys

                165                 170
<210>  36
<211>  528
<212>  DNA
<213>  Porcine reproductive and respiratory syndrome virus

<400>  36
atgggcagca gcttggacga cttctgccac gacagcaccg ccccccagaa agtgttgttg      60
gccttcagca tcacctacac ccccatcatg atctacgcct tgaaagtgag caggggcagg     120
ctcttgggct tgttgcactt gttgatcttc ttgaactgcg ccttcacctt cggctacatg     180
accttcgtgc acttccagag caccaacaaa gtggccttga ccatgggcgc cgtggtggcc     240
ttgttgtggg gcgtgtacag cgccatcgag acctggagat tcatcaccag cagatgcagg     300
ctctgcttgt tgggcaggaa gtacatcttg gcccccgccc accacgtgga gagcgccgcc      360
ggcttccacc ccatcgccgc cagcgacaac cacgccttcg tggtgaggag gcccggcagc     420
accaccgtga acggcacctt ggtgcccggc ttgaagagct tggtgttggg cggcaggaag     480
gccgtgaaga ggggcgtggt gaacttggtg aagtacgcca agagatct                  528

<210>  37
<211>  174
<212>  PRT
<213>  Porcine reproductive and respiratory syndrome virus

<400>  37
Met Gly Ser Ser Leu Asp Asp Phe Cys Asn Asp Ser Thr Ala Pro Gln
1               5                   10                  15
Lys Val Leu Leu Ala Phe Ser Ile Thr Tyr Thr Pro Val Met Ile Tyr
            20                  25                  30
Ala Leu Lys Val Ser Arg Gly Arg Leu Leu Gly Leu Leu His Leu Leu
        35                  40                  45
Ile Phe Leu Asn Cys Ala Phe Thr Phe Gly Tyr Met Thr Phe Val His
    50                  55                  60
Phe Glu Ser Thr Asn Arg Val Ala Leu Thr Met Gly Ala Val Val Ala
65                  70                  75                  80
Leu Leu Trp Gly Val Tyr Ser Ala Ile Glu Thr Trp Lys Phe Ile Thr
                85                  90                  95
Ser Arg Cys Arg Leu Cys Leu Leu Gly Arg Lys Tyr Ile Leu Ala Pro
            100                 105                 110
Ala His His Val Glu Ser Ala Ala Gly Phe His Pro Ile Ala Ala Asn
        115                 120                 125
Asp Asn His Ala Phe Val Val Arg Arg Pro Gly Ser Thr Thr Val Asn
    130                 135                 140
Gly Thr Leu Val Pro Gly Leu Lys Ser Leu Val Leu Gly Gly Arg Lys
145                 150                 155                 160
Ala Val Lys Gln Gly Val Val Asn Leu Val Lys Tyr Ala Lys
                165                 170

<210>  38
<211>  174
<212>  PRT
<213>  Porcine reproductive and respiratory syndrome virus

<400>  38
Met Gly Ser Ser Leu Asp Asp Phe Cys Asn Asp Ser Thr Ala Pro Gln
1               5                   10                  15
Lys Val Leu Leu Ala Phe Ser Ile Thr Tyr Thr Pro Val Met Ile Tyr
            20                  25                  30
Ala Leu Lys Val Ser Arg Gly Arg Leu Leu Gly Leu Leu His Leu Leu
        35                  40                  45
Ile Phe Leu Asn Cys Ala Phe Thr Phe Gly Tyr Met Thr Phe Val His
    50                  55                  60
Phe Glu Ser Thr Asn Arg Val Ala Leu Thr Met Gly Ala Val Val Ala
65                  70                  75                  80
Leu Leu Trp Gly Val Tyr Ser Ala Ile Glu Thr Trp Lys Phe Ile Thr
                85                  90                  95
Ser Arg Cys Arg Leu Cys Leu Leu Gly Arg Lys Tyr Ile Leu Ala Pro
            100                 105                 110

Ala His His Val Glu Ser Ala Ala Gly Phe His Pro Ile Ala Ala Asn
        115                 120                 125
Asp Asn His Ala Phe Val Val Arg Arg Pro Gly Ser Thr Thr Val Asn
    130                 135                 140
Gly Thr Leu Val Pro Gly Leu Lys Ser Leu Val Leu Gly Gly Arg Lys
145                 150                 155                 160
Ala Val Lys Gln Gly Val Val Asn Leu Val Lys Tyr Ala Lys
                165                 170

<210>  39
<211>  174
<212>  PRT
<213>  Porcine reproductive and respiratory syndrome virus

<400>  39
Met Gly Ser Ser Leu Asp Asp Phe Cys Asn Asp Ser Thr Ala Pro Gln
1               5                   10                  15
Lys Val Leu Leu Ala Phe Ser Ile Thr Tyr Thr Pro Val Met Ile Tyr
            20                  25                  30
Ala Leu Lys Val Ser Arg Gly Arg Leu Leu Gly Leu Leu His Leu Leu
        35                  40                  45
Ile Phe Leu Asn Cys Ala Phe Thr Phe Gly Tyr Met Thr Phe Val His
    50                  55                  60
Phe Glu Ser Thr Asn Arg Val Ala Leu Thr Met Gly Ala Val Val Ala
65                  70                  75                  80
Leu Leu Trp Gly Val Tyr Ser Ala Ile Glu Thr Trp Lys Phe Ile Thr
                85                  90                  95
Ser Arg Cys Arg Leu Cys Leu Leu Gly Arg Lys Tyr Ile Leu Ala Pro
            100                 105                 110
Ala His His Val Glu Ser Ala Ala Gly Phe His Pro Ile Ala Ala Asn
        115                 120                 125
Asp Asn His Ala Phe Val Val Arg Arg Pro Gly Ser Thr Thr Val Asn
    130                 135                 140
Gly Thr Leu Val Pro Gly Leu Lys Ser Leu Val Leu Gly Gly Arg Lys
145                 150                 155                 160
Ala Val Lys Gln Gly Val Val Asn Leu Val Lys Tyr Ala Lys
                165                 170

<210>  40
<211>  174
<212>  PRT
<213>  Porcine reproductive and respiratory syndrome virus

<400>  40
Met Gly Ser Ser Leu Asp Asp Phe Cys Asn Asp Ser Thr Ala Pro Gln
1               5                   10                  15
Lys Val Leu Leu Ala Phe Ser Ile Thr Tyr Thr Pro Val Met Ile Tyr
            20                  25                  30
Ala Leu Lys Val Ser Arg Gly Arg Leu Leu Gly Leu Leu His Leu Leu
        35                  40                  45
Ile Phe Leu Asn Cys Ala Phe Thr Phe Gly Tyr Met Thr Phe Val His
    50                  55                  60
Phe Glu Ser Thr Asn Arg Val Ala Leu Thr Met Gly Ala Val Val Ala
65                  70                  75                  80
Leu Leu Trp Gly Val Tyr Ser Ala Ile Glu Thr Trp Lys Phe Ile Thr
                85                  90                  95
Ser Arg Cys Arg Leu Cys Leu Leu Gly Arg Lys Tyr Ile Leu Ala Pro
            100                 105                 110
Ala His His Val Glu Ser Ala Ala Gly Phe His Pro Ile Ala Ala Asn
        115                 120                 125
Asp Asn His Ala Phe Val Val Arg Arg Pro Gly Ser Thr Thr Val Asn

130                 135                 140
Gly Thr Leu Val Pro Gly Leu Lys Ser Leu Val Leu Gly Gly Arg Lys
145                 150                 155                 160
Ala Val Lys Gln Gly Val Val Asn Leu Val Lys Tyr Ala Lys
                165                 170

<210>  41
<211>  174
<212>  PRT
<213>  Porcine reproductive and respiratory syndrome virus

<400>  41
Met Gly Ser Ser Leu Asp Asp Phe Cys Asn Asp Ser Thr Ala Pro Gln
1               5                   10                  15
Lys Val Leu Leu Ala Phe Ser Ile Thr Tyr Thr Pro Val Met Ile Tyr
            20                  25                  30
Ala Leu Lys Val Ser Arg Gly Arg Leu Leu Gly Leu Leu His Leu Leu
        35                  40                  45
Ile Phe Leu Asn Cys Ala Phe Thr Phe Gly Tyr Met Thr Phe Val His
    50                  55                  60
Phe Gln Ser Thr Asn Arg Val Ala Leu Thr Met Gly Ala Val Val Ala
65                  70                  75                  80
Leu Leu Trp Gly Val Tyr Ser Ala Ile Glu Thr Trp Lys Phe Ile Thr
                85                  90                  95
Ser Arg Cys Arg Leu Cys Leu Leu Gly Arg Lys Tyr Ile Leu Ala Pro
            100                 105                 110
Ala His His Val Glu Ser Ala Ala Gly Phe His Pro Ile Ala Ala Asn
        115                 120                 125
Asp Asn His Ala Phe Val Val Arg Arg Pro Gly Ser Thr Thr Val Asn
    130                 135                 140
Gly Thr Leu Val Pro Gly Leu Lys Ser Leu Val Leu Gly Gly Arg Lys
145                 150                 155                 160
Ala Val Lys Gln Gly Val Val Asn Leu Val Lys Tyr Ala Lys
                165                 170

<210>  42
<211>  174
<212>  PRT
<213>  Porcine reproductive and respiratory syndrome virus

<400>  42
Met Gly Ser Ser Leu Asp Asp Phe Cys Tyr Asp Ser Thr Ala Pro Gln
1               5                   10                  15
Lys Val Leu Leu Ala Phe Ser Ile Thr Tyr Thr Pro Val Met Ile Tyr
            20                  25                  30
Ala Leu Lys Val Ser Arg Gly Arg Leu Leu Gly Leu Leu His Leu Leu
        35                  40                  45
Ile Phe Leu Asn Cys Thr Phe Thr Phe Gly Tyr Met Thr Cys Val His
    50                  55                  60
Phe Asn Ser Thr Asn Lys Val Ala Leu Thr Met Gly Ala Val Val Ala
65                  70                  75                  80
Leu Leu Trp Gly Val Tyr Ser Ala Ile Glu Thr Trp Lys Phe Ile Thr
                85                  90                  95
Ser Arg Cys Arg Leu Cys Leu Leu Gly Arg Lys Tyr Ile Leu Ala Pro
            100                 105                 110
Ala His His Val Glu Ser Ala Ala Gly Phe His Pro Ile Ala Ala Asn
        115                 120                 125
Asp Asn His Ala Phe Val Val Arg Arg Pro Gly Ser Thr Thr Val Asn
    130                 135                 140
Gly Thr Leu Val Pro Gly Leu Lys Ser Leu Val Leu Gly Gly Arg Lys
145                 150                 155                 160

Ala Val Lys Gln Gly Val Val Asn Leu Val Lys Tyr Ala Lys
                165                 170

<210>  43
<211>  173
<212>  PRT
<213>  Porcine reproductive and respiratory syndrome virus

<400>  43
Met Gly Gly Leu Asp Asp Phe Cys Asn Asp Pro Ile Ala Ala Gln Lys
1               5                   10                  15
Leu Val Leu Ala Phe Ser Ile Thr Tyr Thr Pro Ile Met Ile Tyr Ala
            20                  25                  30
Leu Lys Val Ser Arg Gly Arg Leu Leu Gly Leu Leu His Ile Leu Ile
        35                  40                  45
Phe Leu Asn Cys Ser Phe Thr Phe Gly Tyr Met Thr Tyr Val His Phe
    50                  55                  60
Gln Ser Thr Asn Arg Val Ala Leu Thr Leu Gly Ala Val Val Ala Leu
65                  70                  75                  80
Leu Trp Gly Val Tyr Ser Phe Thr Glu Ser Trp Lys Phe Ile Thr Ser
                85                  90                  95
Arg Cys Arg Leu Cys Cys Leu Gly Arg Arg Tyr Ile Leu Ala Pro Ala
            100                 105                 110
His His Val Glu Ser Ala Ala Gly Leu His Ser Ile Ser Ala Ser Gly
        115                 120                 125
Asn Arg Ala Tyr Ala Val Arg Lys Pro Gly Leu Thr Ser Val Asn Gly
    130                 135                 140
Thr Leu Val Pro Gly Leu Arg Ser Leu Val Leu Gly Gly Lys Arg Ala
145                 150                 155                 160
Val Lys Arg Gly Val Val Asn Leu Val Lys Tyr Gly Arg
                165                 170


<210>  44
<211>  173
<212>  PRT
<213>  Porcine reproductive and respiratory syndrome virus

<400>  44
Met Gly Gly Leu Asp Asp Phe Cys Asn Asp Pro Thr Ala Ala Gln Lys
1               5                   10                  15
Leu Val Leu Ala Phe Ser Ile Thr Tyr Thr Pro Ile Met Ile Tyr Ala
            20                  25                  30
Leu Lys Val Ser Arg Gly Arg Leu Leu Gly Leu Leu His Ile Leu Ile
        35                  40                  45
Phe Leu Asn Cys Ser Phe Thr Phe Gly Tyr Met Thr Tyr Val His Phe
    50                  55                  60
Gln Ser Ala Asn Arg Val Ala Leu Thr Leu Gly Ala Val Val Ala Leu
65                  70                  75                  80
Leu Trp Gly Val Tyr Ser Leu Thr Glu Ser Trp Lys Phe Ile Thr Ser
                85                  90                  95
Arg Cys Arg Leu Cys Cys Leu Gly Arg Arg Tyr Ile Leu Ala Pro Ala
            100                 105                 110
His His Val Glu Ser Ala Ala Gly Leu His Ser Ile Ser Ala Ser Gly
        115                 120                 125
Asn Arg Ala Tyr Ala Val Arg Lys Pro Gly Leu Thr Ser Val Asn Gly
    130                 135                 140
Thr Leu Val Pro Gly Leu Arg Ser Leu Val Leu Gly Gly Lys Arg Ala
145                 150                 155                 160
Val Lys Arg Gly Val Val Asn Leu Val Lys Tyr Gly Arg
                165                 170

<210>  45
<211>  173
<212>  PRT
<213>  Porcine reproductive and respiratory syndrome virus

<400>  45
Met Gly Ser Leu Asp Asp Phe Cys Asn Asp Pro Thr Ala Ala Gln Lys
1               5                   10                  15
Leu Val Leu Ala Phe Ser Ile Thr Tyr Thr Pro Ile Met Ile Tyr Ala
            20                  25                  30
Leu Lys Val Ser Arg Gly Arg Leu Leu Gly Leu Leu His Ile Leu Ile
        35                  40                  45
Phe Leu Asn Cys Ser Phe Thr Phe Gly Tyr Met Thr Tyr Val His Phe
    50                  55                  60
Gln Ser Thr Asn Arg Val Ala Phe Thr Leu Gly Ala Val Val Ala Leu
65                  70                  75                  80
Leu Trp Gly Val Tyr Ser Phe Thr Glu Ser Trp Lys Phe Ile Thr Ser
                85                  90                  95
Arg Cys Arg Leu Cys Cys Leu Gly Arg Gln Tyr Ile Leu Ala Pro Ala
            100                 105                 110
His His Val Glu Ser Ala Ala Gly Leu His Ser Ile Pro Ala Ser Gly
        115                 120                 125
Asn Arg Ala Tyr Ala Val Arg Lys Pro Gly Leu Thr Ser Val Asn Gly
    130                 135                 140
Thr Leu Val Pro Gly Leu Arg Ser Leu Val Leu Gly Gly Lys Arg Ala
145                 150                 155                 160
Val Lys Arg Gly Val Val Asn Leu Val Lys Tyr Gly Arg
                165                 170

<210>  46
<211>  173
<212>  PRT
<213>  Porcine reproductive and respiratory syndrome virus

<400>  46
Met Gly Ser Leu Asp Gly Phe Cys Asp Glu Pro Ala Ala Val Gln Lys
1               5                   10                  15
Leu Val Leu Ala Phe Ser Thr Thr Tyr Thr Pro Ile Met Ile Tyr Ala
            20                  25                  30
Leu Lys Val Ser Arg Gly Arg Leu Leu Gly Leu Leu His Ile Leu Ile
        35                  40                  45
Phe Leu Asn Cys Ser Phe Thr Phe Gly Tyr Met Thr Tyr Val His Phe
    50                  55                  60
Gln Ser Ile Asn Arg Val Ala Phe Thr Leu Gly Ala Val Val Ala Leu
65                  70                  75                  80
Leu Trp Gly Val Tyr Ser Phe Thr Glu Ser Trp Lys Ser Ile Thr Ser
                85                  90                  95
Arg Cys Arg Leu Cys Cys Leu Gly Arg Arg Tyr Ile Leu Ala Pro Ala
            100                 105                 110
His His Val Glu Ser Ala Ala Gly Leu His Ser Ile Pro Ala Ser Gly
        115                 120                 125
Asn Arg Ala Tyr Ala Val Arg Lys Pro Gly Leu Thr Ser Val Asn Gly
    130                 135                 140
Thr Leu Val Pro Gly Leu Arg Ser Leu Val Leu Gly Gly Lys Arg Ala
145                 150                 155                 160
Val Lys Arg Gly Val Val Asn Leu Val Lys Tyr Gly Arg
                165                 170

<210>  47
<211>  173
<212>  PRT

<213>  Porcine reproductive and respiratory syndrome virus

<400>  47
Met Gly Ser Leu Asp Asp Phe Cys Gly Asp Pro Thr Ala Ala Gln Lys
1               5                   10                  15
Leu Val Leu Ala Phe Ser Ile Thr Tyr Thr Pro Ile Met Ile Tyr Ala
            20                  25                  30
Leu Lys Val Ser Arg Gly Arg Leu Leu Gly Leu Leu His Ile Leu Ile
        35                  40                  45
Phe Leu Asn Cys Ser Phe Thr Phe Gly Tyr Met Thr Tyr Val His Phe
    50                  55                  60
Glu Ser Thr Asn Arg Val Ala Phe Thr Met Gly Ala Val Val Ala Leu
65                  70                  75                  80
Leu Trp Gly Val Tyr Ser Phe Ile Glu Ser Trp Lys Phe Ile Thr Ser
                85                  90                  95
Arg Cys Arg Leu Cys Cys Leu Gly Arg Arg Tyr Ile Leu Ala Pro Ala
            100                 105                 110
His His Val Glu Ser Ala Ala Gly Leu His Pro Ile Pro Ala Ser Gly
        115                 120                 125
Asn His Ala Tyr Ala Val Arg Lys Pro Gly Leu Thr Ser Val Asn Gly
    130                 135                 140
Thr Leu Val Pro Gly Leu Arg Gly Leu Val Leu Gly Gly Lys Arg Ala
145                 150                 155                 160
Val Arg Arg Gly Val Val Asn Leu Val Lys Tyr Gly Arg
                165                 170


<210>  48
<211>  173
<212>  PRT
<213>  Porcine reproductive and respiratory syndrome virus

<400>  48
Met Gly Ser Leu Asp Asp Phe Cys Phe Asp His Thr Ala Ala Gln Lys
1               5                   10                  15
Leu Val Leu Ala Phe Ser Ile Thr Tyr Thr Pro Ile Met Ile Tyr Ala
            20                  25                  30
Leu Lys Val Ser Arg Gly Arg Leu Leu Gly Leu Leu His Ile Leu Ile
        35                  40                  45
Phe Leu Asn Cys Ala Phe Thr Phe Gly Tyr Met Thr His Val His Phe
    50                  55                  60
Glu Ser Thr Asn Arg Val Ala Phe Thr Met Gly Ala Val Val Ala Leu
65                  70                  75                  80
Leu Trp Gly Ile Tyr Ser Phe Ile Glu Ser Trp Lys Phe Ile Thr Ser
                85                  90                  95
Arg Cys Arg Leu Cys Cys Leu Gly Arg Arg Tyr Ile Leu Ala Pro Ala
            100                 105                 110
His His Val Glu Ser Ala Ala Gly Leu His Ser Ile Pro Ala Ser Gly
        115                 120                 125
Asn Arg Ala Tyr Ala Val Arg Lys Pro Gly Leu Thr Ser Val Asn Gly
    130                 135                 140
Thr Leu Val Pro Gly Leu Arg Ser Leu Val Leu Gly Gly Lys Arg Ala
145                 150                 155                 160
Val Lys Arg Gly Val Val Asn Leu Val Lys Tyr Gly Arg
                165                 170


<210>  49
<211>  173
<212>  PRT
<213>  Porcine reproductive and respiratory syndrome virus

<400>  49

Met Gly Gly Leu Asp Asp Phe Cys Tyr Asp Ser Thr Ala Val Gln Lys
1               5                   10                  15
Leu Val Leu Ala Phe Ser Ile Thr Tyr Thr Pro Ile Met Ile Tyr Ala
            20                  25                  30
Leu Lys Val Ser Arg Gly Arg Leu Leu Gly Leu Leu His Ile Leu Ile
        35                  40                  45
Phe Leu Asn Cys Ser Phe Thr Phe Gly Tyr Met Thr Tyr Val His Phe
    50                  55                  60
Ser Ser Thr Ser Arg Val Ala Phe Thr Met Gly Ala Val Val Ala Leu
65                  70                  75                  80
Leu Trp Gly Val Tyr Ser Phe Ile Glu Ser Trp Lys Phe Ile Thr Ser
                85                  90                  95
Arg Cys Arg Leu Cys Cys Leu Gly Arg Arg Tyr Ile Leu Ala Pro Ala
            100                 105                 110
His His Val Glu Ser Ala Ala Gly Leu His Pro Ile Pro Ala Ser Gly
        115                 120                 125
Asn Gln Ala Tyr Ala Val Arg Lys Pro Gly Leu Thr Ser Val Asn Gly
    130                 135                 140
Thr Leu Val Pro Gly Leu Arg Gly Leu Val Leu Gly Gly Lys Arg Ala
145                 150                 155                 160
Val Lys Arg Gly Val Val Asn Leu Val Lys Tyr Gly Arg
                165                 170


<210>  50
<211>  173
<212>  PRT
<213>  Porcine reproductive and respiratory syndrome virus

<400>  50
Met Gly Gly Ile Asp Gly Phe Cys His Asp Pro Thr Ala Ala Gln Lys
1               5                   10                  15
Val Val Leu Ala Phe Ser Ile Thr Tyr Thr Pro Ile Met Ile Tyr Ala
            20                  25                  30
Leu Lys Val Ser Arg Gly Arg Leu Leu Gly Leu Leu His Ile Leu Ile
        35                  40                  45
Phe Leu Asn Cys Ser Phe Thr Phe Gly Tyr Met Thr Tyr Val His Phe
    50                  55                  60
Lys Ser Thr Asn Arg Val Ala Leu Thr Met Gly Ala Ile Val Ala Leu
65                  70                  75                  80
Leu Trp Gly Ile Tyr Ser Phe Ile Glu Ser Trp Lys Phe Ile Thr Ser
                85                  90                  95
Arg Cys Arg Leu Cys Cys Leu Gly Arg Arg Tyr Ile Leu Ala Pro Ala
            100                 105                 110
His His Val Glu Ser Ala Ala Gly Leu His Ser Ile Pro Ala Ser Gly
        115                 120                 125
Asn Gln Ala Tyr Ala Val Arg Lys Pro Gly Leu Thr Ser Val Asn Gly
    130                 135                 140
Thr Leu Val Pro Gly Leu Arg Ser Leu Val Leu Gly Gly Lys Arg Ala
145                 150                 155                 160
Val Lys Arg Gly Val Val Asn Leu Val Lys Tyr Gly Arg
                165                 170


<210>  51
<211>  178
<212>  PRT
<213>  Porcine reproductive and respiratory syndrome virus

<400>  51
Met Ala Ala Ser Leu Leu Phe Leu Leu Val Gly Phe Glu Cys Phe Leu
1               5                   10                  15
Val Ser Gln Ala Phe Ala Cys Lys Pro Cys Phe Ser Ser Ser Leu Ser

                20                  25                  30
Asp Ile Lys Thr Asn Thr Thr Ala Ala Ala Ser Phe Ala Val Leu Gln
        35                  40                  45
Asp Ile Ser Cys Leu Arg His Gly Asp Ser Ser Pro Gln Thr Ile Arg
    50                  55                  60
Lys Ser Arg Gln Cys Arg Thr Ala Ile Gly Thr Pro Val Tyr Ile Thr
65                  70                  75                  80
Ile Thr Ala Asn Val Thr Asp Glu Asn Tyr Leu His Ser Ser Asp Leu
                85                  90                  95
Leu Met Leu Ser Ser Cys Leu Phe Tyr Ala Ser Glu Met Ser Glu Lys
            100                 105                 110
Gly Phe Lys Val Val Phe Gly Asn Val Ser Gly Ile Val Ala Val Cys
        115                 120                 125
Val Asn Phe Thr Ser Tyr Val Gln His Val Arg Glu Phe Thr Gln Arg
    130                 135                 140
Ser Leu Val Val Asp His Val Arg Leu Leu His Phe Met Thr Pro Glu
145                 150                 155                 160
Thr Met Arg Trp Ala Thr Val Leu Ala Cys Leu Phe Ala Ile Leu Leu
                165                 170                 175
Ala Ile

<210>  52
<211>  542
<212>  DNA
<213>  Porcine reproductive and respiratory syndrome virus

<400>  52
atggccgcca gcttgttgtt cttgttggtg ggcttcgagt gcttcttggt gagccaggcc      60
ttcgcctgca agccctgctt cagcagcagc ttgagcgaca tcaagaccaa caccaccgcc     120
gccgccagct tcgccgtgtt gcaggacatc agctgcttga ggcacggcga cagcagcccc     180
cagaccatca ggaagagcag gcagtgcagg accgccatcg gcacccccgt gtacatcacc     240
atcaccgcca acgtgaccga cgagaactac ttgcacagca gcgacttgtt gatgttgagc     300
agctgcttgt tctacgccag cgagatgagc gagaagggct tcaaagtggt gttcggcaac     360
gtgagcggca tcgtggccgt gtgcgtgaac ttcaccagct acgtgcagca cgtgagggag     420
ttcacccaga ggagcttggt ggtggaccac gtgaggctct tgcacttcat gacccccgag     480
accatgagat gggccaccgt gttggcctgc ttgttcgcca tcttgttggc catcagatct     540
aa                                                                    542

<210>  53
<211>  103
<212>  PRT
<213>  Escherichia coli

<400>  53
Ala Pro Gln Thr Ile Thr Glu Leu Cys Ser Glu Tyr Arg Asn Thr Gln
1               5                   10                  15
Ile Tyr Thr Ile Asn Asp Lys Ile Leu Ser Tyr Thr Glu Ser Met Ala
            20                  25                  30
Gly Lys Arg Glu Met Val Ile Ile Thr Phe Lys Ser Gly Glu Thr Phe
        35                  40                  45
Gln Val Glu Val Pro Gly Ser Gln His Ile Asp Ser Gln Lys Lys Ala
    50                  55                  60
Ile Glu Arg Met Lys Asp Thr Leu Arg Ile Thr Tyr Leu Thr Glu Thr
65                  70                  75                  80
Lys Ile Asp Lys Leu Cys Val Trp Asn Asn Lys Thr Pro Asn Ser Ile
                85                  90                  95
Ala Ala Ile Ser Met Glu Asn
            100

<210>  54
<211>  309
<212>  DNA
<213>  Escherichia coli

<400>  54
gccccccaga ccatcaccga gttgtgcagc gagtaccgca acacccaaat ctacaccatc      60
aacgacaaga tcctcagcta caccgagagc atggccggca agagggagat ggtgatcatc     120
accttcaaga gcggcgagac cttccaggtc gaggtccccg gcagccagca catcgacagc     180
cagaagaagg ccatcgagag gatgaaggac accctcagga tcacctacct caccgagacc     240
aagatcgaca agctctgcgt ctggaacaac aagaccccca acagcatcgc cgccatcagc     300
atggagaac                                                             309


<210>  55
<211>  12
<212>  PRT
<213>  Artificial Sequence

<220>
<223>  an artificially synthesized peptide

<400>  55
Arg Ser Pro Gly Ser Gly Pro Gly Ser Pro Arg Ser
1               5                   10


<210>  56
<211>  36
<212>  DNA
<213>  Artificial Sequence

<220>
<223>  an artificially synthesized DNA

<400>  56
agatcccctg gttctggtcc tggttctcct agatcc                                36


<210>  57
<211>  17
<212>  PRT
<213>  Artificial Sequence

<220>
<223>  an artificially synthesized peptide

<400>  57
Arg Ser Pro Gly Ser Gly Pro Gly Ser Pro Arg Ser Pro Gly Ser Arg
1               5                   10                  15
Ser


<210>  58
<211>  51
<212>  DNA
<213>  Artificial Sequence

<220>
<223>  an artificially synthesized DNA

<400>  58

agatcccctg gttctggtcc tggttctcct agatcccctg gttccagatc t          51

<210>  59
<211>  22
<212>  PRT
<213>  Artificial Sequence

<220>
<223>  an artificially synthesized peptide

<400>  59
Arg Ser Pro Gly Ser Gly Pro Gly Ser Pro Arg Ser Pro Gly Ser Gly
1               5                   10                  15
Pro Gly Ser Pro Arg Ser
            20

<210>  60
<211>  66
<212>  DNA
<213>  Artificial Sequence

<220>
<223>  an artificially synthesized DNA

<400>  60
agatcccctg gttctggtcc tggttctcct agatcccctg gttctggtcc tggttctcct      60
agatct                                                                 66

<210>  61
<211>  139
<212>  PRT
<213>  Artificial Sequence

<220>
<223>  an artificially synthesized polypeptide

<400>  61
Ala Pro Gln Thr Ile Thr Glu Leu Cys Ser Glu Tyr Arg Asn Thr Gln
1               5                   10                  15
Ile Tyr Thr Ile Asn Asp Lys Ile Leu Ser Tyr Thr Glu Ser Met Ala
            20                  25                  30
Gly Lys Arg Glu Met Val Ile Ile Thr Phe Lys Ser Gly Glu Thr Phe
        35                  40                  45
Gln Val Glu Val Pro Gly Ser Gln His Ile Asp Ser Gln Lys Lys Ala
    50                  55                  60
Ile Glu Arg Met Lys Asp Thr Leu Arg Ile Thr Tyr Leu Thr Glu Thr
65                  70                  75                  80
Lys Ile Asp Lys Leu Cys Val Trp Asn Ser Lys Thr Pro Asn Ser Ile
                85                  90                  95
Ala Ala Ile Ser Met Glu Asn Arg Ser Pro Gly Ser Gly Pro Gly Ser
            100                 105                 110
Pro Arg Ser Asn Ala Ala Ser Ser Ser Ser Ser His Leu Gln Leu Ile
        115                 120                 125
Tyr Asn Leu Thr Leu Cys Glu Leu Asn Gly Thr
    130                 135

<210>  62
<211>  417
<212>  DNA

<213>  Artificial Sequence

<220>
<223>  artificially synthesized polynucleotide

<400>  62
gccccccaga ccatcaccga gttgtgcagc gagtaccgca acacccaaat ctacaccatc      60
aacgacaaga tcctcagcta caccgagagc atggccggca agagggagat ggtgatcatc     120
accttcaaga gcggcgagac cttccaggtc gaggtccccg gcagccagca catcgacagc     180
cagaagaagg ccatcgagag gatgaaggac accctcagga tcacctacct caccgagacc     240
aagatcgaca agctctgcgt ctggaacagc aagaccccca acagcatcgc cgccatcagc     300
atggagaaca gatcccctgg ttctggtcct ggttctccta gatctaacgc cgcctcctcg     360
tcctcctcgc acctccagct catctacaac ttgaccctct gcgagctgaa cggcacc        417


<210>  63
<211>  24
<212>  PRT
<213>  Nicotiana tabacum

<400>  63
Met Gly Arg Met Ser Ile Pro Met Met Gly Phe Val Val Leu Cys Leu
1               5                   10                  15
Trp Ala Val Val Ala Glu Gly Ser
            20


<210>  64
<211>  72
<212>  DNA
<213>  Nicotiana tabacum

<400>  64
atggggagaa tgtcaatacc catgatgggt tttgtggtgt tatgtctatg ggcagtggta      60
gcagaaggat cc                                                          72


<210>  65
<211>  4
<212>  PRT
<213>  Artificial Sequence

<220>
<223>  an artificially synthesized peptide

<400>  65
His Asp Glu Leu
1


<210>  66
<211>  7
<212>  PRT
<213>  Artificial Sequence

<220>
<223>  an artificially synthesized peptide

<400>  66
Asp Leu Leu Val Asp Thr Met
1               5

<210>  67
<211>  91
<212>  DNA
<213>  Nicotiana tabacum

<400>  67
tatttaactc agtattcaga aacaacaaaa gttcttctct acataaaatt ttcctatttt      60
agtgatcagt gaaggaaatc aagaaaaata a                                     91


<210>  68
<211>  91
<212>  DNA
<213>  Nicotiana tabacum

<400>  68
tatttaactc agtattcaga aacaacaaaa gttcttctct acataaaatt ttcctatttt      60
agtgatcagt gaaggaaatc aagaaaaaaa g                                     91


<210>  69
<211>  640
<212>  DNA
<213>  Artificial Sequence

<220>
<223>  an artificially synthesized DNA construct

<400>  69
tatttaactc agtattcaga aacaacaaaa gttcttctct acataaaatt ttcctatttt      60
agtgatcagt gaaggaaatc aagaaaaaaa gatggggaga atgtcaatac ccatgatggg     120
ttttgtggtg ttatgtctat gggcagtggt agcagaagga ggatccgccc cccagaccat     180
caccgagttg tgcagcgagt accgcaacac ccaaatctac accatcaacg acaagatcct     240
cagctacacc gagagcatgg ccggcaagag ggagatggtg atcatcacct tcaagagcgg     300
cgagaccttc caggtcgagg tccccggcag ccagcacatc gacagccaga agaaggccat     360
cgagaggatg aaggacaccc tcaggatcac ctacctcacc gagaccaaga tcgacaagct     420
ctgcgtctgg aacagcaaga cccccaacag catcgccgcc atcagcatgg agaacagatc     480
ccctggttct ggtcctggtt ctcctagatc taacgccgcc tcctcgtcct cctcgcacct     540
ccagctcatc tacaacttga ccctctgcga gctgaacggc accagatctt atccttatga     600
ttatcctgat tatgctggat ctgaacatga tgaattgtga                           640


<210>  70
<211>  625
<212>  DNA
<213>  Artificial Sequence

<220>
<223>  an artificially synthesized DNA construct

<400>  70
tatttaactc agtattcaga aacaacaaaa gttcttctct acataaaatt ttcctatttt      60
agtgatcagt gaaggaaatc aagaaaaaaa gatggggaga atgtcaatac ccatgatggg     120
ttttgtggtg ttatgtctat gggcagtggt agcagaagga ggatccgccc cccagaccat     180
caccgagttg tgcagcgagt accgcaacac ccaaatctac accatcaacg acaagatcct     240
cagctacacc gagagcatgg ccggcaagag ggagatggtg atcatcacct tcaagagcgg     300
cgagaccttc caggtcgagg tccccggcag ccagcacatc gacagccaga agaaggccat     360
cgagaggatg aaggacaccc tcaggatcac ctacctcacc gagaccaaga tcgacaagct     420
ctgcgtctgg aacagcaaga cccccaacag catcgccgcc atcagcatgg agaacagatc     480
ccctggttct ggtcctggtt ctcctagatc taacgccgcc tcctcgtcct cctcgcacct     540
ccagctcatc tacaacttga ccctctgcga gctgaacggc accagatctt atccttatga     600
ttatcctgat tatgctggat cttga                                           625

<210>  71
<211>  646
<212>  DNA
<213>  Artificial Sequence

<220>
<223>  an artificially synthesized DNA construct

<400>  71
tatttaactc agtattcaga aacaacaaaa gttcttctct acataaaatt ttcctatttt      60
agtgatcagt gaaggaaatc aagaaaaaaa gatggggaga atgtcaatac ccatgatggg     120
ttttgtggtg ttatgtctat gggcagtggt agcagaagga ggatccgccc cccagaccat     180
caccgagttg tgcagcgagt accgcaacac ccaaatctac accatcaacg acaagatcct     240
cagctacacc gagagcatgg ccggcaagag ggagatggtg atcatcacct tcaagagcgg     300
cgagaccttc caggtcgagg tccccggcag ccagcacatc gacagccaga agaaggccat     360
cgagaggatg aaggacaccc tcaggatcac ctacctcacc gagaccaaga tcgacaagct     420
ctgcgtctgg aacagcaaga cccccaacag catcgccgcc atcagcatgg agaacagatc     480
ccctggttct ggtcctggtt ctcctagatc taacgccgcc tcctcgtcct cctcgcacct     540
ccagctcatc tacaacttga ccctctgcga gctgaacggc accagatctt atccttatga     600
ttatcctgat tatgctggat ctgatttgtt ggttgatact atgtga                    646


<210>  72
<211>  562
<212>  DNA
<213>  Artificial Sequence

<220>
<223>  an artificially synthesized DNA construct

<400>  72
tatttaactc agtattcaga aacaacaaaa gttcttctct acataaaatt ttcctatttt      60
agtgatcagt gaaggaaatc aagaaaaaaa gatggggaga atgtcaatac ccatgatggg     120
ttttgtggtg ttatgtctat gggcagtggt agcagaagga ggatccgccc cccagaccat     180
caccgagttg tgcagcgagt accgcaacac ccaaatctac accatcaacg acaagatcct     240
cagctacacc gagagcatgg ccggcaagag ggagatggtg atcatcacct tcaagagcgg     300
cgagaccttc caggtcgagg tccccggcag ccagcacatc gacagccaga agaaggccat     360
cgagaggatg aaggacaccc tcaggatcac ctacctcacc gagaccaaga tcgacaagct     420
ctgcgtctgg aacagcaaga cccccaacag catcgccgcc atcagcatgg agaacagatc     480
ccctggttct ggtcctggtt ctcctagatc ttatccttat gattatcctg attatgctgg     540
atctgaacat gatgaattgt ga                                              562


<210>  73
<211>  733
<212>  DNA
<213>  Artificial Sequence

<220>
<223>  an artificially synthesized DNA construct

<400>  73
tatttaactc agtattcaga aacaacaaaa gttcttctct acataaaatt ttcctatttt      60
agtgatcagt gaaggaaatc aagaaaaaaa gatgttgggc aagtgcttga ccgccggctg     120
ctgctccagg ctccccttct tgtggtgcat cgtgcccttc tgcttggccg ccttggtgaa     180
cgccgcctcc tcgtcctcct cgcacctcca gctcatctac aacttgaccc tctgcgagct     240
gaacggcacc gactggttgg ccgacaagtt cgactgggcc gtggagagct tcgtgatctt     300
ccccgtgttg acccacatcg tgagctactg cgccttgacc accagccact tcttggacac     360
cgtgggcttg gtggccgtga gcaccgccgg cttctaccac ggcagatacg tgttgagcag     420
catctacgcc gtgtgcgcct tggccgcctt ggtgtgcttc gtgatcaggc tcaccaagaa     480
ctgcatgagc tggagataca gctgcaccag atacaccaac ttcttgttgg acaccaaggg     540
caggctctac agatggagga gccccgtgat catcgagaag ggcggcaaag tggaagtgga     600

gggccacttg atcgacttga agagggtggt gttggacggc agcgccgcca cccccatcac 660
caaagtgagc gccgagcagt ggggccaccc cagatcttat ccttatgatt atcctgatta 720
tgctggatct tga 733

<210> 74
<211> 730
<212> DNA
<213> Artificial Sequence

<220>
<223> an artificially synthesized DNA construct

<400> 74
tatttaactc agtattcaga aacaacaaaa gttcttctct acataaaatt ttcctatttt 60
agtgatcagt gaaggaaatc aagaaaaaaa gatgttgggc aagtgcttga ccgccggctg 120
ctgctccagg ctccccttct tgtggtgcat cgtgcccttc tgcttggccg ccttggtgaa 180
cgccgcctcc tcgtcctcct cgcacctcca gctcatctac aacttgaccc tctgcgagct 240
gaacggcacc gactggttgg ccgacaagtt cgactgggcc gtggagagct tcgtgatctt 300
ccccgtgttg acccacatcg tgagctactg cgccttgacc accagccact tcttggacac 360
cgtgggcttg gtggccgtga gcaccgccgg cttctaccac ggcagatacg tgttgagcag 420
catctacgcc gtgtgcgcct tggccgcctt ggtgtgcttc gtgatcaggc tcaccaagaa 480
ctgcatgagc tggagataca gctgcaccag atacaccaac ttcttgttgg acaccaaggg 540
caggctctac agatggagga gccccgtgat catcgagaag ggcggcaaag tggaagtgga 600
gggccacttg atcgacttga agagggtggt gttggacggc agcgccgcca cccccatcac 660
caaagtgagc gccgagcagt ggggccaccc cagatctgat tataaggatg acgatgacaa 720
gggatcttga 730

<210> 75
<211> 1372
<212> DNA
<213> Artificial Sequence

<220>
<223> an artificially synthesized DNA construct

<400> 75
tatttaactc agtattcaga aacaacaaaa gttcttctct acataaaatt ttcctatttt 60
agtgatcagt gaaggaaatc aagaaaaaaa gatgggcagc agcttggacg acttctgcca 120
cgacagcacc gccccccaga aagtgttgtt ggccttcagc atcacctaca cccccatcat 180
gatctacgcc ttgaaagtga gcaggggcag gctcttgggc ttgttgcact tgttgatctt 240
cttgaactgc gccttcacct tcggctacat gaccttcgtg cacttccaga gcaccaacaa 300
agtggccttg accatgggcg ccgtggtggc cttgttgtgg ggcgtgtaca gcgccatcga 360
gacctggaga ttcatcacca gcagatgcag gctctgcttg ttgggcagga agtacatctt 420
ggcccccgcc caccacgtgg agagcgccgc cggcttccac cccatcgccg ccagcgacaa 480
ccacgccttc gtggtgagga ggcccggcag caccaccgtg aacggcacct tggtgcccgg 540
cttgaagagc ttggtgttgg gcggcaggaa ggccgtgaag aggggcgtgg tgaacttggt 600
gaagtacgcc aagagatcca gcaagggcga ggagctgttc accggggtgg tgcccatcct 660
ggtcgagctg gacggcgacg taaacggcca caagttcagc gtgtccggcg agggcgaggg 720
cgatgccacc tacggcaagc tgaccctgaa gttcatctgc accaccggca agctgcccgt 780
gccctggccc accctcgtga ccaccttcgg ctacggcctg cagtgcttcg cccgctaccc 840
cgaccacatg aagcagcacg acttcttcaa gtccgccatg cccgaaggct acgtccagga 900
gcgcaccatc ttcttcaagg acgacggcaa ctacaagacc cgcgccgagg tgaagttcga 960
gggcgacacc ctggtgaacc gcatcgagct gaagggcatc gacttcaagg aggacggcaa 1020
catcctgggg cacaagctgg agtacaacta caacagccac aacgtctata tcatggccga 1080
caagcagaag aacggcatca aggtgaactt caagatccgc cacaacatcg aggacggcag 1140
cgtgcagctc gccgaccact accagcagaa cacccccatc ggcgacggcc ccgtgctgct 1200
gcccgacaac cactacctga gctaccagtc cgccctgagc aaagacccca acgagaagcg 1260
cgatcacatg gtcctgctgg agttcgtgac cgccgccggg atcactctcg gcatggacga 1320
gctgtacaag agatcttatc cttatgatta tcctgattat gctggatctt ga 1372

<210> 76
<211> 5720
<212> DNA
<213> Artificial Sequence

<220>
<223> an artificially synthesized DNA construct

<400> 76
aagcttgcat gcctgcaggt cgactctaga ttagcctttt caatttcaga aagaatgcta 60
acccacagat ggttagagag gcttacgcag caggtctcat caagacgatc tacccgagca 120
ataatctcca ggaaatcaaa taccttccca agaaggttaa agatgcagtc aaaagattca 180
ggactaactg catcaagaac acagagaaag atatatttct caagatcaga agtactattc 240
cagtatggac gattcaaggc ttgcttcaca aaccaaggca agtaatagag attggagtct 300
ctaaaaaggt agttcccact gaatcaaagg ccatggagtc aaagattcaa atagaggacc 360
taacagaact cgccgtaaag actggcgaac agttcataca gagtctctta cgactcaatg 420
acaagaagaa aatcttcgtc aacatggtgg agcacgacac acttgtctac tccaaaaata 480
tcaaagatac agtctcagaa gaccaaaggg caattgagac ttttcaacaa agggtaatat 540
ccggaaacct cctcggattc cattgcccag ctatctgtca ctttattgtg aagatagtgg 600
aaaaggaagg tggctcctac aaatgccatc attgcgataa aggaaaggcc atcgttgaag 660
atgcctctgc cgacagtggt cccaaagatg gacccccacc cacgaggagc atcgtggaaa 720
aagaagacgt tccaaccacg tcttcaaagc aagtggattg atgtgatatc tccactgacg 780
taagggatga cgcacaatcc cactatcctt cgcaagaccc ttcctctata taaggaagtt 840
catttcattt ggagagaaca cgcccgggta cctatttaac tcagtattca gaaacaacaa 900
aagttcttct ctacataaaa ttttcctatt ttagtgatca gtgaaggaaa tcaagaaaaa 960
aagatgttgg gcaagtgctt gaccgccggc tgctgctcca ggctcccctt cttgtggtgc 1020
atcgtgccct tctgcttggc cgccttggtg aacgccgcct cctcgtcctc ctcgcacctc 1080
cagctcatct acaacttgac cctctgcgag ctgaacggca ccgactggtt ggccgacaag 1140
ttcgactggg ccgtggagag cttcgtgatc ttccccgtgt tgacccacat cgtgagctac 1200
tgcgccttga ccaccagcca cttcttggac accgtgggct tggtggccgt gagcaccgcc 1260
ggcttctacc acggcagata cgtgttgagc agcatctacg ccgtgtgcgc cttggccgcc 1320
ttggtgtgct tcgtgatcag gctcaccaag aactgcatga gctggagata cagctgcacc 1380
agatacacca acttcttgtt ggacaccaag ggcaggctct acagatggag gagccccgtg 1440
atcatcgaga agggcggcaa agtggaagtg gagggccact tgatcgactt gaagagggtg 1500
gtgttggacg gcagcgccgc cacccccatc accaaagtga gcgccgagca gtggggccac 1560
cccagatctt atccttatga ttatcctgat tatgctggat cttgatcttg aaatcaagct 1620
agcttatcga taccgtcgac ctcgaggggg ggcccggtac ccgggagctc aatatgaaga 1680
tgaagatgaa atatttggtg tgtcaaataa aaagctagct tgtgtgctta agtttgtgtt 1740
tttttcttgg cttgttgtgt tatgaatttg tggctttttc taatattaaa tgaatgtaag 1800
atctcattat aatgaataaa caaatgtttc tataatccat tgtgaatgtt ttgttggatc 1860
tcttcgcata taactactgt atgtgctatg gtatggacta tggaatatga ttaaagataa 1920
gactagatgg gctcatagag taaaacgagg cgagggacct ataaacctcc cttcatcatg 1980
ctatttcatg atctatttta taaaataaag atgtagaaaa aagtaagcgt aataaccgca 2040
aaacaaatga tttaaaacat ggcacataat gaggagatta agttcggttt acgtttattt 2100
tagtactaat tgtaacgtga gactacgtat cgggaatcgc ctaattaaag cattaatgcg 2160
aacctgatta gattcaccga ccctcctatc gtgtcgacct ttctgtttct tagaattttt 2220
tggtagtcta tgtactaata atgtcagctt cgtatttatt tcataagcaa tttgcatttg 2280
caatttgttt tttactttta tttttattgt attgtggaat gtggactcgt accaacatga 2340
agttatatac caccaaaaaa attacagtta gtcaaaagat tcacgagtga gagctactta 2400
tgattgtctt ttacgtatat gtctaattgt ctatttgctc aataatcttt gtactttctt 2460
ttgtcgttga taaaatcaca aagttccaaa agtaatcgaa tgatttgctt ttaagaaaag 2520
aagagctcaa taattcaaca tatatctgta cactagatta gccttttcaa tttcagaaag 2580
aatgctaacc cacagatggt tagagaggct tacgcagcag gtctcatcaa gacgatctac 2640
ccgagcaata atctccagga aatcaaatac cttcccaaga aggttaaaga tgcagtcaaa 2700
agattcagga ctaactgcat caagaacaca gagaaagata tatttctcaa gatcagaagt 2760
actattccag tatggacgat tcaaggcttg cttcacaaac caaggcaagt aatagagatt 2820
ggagtctcta aaaaggtagt tcccactgaa tcaaaggcca tggagtcaaa gattcaaata 2880
gaggacctaa cagaactcgc cgtaaagact ggcgaacagt tcatacagag tctcttacga 2940
ctcaatgaca agaagaaaat cttcgtcaac atggtggagc acgacacact tgtctactcc 3000
aaaaatatca aagatacagt ctcagaagac caaagggcaa ttgagacttt tcaacaaagg 3060
gtaatatccg gaaacctcct cggattccat tgcccagcta tctgtcactt tattgtgaag 3120
atagtggaaa aggaaggtgg ctcctacaaa tgccatcatt gcgataaagg aaaggccatc 3180
gttgaagatg cctctgccga cagtggtccc aaagatggac ccccacccac gaggagcatc 3240

gtggaaaaag aagacgttcc aaccacgtct tcaaagcaag tggattgatg tgatatctcc 3300
actgacgtaa gggatgacgc acaatcccac tatccttcgc aagacccttc ctctatataa 3360
ggaagttcat ttcatttgga gagaacacgc ccgggtacct atttaactca gtattcagaa 3420
acaacaaaag ttcttctcta cataaaattt tcctatttta gtgatcagtg aaggaaatca 3480
agaaaaaaag atgggcagca gcttggacga cttctgccac gacagcaccg ccccccagaa 3540
agtgttgttg gccttcagca tcacctacac ccccatcatg atctacgcct tgaaagtgag 3600
caggggcagg ctcttgggct tgttgcactt gttgatcttc ttgaactgcg ccttcacctt 3660
cggctacatg accttcgtgc acttccagag caccaacaaa gtggccttga ccatgggcgc 3720
cgtggtggcc ttgttgtggg gcgtgtacag cgccatcgag acctggagat tcatcaccag 3780
cagatgcagg ctctgcttgt tgggcaggaa gtacatcttg gcccccgccc accacgtgga 3840
gagcgccgcc ggcttccacc ccatcgccgc cagcgacaac cacgccttcg tggtgaggag 3900
gcccggcagc accaccgtga acggcacctt ggtgcccggc ttgaagagct tggtgttggg 3960
cggcaggaag gccgtgaaga ggggcgtggt gaacttggtg aagtacgcca agagatccag 4020
caagggcgag gagctgttca ccggggtggt gcccatcctg gtcgagctgg acggcgacgt 4080
aaacggccac aagttcagcg tgtccggcga gggcgagggc gatgccacct acggcaagct 4140
gaccctgaag ttcatctgca ccaccggcaa gctgcccgtg ccctggccca ccctcgtgac 4200
caccttcggc tacggcctgc agtgcttcgc ccgctacccc gaccacatga agcagcacga 4260
cttcttcaag tccgccatgc ccgaaggcta cgtccaggag cgcaccatct tcttcaagga 4320
cgacggcaac tacaagaccc gcgccgaggt gaagttcgag ggcgacaccc tggtgaaccg 4380
catcgagctg aagggcatcg acttcaagga ggacggcaac atcctggggc acaagctgga 4440
gtacaactac aacagccaca acgtctatat catggccgac aagcagaaga acggcatcaa 4500
ggtgaacttc aagatccgcc acaacatcga ggacggcagc gtgcagctcg ccgaccacta 4560
ccagcagaac acccccatcg gcgacggccc cgtgctgctg cccgacaacc actacctgag 4620
ctaccagtcc gccctgagca aagaccccaa cgagaagcgc gatcacatgg tcctgctgga 4680
gttcgtgacc gccgccggga tcactctcgg catggacgag ctgtacaaga gatcttatcc 4740
ttatgattat cctgattatg ctggatcttg atcttgaaat caagctagct tatcgatacc 4800
gtcgacctcg aggggggccc cggtacccgg gagctcaata tgaagatgaa gatgaaatat 4860
ttggtgtgtc aaataaaaag ctagcttgtg tgcttaagtt tgtgtttttt tcttggcttg 4920
ttgtgttatg aatttgtggc tttttctaat attaaatgaa tgtaagatct cattataatg 4980
aataaacaaa tgtttctata atccattgtg aatgttttgt tggatctctt cgcatataac 5040
tactgtatgt gctatggtat ggactatgga atatgattaa agataagact agatgggctc 5100
atagagtaaa acgaggcgag ggacctataa acctcccttc atcatgctat ttcatgatct 5160
attttataaa ataaagatgt agaaaaaagt aagcgtaata accgcaaaac aaatgattta 5220
aaacatggca cataatgagg agattaagtt cggtttacgt ttattttagt actaattgta 5280
acgtgagact acgtatcggg aatcgcctaa ttaaagcatt aatgcgaacc tgattagatt 5340
caccgaccct cctatcgtgt cgacctttct gtttcttaga attttttggt agtctatgta 5400
ctaataatgt cagcttcgta tttatttcat aagcaatttg catttgcaat ttgtttttta 5460
cttttatttt tattgtattg tggaatgtgg actcgtacca acatgaagtt atataccacc 5520
aaaaaaatta cagttagtca aaagattcac gagtgagagc tacttatgat tgtcttttac 5580
gtatatgtct aattgtctat ttgctcaata atctttgtac tttcttttgt cgttgataaa 5640
atcacaaagt tccaaaagta atcgaatgat ttgcttttaa gaaaagaaga gctcaataat 5700
tcaacatata tctgtacact 5720

<210> 77
<211> 5717
<212> DNA
<213> Artificial Sequence

<220>
<223> an artificially synthesized DNA construct

<400> 77
   aagcttgcat gcctgcaggt cgactctaga ttagcctttt caatttcaga aagaatgcta 60
   acccacagat ggttagagag gcttacgcag caggtctcat caagacgatc tacccgagca 120
   ataatctcca ggaaatcaaa taccttccca agaaggttaa agatgcagtc aaaagattca 180
   ggactaactg catcaagaac acagagaaag atatatttct caagatcaga agtactattc 240
   cagtatggac gattcaaggc ttgcttcaca aaccaaggca agtaatagag attggagtct 300
   ctaaaaaggt agttcccact gaatcaaagg ccatggagtc aaagattcaa atagaggacc 360
   taacagaact cgccgtaaag actggcgaac agttcataca gagtctctta cgactcaatg 420
   acaagaagaa aatcttcgtc aacatggtgg agcacgacac acttgtctac tccaaaaata 480
   tcaaagatac agtctcagaa gaccaaaggg caattgagac ttttcaacaa agggtaatat 540
   ccggaaacct cctcggattc cattgcccag ctatctgtca ctttattgtg aagatagtgg 600
   aaaaggaagg tggctcctac aaatgccatc attgcgataa aggaaaggcc atcgttgaag 660
   atgcctctgc cgacagtggt cccaaagatg gacccccacc cacgaggagc atcgtggaaa 720
   aagaagacgt tccaaccacg tcttcaaagc aagtggattg atgtgatatc tccactgacg 780
   taagggatga cgcacaatcc cactatcctt cgcaagaccc ttcctctata taaggaagtt 840
   catttcattt ggagagaaca cgcccgggta cctatttaac tcagtattca gaaacaacaa 900
   aagttcttct ctacataaaa ttttcctatt ttagtgatca gtgaaggaaa tcaagaaaaa 960
   aagatgttgg gcaagtgctt gaccgccggc tgctgctcca ggctcccctt cttgtggtgc 1020
   atcgtgccct tctgcttggc cgccttggtg aacgccgcct cctcgtcctc ctcgcacctc 1080
   cagctcatct acaacttgac cctctgcgag ctgaacggca ccgactggtt ggccgacaag 1140
   ttcgactggg ccgtggagag cttcgtgatc ttccccgtgt tgacccacat cgtgagctac 1200
   tgcgccttga ccaccagcca cttcttggac accgtgggct tggtggccgt gagcaccgcc 1260
   ggcttctacc acggcagata cgtgttgagc agcatctacg ccgtgtgcgc cttggccgcc 1320
   ttggtgtgct tcgtgatcag gctcaccaag aactgcatga gctggagata cagctgcacc 1380
   agatacacca acttcttgtt ggacaccaag ggcaggctct acagatggag gagccccgtg 1440
   atcatcgaga agggcggcaa agtggaagtg gagggccact tgatcgactt gaagagggtg 1500
   gtgttggacg gcagcgccgc cacccccatc accaaagtga gcgccgagca gtggggccac 1560
   cccagatctg attataagga tgacgatgac aaggatcttg atcttgaaat tcaagctagc 1620
   ttatcgatac cgtcgacctc gagggggggc ccggtacccg ggagctcaat atgaagatga 1680
   agatgaaata tttggtgtgt caaataaaaa gctagcttgt gtgcttaagt ttgtgttttt 1740
   ttcttggctt gttgtgttat gaatttgtgg ctttttctaa tattaaatga atgtaagatc 1800
   tcattataat gaataaacaa atgtttctat aatccattgt gaatgttttg ttggatctct 1860
   tcgcatataa ctactgtatg tgctatggta tggactatgg aatatgatta aagataagac 1920
   tagatgggct catagagtaa aacgaggcga gggacctata aacctccctt catcatgcta 1980
   tttcatgatc tattttataa aataaagatg tagaaaaaag taagcgtaat aaccgcaaaa 2040
   caaatgattt aaaacatggc acataatgag gagattaagt tcggtttacg tttattttag 2100
   tactaattgt aacgtgagac tacgtatcgg gaatcgccta attaaagcat taatgcgaac 2160
   ctgattagat tcaccgaccc tcctatcgtg tcgacctttc tgtttcttag aattttttgg 2220
   tagtctatgt actaataatg tcagcttcgt atttatttca taagcaattt gcatttgcaa 2280
   tttgtttttt acttttattt ttattgtatt gtggaatgtg gactcgtacc aacatgaagt 2340
   tatataccac caaaaaaatt acagttagtc aaaagattca cgagtgagag ctacttatga 2400
   ttgtctttta cgtatatgtc taattgtcta tttgctcaat aatctttgta ctttcttttg 2460
   tcgttgataa aatcacaaag ttccaaaagt aatcgaatga tttgctttta agaaaagaag 2520
   agctcaataa ttcaacatat atctgtacac tagattagcc ttttcaattt cagaaagaat 2580
   gctaacccac agatggttag agaggcttac gcagcaggtc tcatcaagac gatctacccg 2640
   agcaataatc tccaggaaat caaatacctt cccaagaagg ttaaagatgc agtcaaaaga 2700
   ttcaggacta actgcatcaa gaacacagag aaagatatat ttctcaagat cagaagtact 2760
   attccagtat ggacgattca aggcttgctt cacaaaccaa ggcaagtaat agagattgga 2820
   gtctctaaaa aggtagttcc cactgaatca aaggccatgg agtcaaagat tcaaatagag 2880
   gacctaacag aactcgccgt aaagactggc gaacagttca tacagagtct cttacgactc 2940
   aatgacaaga agaaaatctt cgtcaacatg gtggagcacg acacacttgt ctactccaaa 3000
   aatatcaaag atacagtctc agaagaccaa agggcaattg agacttttca acaaagggta 3060
   atatccggaa acctcctcgg attccattgc ccagctatct gtcactttat tgtgaagata 3120
   gtggaaaagg aaggtggctc ctacaaatgc catcattgcg ataaaggaaa ggccatcgtt 3180
   gaagatgcct ctgccgacag tggtcccaaa gatggacccc cacccacgag gagcatcgtg 3240
   gaaaaagaag acgttccaac cacgtcttca aagcaagtgg attgatgtga tatctccact 3300
   gacgtaaggg atgacgcaca atcccactat ccttcgcaag acccttcctc tatataagga 3360
   agttcatttc atttggagag aacacgcccg ggtacctatt taactcagta ttcagaaaca 3420
   acaaaagttc ttctctacat aaaattttcc tattttagtg atcagtgaag gaaatcaaga 3480
   aaaaaagatg ggcagcagct tggacgactt ctgccacgac agcaccgccc cccagaaagt 3540
   gttgttggcc ttcagcatca cctacacccc catcatgatc tacgccttga aagtgagcag 3600
   gggcaggctc ttgggcttgt tgcacttgtt gatcttcttg aactgcgcct tcaccttcgg 3660
   ctacatgacc ttcgtgcact tccagagcac caacaaagtg gccttgacca tgggcgccgt 3720
   ggtggccttg ttgtggggcg tgtacagcgc catcgagacc tggagattca tcaccagcag 3780
   atgcaggctc tgcttgttgg gcaggaagta catcttggcc cccgcccacc acgtggagag 3840
   cgccgccggc ttccacccca tcgccgccag cgacaaccac gccttcgtgg tgaggaggcc 3900
   cggcagcacc accgtgaacg gcaccttggt gcccggcttg aagagcttgg tgttgggcgg 3960
   caggaaggcc gtgaagaggg gcgtggtgaa cttggtgaag tacgccaaga gatccagcaa 4020
   gggcgaggag ctgttcaccg gggtggtgcc catcctggtc gagctggacg gcgacgtaaa 4080
   cggccacaag ttcagcgtgt ccggcgaggg cgagggcgat gccacctacg gcaagctgac 4140
   cctgaagttc atctgcacca ccggcaagct gcccgtgccc tggcccaccc tcgtgaccac 4200
   cttcggctac ggcctgcagt gcttcgcccg ctaccccgac cacatgaagc agcacgactt 4260
   cttcaagtcc gccatgcccg aaggctacgt ccaggagcgc accatcttct tcaaggacga 4320
   cggcaactac aagacccgcg ccgaggtgaa gttcgagggc gacaccctgg tgaaccgcat 4380
   cgagctgaag ggcatcgact tcaaggagga cggcaacatc ctggggcaca agctggagta 4440
   caactacaac agccacaacg tctatatcat ggccgacaag cagaagaacg gcatcaaggt 4500
   gaacttcaag atccgccaca acatcgagga cggcagcgtg cagctcgccg accactacca 4560
   gcagaacacc cccatcggcg acggccccgt gctgctgccc gacaaccact acctgagcta 4620
   ccagtccgcc ctgagcaaag accccaacga gaagcgcgat cacatggtcc tgctggagtt 4680
   cgtgaccgcc gccgggatca ctctcggcat ggacgagctg tacaagagat cttatcctta 4740
   tgattatcct gattatgctg gatcttgatc ttgaaatcaa gctagcttat cgataccgtc 4800
   gacctcgagg gggggcccgg tacccgggag ctcaatatga agatgaagat gaaatatttg 4860
   gtgtgtcaaa taaaaagcta gcttgtgtgc ttaagtttgt gtttttttct tggcttgttg 4920
   tgttatgaat ttgtggcttt ttctaatatt aaatgaatgt aagatctcat tataatgaat 4980
   aaacaaatgt ttctataatc cattgtgaat gttttgttgg atctcttcgc atataactac 5040
   tgtatgtgct atggtatgga ctatggaata tgattaaaga taagactaga tgggctcata 5100
   gagtaaaacg aggcgaggga cctataaacc tcccttcatc atgctatttc atgatctatt 5160
   ttataaaata aagatgtaga aaaaagtaag cgtaataacc gcaaaacaaa tgatttaaaa 5220
   catggcacat aatgaggaga ttaagttcgg tttacgttta ttttagtact aattgtaacg 5280
   tgagactacg tatcgggaat cgcctaatta aagcattaat gcgaacctga ttagattcac 5340
   cgaccctcct atcgtgtcga cctttctgtt tcttagaatt ttttggtagt ctatgtacta 5400
   ataatgtcag cttcgtattt atttcataag caatttgcat ttgcaatttg ttttttactt 5460
   ttatttttat tgtattgtgg aatgtggact cgtaccaaca tgaagttata taccaccaaa 5520
   aaaattacag ttagtcaaaa gattcacgag tgagagctac ttatgattgt cttttacgta 5580
   tatgtctaat tgtctatttg ctcaataatc tttgtacttt cttttgtcgt tgataaaatc 5640
   acaaagttcc aaaagtaatc gaatgatttg cttttaagaa aagaagagct caataattca 5700
   acatatatct gtacact 5717

<210> 78
<211> 5729
<212> DNA
<213> Artificial Sequence

<220>
<223> an artificially synthesized DNA construct

<400> 78
aagcttgcat gcctgcaggt cgactctaga ttagcctttt caatttcaga aagaatgcta 60
acccacagat ggttagagag gcttacgcag caggtctcat caagacgatc tacccgagca 120
ataatctcca ggaaatcaaa taccttccca agaaggttaa agatgcagtc aaaagattca 180
ggactaactg catcaagaac acagagaaag atatatttct caagatcaga agtactattc 240
cagtatggac gattcaaggc ttgcttcaca aaccaaggca agtaatagag attggagtct 300
ctaaaaaggt agttcccact gaatcaaagg ccatggagtc aaagattcaa atagaggacc 360
taacagaact cgccgtaaag actggcgaac agttcataca gagtctctta cgactcaatg 420
acaagaagaa aatcttcgtc aacatggtgg agcacgacac acttgtctac tccaaaaata 480
tcaaagatac agtctcagaa gaccaaaggg caattgagac ttttcaacaa agggtaatat 540
ccggaaacct cctcggattc cattgcccag ctatctgtca ctttattgtg aagatagtgg 600
aaaaggaagg tggctcctac aaatgccatc attgcgataa aggaaaggcc atcgttgaag 660
atgcctctgc cgacagtggt cccaaagatg gacccccacc cacgaggagc atcgtggaaa 720
aagaagacgt tccaaccacg tcttcaaagc aagtggattg atgtgatatc tccactgacg 780
taagggatga cgcacaatcc cactatcctt cgcaagaccc ttcctctata taaggaagtt 840
catttcattt ggagagaaca cgcccgggta cctatttaac tcagtattca gaaacaacaa 900
aagttcttct ctacataaaa ttttcctatt ttagtgatca gtgaaggaaa tcaagaaaaa 960
aagatgttgg gcaagtgctt gaccgccggc tgctgctcca ggctcccctt cttgtggtgc 1020
atcgtgccct tctgcttggc cgccttggtg aacgccgcct cctcgtcctc ctcgcacctc 1080
cagctcatct acaacttgac cctctgcgag ctgaacggca ccgactggtt ggccgacaag 1140
ttcgactggg ccgtggagag cttcgtgatc ttccccgtgt tgacccacat cgtgagctac 1200
tgcgccttga ccaccagcca cttcttggac accgtgggct tggtggccgt gagcaccgcc 1260
ggcttctacc acggcagata cgtgttgagc agcatctacg ccgtgtgcgc cttggccgcc 1320
ttggtgtgct tcgtgatcag gctcaccaag aactgcatga gctggagata cagctgcacc 1380
agatacacca acttcttgtt ggacaccaag ggcaggctct acagatggag gagccccgtg 1440
atcatcgaga agggcggcaa agtggaagtg gagggccact tgatcgactt gaagagggtg 1500
gtgttggacg gcagcgccgc cacccccatc accaaagtga gcgccgagca gtggggccac 1560
cccagatctg attataagga tgacgatgac aaggatcttg atcttgaaa tcaagctagc 1620
ttatcgatac cgtcgacctc gagggggggc ccggtacccg ggagctcaat atgaagatga 1680
agatgaaata tttggtgtgt caaataaaaa gctagcttgt gtgcttaagt ttgtgttttt 1740

ttcttggctt gttgtgttat gaatttgtgg ctttttctaa tattaaatga atgtaagatc 1800
tcattataat gaataaacaa atgtttctat aatccattgt gaatgttttg ttggatctct 1860
tcgcatataa ctactgtatg tgctatggta tggactatgg aatatgatta aagataagac 1920
tagatgggct catagagtaa aacgaggcga gggacctata aacctccctt catcatgcta 1980
tttcatgatc tattttataa aataaagatg tagaaaaaag taagcgtaat aaccgcaaaa 2040
caaatgattt aaaacatggc acataatgag gagattaagt tcggtttacg tttattttag 2100
tactaattgt aacgtgagac tacgtatcgg gaatcgccta attaaagcat taatgcgaac 2160
ctgattagat tcaccgaccc tcctatcgtg tcgacctttc tgtttcttag aattttttgg 2220
tagtctatgt actaataatg tcagcttcgt atttatttca taagcaattt gcatttgcaa 2280
tttgtttttt acttttattt ttattgtatt gtggaatgtg gactcgtacc aacatgaagt 2340
tatataccac caaaaaaatt acagttagtc aaaagattca cgagtgagag ctacttatga 2400
ttgtctttta cgtatatgtc taattgtcta tttgctcaat aatctttgta ctttcttttg 2460
tcgttgataa aatcacaaag ttccaaaagt aatcgaatga tttgctttta agaaaagaag 2520
agctcaataa ttcaacatat atctgtacac tagattagcc ttttcaattt cagaaagaat 2580
gctaacccac agatggttag agaggcttac gcagcaggtc tcatcaagac gatctacccg 2640
agcaataatc tccaggaaat caaatacctt cccaagaagg ttaaagatgc agtcaaaaga 2700
ttcaggacta actgcatcaa gaacacagag aaagatatat ttctcaagat cagaagtact 2760
attccagtat ggacgattca aggcttgctt cacaaaccaa ggcaagtaat agagattgga 2820
gtctctaaaa aggtagttcc cactgaatca aaggccatgg agtcaaagat tcaaatagag 2880
gacctaacag aactcgccgt aaagactggc gaacagttca tacagagtct cttacgactc 2940
aatgacaaga agaaaatctt cgtcaacatg gtggagcacg acacacttgt ctactccaaa 3000
aatatcaaag atacagtctc agaagaccaa agggcaattg agacttttca acaaagggta 3060
atatccggaa acctcctcgg attccattgc ccagctatct gtcactttat tgtgaagata 3120
gtggaaaagg aaggtggctc ctacaaatgc catcattgcg ataaaggaaa ggccatcgtt 3180
gaagatgcct ctgccgacag tggtcccaaa gatggacccc cacccacgag gagcatcgtg 3240
gaaaaagaag acgttccaac cacgtcttca aagcaagtgg attgatgtga tatctccact 3300
gacgtaaggg atgacgcaca atcccactat ccttcgcaag acccttcctc tatataagga 3360
agttcatttc atttggagag aacacgcccg ggtacctatt taactcagta ttcagaaaca 3420
acaaaagttc ttctctacat aaaattttcc tattttagtg atcagtgaag gaaatcaaga 3480
aaaaaagatg gccgccagct tgttgttctt gttggtgggc ttcgagtgct tcttggtgag 3540
ccaggccttc gcctgcaagc cctgcttcag cagcagcttg agcgacatca agaccaacac 3600
caccgccgcc gccagcttcg ccgtgttgca ggacatcagc tgcttgaggc acggcgacag 3660
cagcccccag accatcagga agagcaggca gtgcaggacc gccatcggca cccccgtgta 3720
catcaccatc accgccaacg tgaccgacga gaactacttg cacagcagcg acttgttgat 3780
gttgagcagc tgcttgttct acgccagcga gatgagcgag aagggcttca aagtggtgtt 3840
cggcaacgtg agcggcatcg tggccgtgtg cgtgaacttc accagctacg tgcagcacgt 3900
gagggagttc acccagagga gcttggtggt ggaccacgtg aggctcttgc acttcatgac 3960
ccccgagacc atgagatggg ccaccgtgtt ggcctgcttg ttcgccatct tgttggccat 4020
cagatctagc aagggcgagg agctgttcac cggggtggtg cccatcctgg tcgagctgga 4080
cggcgacgta aacggccaca agttcagcgt gtccggcgag ggcgagggcg atgccaccta 4140
cggcaagctg accctgaagt tcatctgcac caccggcaag ctgcccgtgc cctggcccac 4200
cctcgtgacc accttcggct acggcctgca gtgcttcgcc cgctaccccg accacatgaa 4260
gcagcacgac ttcttcaagt ccgccatgcc cgaaggctac gtccaggagc gcaccatctt 4320
cttcaaggac gacggcaact acaagacccg cgccgaggtg aagttcgagg gcgacaccct 4380
ggtgaaccgc atcgagctga agggcatcga cttcaaggag gacggcaaca tcctggggca 4440
caagctggag tacaactaca acagccacaa cgtctatatc atggccgaca agcagaagaa 4500
cggcatcaag gtgaacttca agatccgcca caacatcgag gacggcagcg tgcagctcgc 4560
cgaccactac cagcagaaca cccccatcgg cgacggcccc gtgctgctgc ccgacaacca 4620
ctacctgagc taccagtccg ccctgagcaa agaccccaac gagaagcgcg atcacatggt 4680
cctgctggag ttcgtgaccg ccgccgggat cactctcggc atggacgagc tgtacaagag 4740
atcttatcct tatgattatc ctgattatgc tggatcttga tcttgaaatc aagctagctt 4800
atcgataccg tcgacctcga gggggggccc ggtacccggg agctcaatat gaagatgaag 4860
atgaaatatt tggtgtgtca aataaaaagc tagcttgtgt gcttaagttt gtgttttttt 4920
cttggcttgt tgtgttatga atttgtggct ttttctaata ttaaatgaat gtaagatctc 4980
attataatga ataaacaaat gtttctataa tccattgtga atgttttgtt ggatctcttc 5040
gcatataact actgtatgtg ctatggtatg gactatggaa tatgattaaa gataagacta 5100
gatgggctca tagagtaaaa cgaggcgagg gacctataaa cctcccttca tcatgctatt 5160
tcatgatcta ttttataaaa taaagatgta gaaaaaagta agcgtaataa ccgcaaaaca 5220
aatgatttaa aacatggcac ataatgagga gattaagttc ggtttacgtt tattttagta 5280
ctaattgtaa cgtgagacta cgtatcggga atcgcctaat taaagcatta atgcgaacct 5340
gattagattc accgaccctc ctatcgtgtc gacctttctg tttcttagaa ttttttggta 5400
gtctatgtac taataatgtc agcttcgtat ttatttcata agcaatttgc atttgcaatt 5460
tgttttttac ttttattttt attgtattgt ggaatgtgga ctcgtaccaa catgaagtta 5520

tataccacca aaaaaattac agttagtcaa aagattcacg agtgagagct acttatgatt 5580
gtcttttacg tatatgtcta attgtctatt tgctcaataa tctttgtact ttcttttgtc 5640
gttgataaaa tcacaaagtt ccaaaagtaa tcgaatgatt tgcttttaag aaaagaagag 5700
ctcaataatt caacatatat ctgtacact 5729

<210> 79
<211> 884
<212> DNA
<213> Arabidopsis thaliana

<400> 79
aatatgaaga tgaagatgaa atatttggtg tgtcaaataa aaagctagct tgtgtgctta 60
agtttgtgtt tttttcttgg cttgttgtgt tatgaatttg tggctttttc taatattaaa 120
tgaatgtaag atctcattat aatgaataaa caaatgtttc tataatccat tgtgaatgtt 180
ttgttggatc tcttcgcata taactactgt atgtgctatg gtatggacta tggaatatga 240
ttaaagataa gactagatgg gctcatagag taaaacgagg cgagggacct ataaacctcc 300
cttcatcatg ctatttcatg atctatttta taaaataaag atgtagaaaa aagtaagcgt 360
aataaccgca aaacaaatga tttaaaacat ggcacataat gaggagatta agttcggttt 420
acgtttattt tagtactaat tgtaacgtga gactacgtat cgggaatcgc ctaattaaag 480
cattaatgcg aacctgatta gattcaccga ccctcctatc gtgtcgacct ttctgtttct 540
tagaattttt tggtagtcta tgtactaata atgtcagctt cgtatttatt tcataagcaa 600
tttgcatttg caatttgttt tttactttta tttttattgt attgtggaat gtggactcgt 660
accaacatga agttatatac caccaaaaaa attacagtta gtcaaaagat tcacgagtga 720
gagctactta tgattgtctt ttacgtatat gtctaattgt ctatttgctc aataatcttt 780
gtactttctt ttgtcgttga taaaatcaca aagttccaaa agtaatcgaa tgatttgctt 840
ttaagaaaag aagagctcaa taattcaaca tatatctgta cact 884

<210> 80
<211> 44
<212> DNA
<213> Artificial Sequence

<220>
<223> an artificially synthesized primer

<400> 80
ttggatccgc cccccagacc atcaccgagt tgtgcagcga gtac 44

<210> 81
<211> 39
<212> DNA
<213> Artificial Sequence

<220>
<223> an artificially synthesized primer

<400> 81
cgttgatggt gtagatttgg gtgttgcggt actcgctgc 39

<210> 82
<211> 39
<212> DNA
<213> Artificial Sequence

<220>
<223> an artificially synthesized primer

<400> 82
ccatcaacga caagatcctc agctacaccg agagcatgg 39

<210>  83
<211>  43
<212>  DNA
<213>  Artificial Sequence

<220>
<223>  an artificially synthesized primer

<400>  83
cttgaaggtg atgatcacca tctccctctt gccggccatg ctc                43


<210>  84
<211>  39
<212>  DNA
<213>  Artificial Sequence

<220>
<223>  an artificially synthesized primer

<400>  84
atcaccttca agagcggcga gaccttccag gtcgaggtc                39


<210>  85
<211>  45
<212>  DNA
<213>  Artificial Sequence

<220>
<223>  an artificially synthesized primer

<400>  85
cttctggctg tcgatgtgct ggctgccggg gacctcgacc tggaa                45


<210>  86
<211>  48
<212>  DNA
<213>  Artificial Sequence

<220>
<223>  an artificially synthesized primer

<400>  86
gacagccaga agaaggccat cgagaggatg aaggacaccc tcaggatc                48


<210>  87
<211>  44
<212>  DNA
<213>  Artificial Sequence

<220>
<223>  an artificially synthesized primer

<400>  87
gcagagcttg tcgatcttgg tctcggtgag gtaggtgatc ctga                44


<210>  88

<211>  30
<212>  DNA
<213>  Artificial Sequence

<220>
<223>  an artificially synthesized primer

<400>  88
aagctctgcg tctggaacag caagaccccc                                        30


<210>  89
<211>  68
<212>  DNA
<213>  Artificial Sequence

<220>
<223>  an artificially synthesized primer

<400>  89
aaagatctgt tctccatgct gatggcggcg atgctgttgg gggtcttgtt gttccagacg      60

cagagctt                                                                68


<210>  90
<211>  30
<212>  DNA
<213>  Artificial Sequence

<220>
<223>  an artificially synthesized primer

<400>  90
gatcccctgg ttctggtcct ggttctccta                                        30


<210>  91
<211>  30
<212>  DNA
<213>  Artificial Sequence

<220>
<223>  an artificially synthesized primer

<400>  91
gatctaggag aaccaggacc agaaccaggg                                        30


<210>  92
<211>  33
<212>  DNA
<213>  Artificial Sequence

<220>
<223>  an artificially synthesized primer

<400>  92
gatcttatcc ttatgattat cctgattatg ctg                                    33


<210>  93
<211>  33

<212>  DNA
<213>  Artificial Sequence

<220>
<223>  an artificially synthesized primer

<400>  93
gatccagcat aatcaggata atcataagga taa                                    33


<210>  94
<211>  41
<212>  DNA
<213>  Artificial Sequence

<220>
<223>  an artificially synthesized primer

<400>  94
aagcatgcgg atccaacgcc gcctcctcgt cctcctcgca c                           41


<210>  95
<211>  42
<212>  DNA
<213>  Artificial Sequence

<220>
<223>  an artificially synthesized primer

<400>  95
tcgtcctcct cgcacctcca gctcatctac aacttgaccc tc                          42


<210>  96
<211>  41
<212>  DNA
<213>  Artificial Sequence

<220>
<223>  an artificially synthesized primer

<400>  96
ttagatctgg tgccgttcag ctcgcagagg gtcaagttgt a                           41


<210>  97
<211>  35
<212>  DNA
<213>  Artificial Sequence

<220>
<223>  an artificially synthesized primer

<400>  97
aatgcatgtt gggcaagtgc ttgaccgccg gctgc                                  35


<210>  98
<211>  35
<212>  DNA
<213>  Artificial Sequence

<220>
<223>  an artificially synthesized primer

<400>  98
gcaccacaag aaggggagcc tggagcagca gccgg                                35


<210>  99
<211>  35
<212>  DNA
<213>  Artificial Sequence

<220>
<223>  an artificially synthesized primer

<400>  99
tcttgtggtg catcgtgccc ttctgcttgg ccgcc                                35


<210>  100
<211>  35
<212>  DNA
<213>  Artificial Sequence

<220>
<223>  an artificially synthesized primer

<400>  100
ggaggacgag gaggcggcgt tcaccaaggc ggcca                                35


<210>  101
<211>  42
<212>  DNA
<213>  Artificial Sequence

<220>
<223>  an artificially synthesized primer

<400>  101
tcgtcctcct cgcacctcca gctcatctac aacttgaccc tc                        42


<210>  102
<211>  35
<212>  DNA
<213>  Artificial Sequence

<220>
<223>  an artificially synthesized primer

<400>  102
accagtcggt gccgttcagc tcgcagaggg tcaag                                35


<210>  103
<211>  35
<212>  DNA
<213>  Artificial Sequence

<220>
<223>  an artificially synthesized primer

<400>  103
ccgactggtt ggccgacaag ttcgactggg ccgtg                                35


<210>  104
<211>  35
<212>  DNA
<213>  Artificial Sequence

<220>
<223>  an artificially synthesized primer

<400>  104
tcaacacggg gaagatcacg aagctctcca cggcc                                35


<210>  105
<211>  35
<212>  DNA
<213>  Artificial Sequence

<220>
<223>  an artificially synthesized primer

<400>  105
ccgtgttgac ccacatcgtg agctactgcg ccttg                                35


<210>  106
<211>  35
<212>  DNA
<213>  Artificial Sequence

<220>
<223>  an artificially synthesized primer

<400>  106
cacggtgtcc aagaagtggc tggtggtcaa ggcgc                                35


<210>  107
<211>  35
<212>  DNA
<213>  Artificial Sequence

<220>
<223>  an artificially synthesized primer

<400>  107
gacaccgtgg gcttggtggc cgtgagcacc gccgg                                35


<210>  108
<211>  35
<212>  DNA
<213>  Artificial Sequence

<220>
<223>  an artificially synthesized primer

<400>  108
gctcaacacg tatctgccgt ggtagaagcc ggcgg                                35

<210>  109
<211>  35
<212>  DNA
<213>  Artificial Sequence

<220>
<223>  an artificially synthesized primer

<400>  109
cgtgttgagc agcatctacg ccgtgtgcgc cttgg                                35


<210>  110
<211>  35
<212>  DNA
<213>  Artificial Sequence

<220>
<223>  an artificially synthesized primer

<400>  110
gcctgatcac gaagcacacc aaggcggcca aggcg                                35


<210>  111
<211>  35
<212>  DNA
<213>  Artificial Sequence

<220>
<223>  an artificially synthesized primer

<400>  111
tgatcaggct caccaagaac tgcatgagct ggaga                                35


<210>  112
<211>  35
<212>  DNA
<213>  Artificial Sequence

<220>
<223>  an artificially synthesized primer

<400>  112
agttggtgta tctggtgcag ctgtatctcc agctc                                35


<210>  113
<211>  35
<212>  DNA
<213>  Artificial Sequence

<220>
<223>  an artificially synthesized primer

<400>  113
gatacaccaa cttcttgttg gacaccaagg gcagg                                35


<210>  114
<211>  35

<212>  DNA
<213>  Artificial Sequence

<220>
<223>  an artificially synthesized primer

<400>  114
tgatcacggg gctcctccat ctgtagagcc tgccc                                35


<210>  115
<211>  35
<212>  DNA
<213>  Artificial Sequence

<220>
<223>  an artificially synthesized primer

<400>  115
cccgtgatca tcgagaaggg cggcaaagtg gaagt                                35


<210>  116
<211>  35
<212>  DNA
<213>  Artificial Sequence

<220>
<223>  an artificially synthesized primer

<400>  116
tcaagtcgat caagtggccc tccacttcca ctttg                                35


<210>  117
<211>  35
<212>  DNA
<213>  Artificial Sequence

<220>
<223>  an artificially synthesized primer

<400>  117
tcgacttgaa gagggtggtg ttggacggca gcgcc                                35


<210>  118
<211>  22
<212>  DNA
<213>  Artificial Sequence

<220>
<223>  an artificially synthesized primer

<400>  118
ggtgatgggg gtggcggcgc tg                                              22


<210>  119
<211>  24
<212>  DNA
<213>  Artificial Sequence

<220>
<223>  an artificially synthesized primer

<400>  119
ccccatcacc aaagtgagcg ccga                                              24


<210>  120
<211>  33
<212>  DNA
<213>  Artificial Sequence

<220>
<223>  an artificially synthesized primer

<400>  120
ttagatctgg ggtggcccca ctgctcggcg ctc                                    33


<210>  121
<211>  35
<212>  DNA
<213>  Artificial Sequence

<220>
<223>  an artificially synthesized primer

<400>  121
aatgcatggg cagcagcttg gacgacttct gccac                                  35


<210>  122
<211>  35
<212>  DNA
<213>  Artificial Sequence

<220>
<223>  an artificially synthesized primer

<400>  122
acactttctg gggggcggtg ctgtcgtggc agaag                                  35


<210>  123
<211>  35
<212>  DNA
<213>  Artificial Sequence

<220>
<223>  an artificially synthesized primer

<400>  123
ccagaaagtg ttgttggcct tcagcatcac ctaca                                  35


<210>  124
<211>  35
<212>  DNA
<213>  Artificial Sequence

<220>
<223>  an artificially synthesized primer

<400>  124
caaggcgtag atcatgatgg gggtgtaggt gatgc                                35


<210>  125
<211>  35
<212>  DNA
<213>  Artificial Sequence

<220>
<223>  an artificially synthesized primer

<400>  125
ctacgccttg aaagtgagca ggggcaggct cttgg                                35


<210>  126
<211>  35
<212>  DNA
<213>  Artificial Sequence

<220>
<223>  an artificially synthesized primer

<400>  126
caagaagatc aacaagtgca acaagcccaa gagcc                                35


<210>  127
<211>  35
<212>  DNA
<213>  Artificial Sequence

<220>
<223>  an artificially synthesized primer

<400>  127
gttgatcttc ttgaactgcg ccttcacctt cggct                                35


<210>  128
<211>  35
<212>  DNA
<213>  Artificial Sequence

<220>
<223>  an artificially synthesized primer

<400>  128
gctctggaag tgcacgaagg tcatgtagcc gaagg                                35


<210>  129
<211>  35
<212>  DNA
<213>  Artificial Sequence

<220>
<223>  an artificially synthesized primer

<400>  129
cttccagagc accaacaaag tggccttgac catgg                                35

<210>  130
<211>  35
<212>  DNA
<213>  Artificial Sequence

<220>
<223>  an artificially synthesized primer

<400>  130
cccacaacaa ggccaccacg gcgcccatgg tcaag                                35


<210>  131
<211>  35
<212>  DNA
<213>  Artificial Sequence

<220>
<223>  an artificially synthesized primer

<400>  131
gttgtggggc gtgtacagcg ccatcgagac ctgga                                35


<210>  132
<211>  35
<212>  DNA
<213>  Artificial Sequence

<220>
<223>  an artificially synthesized primer

<400>  132
agcctgcatc tgctggtgat gaatctccag gtctc                                35


<210>  133
<211>  35
<212>  DNA
<213>  Artificial Sequence

<220>
<223>  an artificially synthesized primer

<400>  133
gatgcaggct ctgcttgttg ggcaggaagt acatc                                35


<210>  134
<211>  35
<212>  DNA
<213>  Artificial Sequence

<220>
<223>  an artificially synthesized primer

<400>  134
ctccacgtgg tgggcggggg ccaagatgta cttcc                                35


<210>  135
<211>  35

<212>  DNA
<213>  Artificial Sequence

<220>
<223>  an artificially synthesized primer

<400>  135
cacgtggaga gcgccgccgg cttccacccc atcgc                                35


<210>  136
<211>  35
<212>  DNA
<213>  Artificial Sequence

<220>
<223>  an artificially synthesized primer

<400>  136
accacgaagg cgtggttgtc gctggcggcg atggg                                35


<210>  137
<211>  35
<212>  DNA
<213>  Artificial Sequence

<220>
<223>  an artificially synthesized primer

<400>  137
ccttcgtggt gaggaggccc ggcagcacca ccgtg                                35


<210>  138
<211>  30
<212>  DNA
<213>  Artificial Sequence

<220>
<223>  an artificially synthesized primer

<400>  138
gccgggcacc aaggtgccgt tcacggtggt                                      30


<210>  139
<211>  23
<212>  DNA
<213>  Artificial Sequence

<220>
<223>  an artificially synthesized primer

<400>  139
tgcccggctt gaagagcttg gtg                                             23


<210>  140
<211>  35
<212>  DNA
<213>  Artificial Sequence

<220>
<223>  an artificially synthesized primer

<400>  140
tcttcacggc cttcctgccg cccaacacca agctc                                35


<210>  141
<211>  27
<212>  DNA
<213>  Artificial Sequence

<220>
<223>  an artificially synthesized primer

<400>  141
gccgtgaaga ggggcgtggt gaacttg                                         27


<210>  142
<211>  35
<212>  DNA
<213>  Artificial Sequence

<220>
<223>  an artificially synthesized primer

<400>  142
ttagatctct tggcgtactt caccaagttc accac                                35


<210>  143
<211>  35
<212>  DNA
<213>  Artificial Sequence

<220>
<223>  an artificially synthesized primer

<400>  143
aatgcatggc cgccagcttg ttgttcttgt tggtg                                35


<210>  144
<211>  35
<212>  DNA
<213>  Artificial Sequence

<220>
<223>  an artificially synthesized primer

<400>  144
ggctcaccaa gaagcactcg aagcccacca acaag                                35


<210>  145
<211>  34
<212>  DNA
<213>  Artificial Sequence

<220>
<223>  an artificially synthesized primer

<400>  145
ggtgagccag gccttcgcct gcaagccctg cttc                                34


<210>  146
<211>  35
<212>  DNA
<213>  Artificial Sequence

<220>
<223>  an artificially synthesized primer

<400>  146
gtcttgatgt cgctcaagct gctgctgaag caggg                               35


<210>  147
<211>  35
<212>  DNA
<213>  Artificial Sequence

<220>
<223>  an artificially synthesized primer

<400>  147
gacatcaaga ccaacaccac cgccgccgcc agctt                               35


<210>  148
<211>  35
<212>  DNA
<213>  Artificial Sequence

<220>
<223>  an artificially synthesized primer

<400>  148
agcagctgat gtcctgcaac acggcgaagc tggcg                               35


<210>  149
<211>  35
<212>  DNA
<213>  Artificial Sequence

<220>
<223>  an artificially synthesized primer

<400>  149
catcagctgc ttgaggcacg gcgacagcag ccccc                               35


<210>  150
<211>  35
<212>  DNA
<213>  Artificial Sequence

<220>
<223>  an artificially synthesized primer

<400>  150
ctgcactgcc tgctcttcct gatggtctgg gggct                               35

<210>  151
<211>  35
<212>  DNA
<213>  Artificial Sequence

<220>
<223>  an artificially synthesized primer

<400>  151
gcagtgcagg accgccatcg gcacccccgt gtaca                                35


<210>  152
<211>  35
<212>  DNA
<213>  Artificial Sequence

<220>
<223>  an artificially synthesized primer

<400>  152
tcggtcacgt tggcggtgat ggtgatgtac acggg                                35


<210>  153
<211>  35
<212>  DNA
<213>  Artificial Sequence

<220>
<223>  an artificially synthesized primer

<400>  153
cgtgaccgac gagaactact tgcacagcag cgact                                35


<210>  154
<211>  35
<212>  DNA
<213>  Artificial Sequence

<220>
<223>  an artificially synthesized primer

<400>  154
gaacaagcag ctgctcaaca tcaacaagtc gctgc                                35


<210>  155
<211>  35
<212>  DNA
<213>  Artificial Sequence

<220>
<223>  an artificially synthesized primer

<400>  155
gctgcttgtt ctacgccagc gagatgagcg agaag                                35


<210>  156
<211>  35

<212>  DNA
<213>  Artificial Sequence

<220>
<223>  an artificially synthesized primer

<400>  156
cgttgccgaa caccactttg aagcccttct cgctc                                35


<210>  157
<211>  35
<212>  DNA
<213>  Artificial Sequence

<220>
<223>  an artificially synthesized primer

<400>  157
cggcaacgtg agcggcatcg tggccgtgtg cgtga                                35


<210>  158
<211>  35
<212>  DNA
<213>  Artificial Sequence

<220>
<223>  an artificially synthesized primer

<400>  158
acgtgctgca cgtagctggt gaagttcacg cacac                                35


<210>  159
<211>  35
<212>  DNA
<213>  Artificial Sequence

<220>
<223>  an artificially synthesized primer

<400>  159
gcagcacgtg agggagttca cccagaggag cttgg                                35


<210>  160
<211>  35
<212>  DNA
<213>  Artificial Sequence

<220>
<223>  an artificially synthesized primer

<400>  160
gtgcaagagc ctcacgtggt ccaccaccaa gctcc                                35


<210>  161
<211>  33
<212>  DNA
<213>  Artificial Sequence

<220>
<223>  an artificially synthesized primer

<400>  161
gctcttgcac ttcatgaccc ccgagaccat gag                                    33


<210>  162
<211>  23
<212>  DNA
<213>  Artificial Sequence

<220>
<223>  an artificially synthesized primer

<400>  162
acggtggccc atctcatggt ctc                                               23


<210>  163
<211>  28
<212>  DNA
<213>  Artificial Sequence

<220>
<223>  an artificially synthesized primer

<400>  163
gccaccgtgt tggcctgctt gttcgcca                                          28


<210>  164
<211>  34
<212>  DNA
<213>  Artificial Sequence

<220>
<223>  an artificially synthesized primer

<400>  164
ttagatctga tggccaacaa gatggcgaac aagc                                   34


<210>  165
<211>  41
<212>  DNA
<213>  Artificial Sequence

<220>
<223>  an artificially synthesized primer

<400>  165
aatctagagt ctatttaact cagtattcag aaacaacaaa a                           41


<210>  166
<211>  30
<212>  DNA
<213>  Artificial Sequence

<220>
<223>  an artificially synthesized primer

<400>  166
aaatgcatct tttttcttga tttccttcac                                        30


<210>  167
<211>  38
<212>  DNA
<213>  Artificial Sequence

<220>
<223>  an artificially synthesized primer

<400>  167
aaggtaccta tttaactcag tattcagaaa caacaaaa                               38


<210>  168
<211>  20
<212>  DNA
<213>  Artificial Sequence

<220>
<223>  an artificially synthesized primer

<400>  168
tgccaaatgt ttgaacgatc                                                   20


<210>  169
<211>  31
<212>  DNA
<213>  Artificial Sequence

<220>
<223>  an artificially synthesized primer

<400>  169
tttggatcca gcaagggcga ggagctgttc a                                      31


<210>  170
<211>  33
<212>  DNA
<213>  Artificial Sequence

<220>
<223>  an artificially synthesized primer

<400>  170
tttagatctc ttgtacagct cgtccatgcc gag                                    33


<210>  171
<211>  30
<212>  DNA
<213>  Artificial Sequence

<220>
<223>  an artificially synthesized primer

<400>  171
gatctgatta taaggatgac gatgacaagg                                        30

<210>  172
<211>  30
<212>  DNA
<213>  Artificial Sequence

<220>
<223>  an artificially synthesized primer

<400>  172
gatcccttgt catcgtcatc cttataatca                                        30


<210>  173
<211>  58
<212>  DNA
<213>  Artificial Sequence

<220>
<223>  primer

<400>  173
aaagcttaag atgcttttgc aagccttcct tttcctcttg gctggtttcg ccgccaag         58


<210>  174
<211>  41
<212>  DNA
<213>  Artificial Sequence

<220>
<223>  primer

<400>  174
aaggatccgg cagaaatctt ggcggcgaaa ccagccaaga g                           41


<210>  175
<211>  20
<212>  DNA
<213>  Artificial Sequence

<220>
<223>  primer

<400>  175
taatacgact cactataggg                                                   20


<210>  176
<211>  27
<212>  DNA
<213>  Artificial Sequence

<220>
<223>  primer

<400>  176
gtaagcgtga cataactaat tacatga                                           27


<210>  177
<211>  23

<212>  PRT
<213>  Artificial Sequence

<220>
<223>  ectGP5(EU)

<400>  177
Asp Gly Ser Gly Ser Ser Ser Thr Tyr Gln Tyr Ile Tyr Asn Leu Thr
1               5                   10                  15
Ile Cys Glu Leu Asn Gly Thr
            20


<210>  178
<211>  62
<212>  DNA
<213>  Artificial Sequence

<220>
<223>  primer

<400>  178
aaggatccga cggctccggg tcctcctcga cctaccagta catctacaac ttgaccatct      60
gc                                                                      62


<210>  179
<211>  27
<212>  DNA
<213>  Artificial Sequence

<220>
<223>  primer

<400>  179
aagagctcac aattcatcat gttcaga                                           27


<210>  180
<211>  44
<212>  DNA
<213>  Artificial Sequence

<220>
<223>  primer

<400>  180
ttggatccgc cccccagacc atcaccgagt tgtgcagcga gtac                        44


<210>  181
<211>  45
<212>  DNA
<213>  Artificial Sequence

<220>
<223>  primer

<400>  181
tttagatctg gtgccggaca gctcgcagag ggtcaaggag tagat                       45


<210>  182

<211>  58
<212>  DNA
<213>  Artificial Sequence

<220>
<223>  primer

<400>  182
gagaaccagg accagaacca ggggatctgg tgccgttcag ctcgcagagg gtcaagtt        58


<210>  183
<211>  50
<212>  DNA
<213>  Artificial Sequence

<220>
<223>  primer

<400>  183
cctggttctg gtcctggttc tcctagatcc gccccccaga ccatcaccga                  50


<210>  184
<211>  68
<212>  DNA
<213>  Artificial Sequence

<220>
<223>  primer

<400>  184
aaagatctgt tctccatgct gatggcggcg atgctgttgg gggtcttgtt gttccagacg      60
cagagctt                                                                68


<210>  185
<211>  41
<212>  DNA
<213>  Artificial Sequence

<220>
<223>  primer

<400>  185
aagcatgcgg atccaacgcc gcctcctcgt cctcctcgca c                           41


<210>  186
<211>  64
<212>  DNA
<213>  Artificial Sequence

<220>
<223>  primer

<400>  186
gagaaccagg accagaacca ggggatctgg tgccggacag ctcgcagagg gtcaaggagt      60
agat                                                                    64


<210>  187
<211>  69

<212>  PRT
<213>  Escherichia coli

<400>  187

Ala Ala Asp Cys Ala Lys Gly Lys Ile Glu Phe Ser Lys Tyr Asn Glu
1               5                   10                  15
Asp Asn Thr Phe Thr Val Lys Val Ser Gly Arg Glu Tyr Trp Thr Asn
            20                  25                  30
Arg Trp Asn Leu Gln Pro Leu Leu Gln Ser Ala Gln Leu Thr Gly Met
        35                  40                  45
Thr Val Thr Ile Ile Ser Asn Thr Cys Ser Ser Gly Ser Gly Phe Ala
    50                  55                  60
Gln Val Lys Phe Asn
65